(19) Europäisches Patentamt
European Patent Office
Office européen des brevets

(11) Veröffentlichungsnummer: **0 228 094 B1**

(12) # EUROPÄISCHE PATENTSCHRIFT

(45) Veröffentlichungstag der Patentschrift: **14.08.91**

(51) Int. Cl.[5]: **C07D 498/04**, A61K 31/535, //(C07D498/04,265:00,239:00)

(21) Anmeldenummer: **86118122.0**

(22) Anmeldetag: **30.12.86**

(54) **Pyrimido[5,4-b][1,4]oxazinderivate, Verfahren zu ihrer Herstellung und diese enthaltende Arzneimittel sowie Zwischenprodukte darstellende Pyrimido[5,4-b][1,4]oxazinderivate.**

(30) Priorität: **30.12.85 HU 504285**

(43) Veröffentlichungstag der Anmeldung:
**08.07.87 Patentblatt 87/28**

(45) Bekanntmachung des Hinweises auf die Patenterteilung:
**14.08.91 Patentblatt 91/33**

(84) Benannte Vertragsstaaten:
**AT BE CH DE FR GB IT LI LU NL SE**

(56) Entgegenhaltungen:
**EP-A- 0 018 139**

**CHEMICAL ABSTRACTS, Band 78, Nr. 19, 14. Mai 1973, Seiten 479,480, Zusammenfassung Nr. 124532e, Columbus, Ohio, US; N.V. SAZONOV et al.: "Nitrogen- and sulfur-containing heterocycles. XXVI. Properties of pyrimido[5,4-b][1,4]oxazin-7-ones"**

(73) Patentinhaber: **CHINOIN Gyogyszer és Vegyészeti Termékek Gyára RT.
To utca 1-5
H-1045 Budapest IV(HU)**

(72) Erfinder: **Kasztreiner, Endre, Dr.
Szabolcska M. u. 7
H-1114 Budapest(HU)**
Erfinder: **Rabloczky, György, Dr.
Battyány u. 3
H-1015 Budapest(HU)**
Erfinder: **Makk, Nándor, Dr.
Erzsébet u. 19
H-1045 Budapest(HU)**
Erfinder: **Cseh, György, Dr.
Népszinház u. 33
H-1081 Budapest(HU)**
Erfinder: **Kuhár, geb. Kürthy, Mária
Kinizsi u. 4
H-1152 Budapest(HU)**
Erfinder: **Diesler, Eszter
Szabolcska M. u. 7
H-1114 Budapest(HU)**

**EP 0 228 094 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

Erfinder: **Jaszlits, Lászl , Dr.**
**Maros u. 4**
**H-1122 Budapest(HU)**
Erfinder: **Sebestyén, Lászl**
**Körakás park 44**
**H-1157 Budapest(HU)**
Erfinder: **Wellmann, János, Dr.**
**Torock u. 12**
**H-1026 Budapest(HU)**
Erfinder: **Tegdes, Anik**
**Victor Hugo u. 43**
**H-1132 Budapest(HU)**
Erfinder: **Sárossy, geb. Kincsesy, Judit**
**2872 Humboldt Avenue**
**Santa Clara, CA 95051(US)**
Erfinder: **Mátyus, Péter, Dr.**
**Solymár u. 10**
**H-1032 Budapest(HU)**
Erfinder: **Varr , András, Dr.**
**Fadrusz u. 2**
**H-1114 Budapest(HU)**
Erfinder: **Szám, Lászl , Dr.**
**Vörösvári u. 3**
**H-1035 Budapest(HU)**
Erfinder: **Végvári, geb. Gyürki, Sarolta**
**Rák czi u. 72**
**H-2621 Veröcemaros(HU)**

(74) Vertreter: **Beszédes, Stephan G., Dr. Patentanwalt**
**Münchener Strasse 80a Postfach 1168**
**W-8060 Dachau(DE)**

**Beschreibung**

Die Erfindung betrifft neue Pyrimido[5,4-b][1,4]oxazinderivate, ein Verfahren zu ihrer Herstellung und diese enthaltende Arzneimittel, insbesondere mit den Kreislauf verstärkender und dabei vor allem die Leistung der ungenügend wirkenden Herzmuskulatur erhöhender und zum Teil auch den Kreislauf der Koronargefäße günstig beeinflussender Wirkung, sowie neue Zwischenprodukte zur Herstellung der ersteren darstellende Pyrimido[5,4-b][1,4]oxazinderivate.

Die erfindungsgemäßen Pyrimido[5,4-b][1,4]oxazinderivate gehören einem bisher nur wenig untersuchten Ringsystem an.

Über diesen Verbindungstyp finden sich nur wenige Mitteilungen im Schrifttum. So wurden von Sazonov und T. S. Safonov vor allem in der 2-Stellung eine Aminogruppe, einen Acetamidorest oder einen Methylrest sowie in der 4-Stellung eine Hydroxygruppe, einen Methylrest oder Chlor aufweisende Derivate (Him. Geterotsikl. Soed. 1972, 1 285) und später in den 6,7-Stellungen eine Doppelbindung und Ethoxycarbonylreste aufweisende Verbindungen (ebenda 1973, 1 694) sowie darauffolgend in der 6-Stellung niedere Alkylreste aufweisende Derivate (ebenda 1976, 681) und schließlich eine in der 4-Stellung einen Morpholinylrest aufweisende Verbindung (ebenda 1978, 391 bis 396) beschrieben.

Es ist allgemein bekannt, daß die mit dem Blutkreislauf verknüpften Krankheiten, zum Beispiel die chronische Herzinsuffizienz, Herzinfarkt und Hypertonie zu den häufigsten Todesursachen gehören. Innerhalb dieser Krankheitskategorie nimmt die Anzahl der Herzkranken und dabei speziell die Anzahl derer, welche an einer chronischen Herzinsuffizienz leiden, zu. Heute wird diese Krankheit vor allem mit Herzglykosiden behandelt. Es ist aber wohlbekannt, daß die Verwendung dieser Arzneimittel aus mehreren Gründen nicht ungefährlich ist. Einerseits sind ungünstige Nebenwirkungen, zum Beispiel Bradykardie und Rhythmusstörungen, in Betracht zu ziehen und andererseits ist die Einstellung der Dosis schwer und beansprucht eine hohe Vorsicht von seiten des Arztes, da die therapeutische Breite dieser Wirkstoffe niedrig ist. Hinzu kommt es, daß sie in einigen Fällen, zum Beispiel in einer akuten oder auf Herzinfarkt folgenden Herzinsuffizienz, nicht verwendet werden können.

Im letzten Jahrzehnt nahm der therapeutische Anspruch an solche Wirkstoffe, welche die Leistung der Herzmuskulatur über eine lange Zeitdauer, nach oraler Verabreichung und ohne Auftreten von ungünstigen Nebenwirkungen zu erhöhen vermögen, zu.

Diese Forschungen ergaben die Schaffung von mehreren neuen cardiotonischen Verbindungen, deren hervorragende größere Gruppen an einigen Beispielen wie folgt charakterisiert werden können.

Ein auch in klinischen Forschungen eingehend untersuchter Vertreter der $ß_1$-Adrenozeptor-Agonisten ist 1-[4'-(Hydroxy)-phenyl]-3-[tert.-butylamino]-propan-2-ol {Prenalterol}. Diese Verbindung erwies sich bei akuter Herzinsuffizienz intravenös verabreicht als sehr wirksam, verbesserte aber die Arbeit des Herzens bei einer oralen Dauerbehandlung nicht (J. Cardiovasc. Pharm. 6 [1984], 491).

1-[5'-(Hydroxy)-6'-(hydroxymethyl)-pyrid-2'-yl]-2-[tert.-butylamino]-ethanol {Pirbuterol}, das sowohl $ß_1$- als auch $ß_2$-Rezeptoren stimuliert, übt eine günstige Wirkung auch während einer mehrwöchigen oralen Behandlung aus; nach einer längeren Anwendungsperiode wurde aber eine Angewöhnung (Toleranz) beobachtet (New Engl. J. Med. 305 [1981], 185).

Die Wirkungsweise der cardiotonischen Substanzen des "Bipyridin"-Typs weicht von der der ß-Adrenozeptor-Agonisten ab, wurde aber bisher noch nicht aufgeklärt.

Ein hervorragender Vertreter dieser Verbindungsgruppe, das 3-Amino-5-(pyrid-4'-yl)-2(1H)-pyridinon oder mit anderer Bezeichnung 5-Amino-3,4'-bipyridin-6(1H)-on {Amrinon}, steigert bedeutend das Herzminutenvolumen der an einer chronischen Herzinsuffizienz leidenden Kranken nach oraler Verabreichung und vermindert auch den Widerstand der peripherischen Blutgefäße, seine therapeutische Verwendbarkeit wird aber durch schwere Blutbild-Nebenwirkungen weitgehend beschränkt (Am. J. Med. 72 [1982], 113; Brit. J. Clin. Pharm. 17 [1984], 317).

Ein Vertreter der Verbindungsklasse der Imidazopyridine, 2-[4'-(Methylsulfinyl)-2'-(methoxy)-phenyl]-1H-imidazo[4,5-b]pyridin {Sulmazol}, hat außer der cardiotonischen Wirkung auch eine gefäßerweiternde Wirkung. Im Laufe der klinischen Versuche wurden Nebenwirkungen, die mit ziemlich hoher Häufigkeit auftraten, festgestellt (Annal. Cardiol. Anghiol. 33 [1984], 219).

In Khim. Geterotsikl. Soedin, 1973, No. 2, Seiten 171 bis 174, referiert in "Chemical Abstracts", Band 78, Nr. 19, 14. Mai 1973, Referat Nr. 124532e, Seiten 479 bis 480 wird die Herstellung von Pyrimido[5,4-b][1,4]oxazin-7-onen der dortigen Formel I, bei welchen unter anderem die 2-Stellung durch einen Methylrest und die 4-Stellung durch ein Chloratom oder die 2- und 6-Stellungen je 1-fach durch Methylreste und die 4-Stellung durch ein Chloratom oder die 2-Stellung durch einen Methylrest, die 6-Stellung durch einen Äthylrest und die 4-Stellung durch ein Chloratom,oder die 2-Stellung durch einen Methylrest und die 4-Stellung durch einen Morpholinorest oder die 2- und 8-Stellungen je 1-fach durch Methylreste und die 4-Stellung durch ein Chloratom oder die 2-Stellung durch einen Methylrest und die 8-Stellung durch einen Benzylrest oder die 2- und 8-Stellungen je 1-fach durch Methylreste und die 4-Stellung durch einen Morpholinorest substituiert sein kann beziehungsweise können, beschrieben. Diese sind also von den erfindungsgemäßen Oxazinderivaten strukturmäßig verschieden. Eine pharmakologische Wirkung ist in dieser Druckschrift nicht erwähnt.

Die EP-A-0018139 beschreibt Pyrido[2,3-d]pyrimidine mit einer Antisekretwirkung beziehungsweise antiallergischen Wirkung.

Aus Khim. Geterotsikl.Soedin,1972, No. 9, Seiten 1 285 bis 1 288, referiert in "Chemical Abstracts", Band 78, 1973, Referat No. 124 532e ist die Herstellung von Pyrimido[5,4-b] [1,4]oxazin-7-onen, bei welchen unter anderem die 2-Stellung und gegebenenfalls 6-Stellung (je) 1-fach durch einen Methylrest und die 4-Stellung durch ein Chloratom oder die 2-Stellung durch einen Methylrest, die 4-Stellung durch ein Chloratom und die 6-Stellung durch einen Äthylrest substituiert sein kann beziehungsweise können, bekannt. Auch diese sind also von den erfindungsgemäßen Oxazinderivaten strukturmäßig verschieden. Eine pharmakologische Wirkung ist auch in dieser Druckschrift nicht erwähnt.

In Khim. Geterotsikl. Soedin, 1976, No. 5, Seiten 681 bis 685, referiert in "Chemical Abstracts", Band 85, 1976, Referat No. 108598v ist die Herstellung von Pyrimido[5,4-b] [1,4]oxazin-7-onen, bei welchen unter anderem die 2-Stellung und gegebenenfalls die 6-Stellung (je) 1-fach durch einen Methylrest und die 4-Stellung durch ein Chloratom oder die 2-Stellung durch einen Methylrest, die 4-Stellung durch ein Chloratom und die 6-Stellung durch einen Äthyl- oder n-Propylrest oder die 2-Stellung durch einen Methylrest, die 6-Stellung durch 2 Methylreste und die 4-Stellung durch ein Chloratom oder die 4-Stellung durch ein Chloratom und die 6-Stellung durch einen Methyl-, Äthyl- oder n-Propylrest je 1-fach substituiert sein kann beziehungsweise können und entsprechende Verbindungen mit einer Hydrazino- oder Azidogruppe in der 4-Stellung beschrieben. Auch diese sind also von den erfindungsgemäßen Oxazinderivaten strukturmäßig verschieden. Eine pharmakologische Wirkung ist auch in dieser Druckschrift nicht erwähnt.

Aus Khim. Geterotsikl. Soedin, 1978, No. 3, Seiten 391 bis 396, referiert in "Chemical Abstracts", Band 89, 1978, Referat No. 43 307 ist die Herstellung von Pyrimido[5,4-b] [1,4]oxazin-7-onen, bei welchen unter anderem die 2-Stellung durch einen Methylrest, die 6-Stellung durch einen Äthylrest und die 4-Stellung durch ein Chloratom substituiert sein kann, bekannt. Auch eine Verbindung mit einem Methylsubstituenten in der 2-Stellung und einem Morpholinosubstituenten in der 4-Stellung ist in dieser Druckschrift beschrieben, bei welcher aber in der 6-Stellung ein n-Butoxycarbonyloxysubstituent vorliegt. Auch diese Verbindungen sind also von den erfindungsgemäßen Oxazinderivaten strukturmäßig verschieden. Eine pharmakologische Wirkung ist auch in dieser Druckschrift nicht erwähnt.

Die biologischen Eigenschaften der wichtigsten Vertreter der von den Herzglykosiden verschiedenen cardiotonischen Substanzen weisen darauf hin, daß ein ständiger Bedarf an neueren dauernd verabreichbaren Wirkstoffen ohne Nebenwirkungen besteht, die also im Vergleich zu den bekannten Substanzen mit höherer Sicherheit anwendbar sind.

Der Erfindung liegt die Aufgabe zugrunde, neue Pyrimido[5,4-b][1,4]oxazinderivate mit überlegenen pharmakodynamischen Wirkungen, insbesondere mit den Kreislauf verstärkenden und dabei vor allem die Leistung der ungenügend wirkenden Herzmuskulatur erhöhender und auch den Kreislauf der Koronargefäße günstig beeinflussender Wirkung, mit geringeren Nebenwirkungen, ein Verfahren zur Herstellung derselben und diese enthaltende Arzneimittel sowie neue Zwischenprodukte für ihre Herstellung zu schaffen.

Das Obige wurde überraschenderweise durch die Erfindung erreicht, indem überraschenderweise festgestellt wurde, daß die im folgenden festgelegten Pyrimido[5,4-b][1,4]oxazinderivate den obigen pharmakologischen Anforderungen genügen.

Gegenstand der Erfindung sind daher Pyrimido[5,4-b][1,4]oxazinderivate der allgemeinen Formel

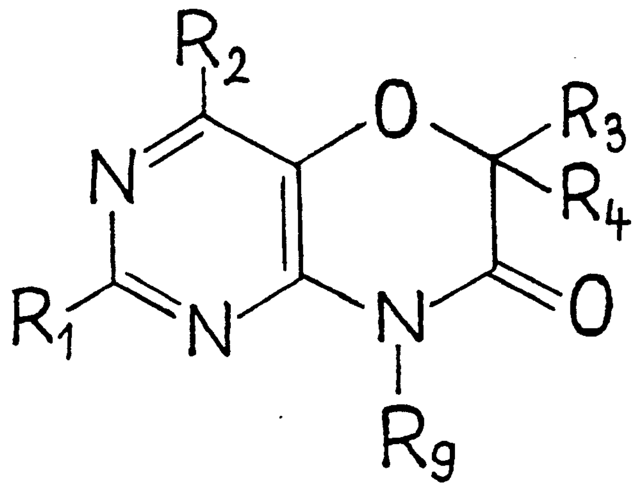

I

worin

$R_1$ für einen Alkylrest mit 1 bis 4 Kohlenstoffatom(en) steht,

$R_2$ ein Wasserstoffatom, ein Halogenatom, eine Azidogruppe, eine Hydrazinogruppe oder einen Rest der allgemeinen Formel

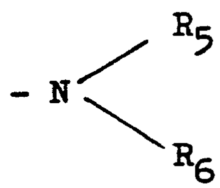

II ,

in welchletzterer

$R_5$ für ein Wasserstoffatom, einen, gegebenenfalls durch eine Hydroxygruppe substituierten Alkylrest mit 1 bis 4 Kohlenstoffatom(en) oder Benzylrest steht und

$R_6$ ein Wasserstoffatom, eine Aminogruppe, einen Cycloalkylrest mit 3 bis 6 Kohlenstoffatomen, einen, gegebenenfalls durch eine Hydroxygruppe, eine Mercaptogruppe, einen Aminocarbonylrest, einen Furylrest, einen 2-Benzo[1,4]dioxanylrest, einen Di-(alkyl)-aminorest mit 1 bis 4 Kohlenstoffatom(en) in jedem Alkylteil oder einen, gegebenenfalls durch ein Halogenatom oder einen Alkylrest mit 1 bis 4 Kohlenstoffatom(en) oder 1 oder mehr Alkoxyrest(e) mit 1 bis 4 Kohlenstoffatom(en) substituierten, Phenyl- oder Phenoxyrest oder einen, gegebenenfalls durch einen, gegebenenfalls durch einen Alkylrest mit 1 bis 4 Kohlenstoffatom(en) oder Benzylrest substituierten, 6 gliedrigen gesättigten Stickstoff aufweisenden und gegebenenfalls außer dem Stickstoffatom noch 1 weiteres Stickstoffatom und/oder 1 Sauerstoffatom aufweisenden heterocyclischen Rest substituierten, Alkylrest mit 1 bis 4 Kohlenstoffatom(en) darstellt oder

$R_5$ und $R_6$ zusammen mit dem Stickstoffatom, an welches sie gebunden sind, einen, gegebenenfalls durch einen Alkoxycarbonylrest mit 2 bis 4 Kohlenstoffatomen oder einen Hydroxyalkylrest mit 1 bis 4 Kohlenstoffatom(en) substituierten, 6-gliedrigen gesättigten und gegebenenfalls außer dem Stickstoffatom noch 1 weiteres Stickstoffatom und/oder 1 Sauerstoffatom aufweisenden heterocyclischen Rest darstellen,

bedeutet,

$R_3$ und $R_4$ , die gleich oder verschieden sein können, voneinander unabhängig für ein Wasserstoffatom oder einen Alkylrest mit 1 bis 4 Kohlenstoffatom(en) stehen und

$R_9$ ein Wasserstoffatom, einen, gegebenenfalls durch eine Oxogruppe, eine Cyanogruppe, einen Aminocarbonylrest, einen Alkoxycarbonylrest mit 2 bis 5 Kohlenstoffatomen, einen Pyridylrest, einen Morpholinylcarbonylrest oder einen Phenylrest monosubstituierten

oder durch 1 oder mehr Hydroxygruppe(n) mono- oder polysubstituierten, Alkylrest bedeutet,

mit den weiteren Maßgaben, daß

a)

$R_2$ von einem Wasserstoffatom, Chloratom, einem 4-Morpholinylrest oder einem Piperidylrest verschieden ist, wenn $R_9$ für ein Wasserstoffatom steht, und

b)

$R_2$ von einem Chloratom oder einem 4-Morpholinylrest verschieden ist, wenn $R_9$ für einen Methyl- oder Benzylrest steht,

einschließlich ihrer Tautomere und ihrer Gemische sowie ihre Säureadditionssalze.

Es ist bevorzugt, daß

a) der Alkylrest, für den $R_1$ und/oder $R_3$ und/oder $R_4$ und/oder $R_5$ und/oder $R_6$ stehen kann beziehungsweise können, und/oder

b) die Alkylteile des Di-(alkyl)-aminorestes, durch welchen der Alkylrest, für welchen $R_6$ stehen kann, substituiert sein kann, oder

c) der Alkylrest oder Alkoxyrest, durch welchen der Phenyl- oder Phenoxyrest, durch den der Alkylrest, für welchen $R_6$ stehen kann, substituiert sein kann, substituiert sein kann, oder

d) der Alkylrest, durch welchen der Benzylrest, durch den der heterocyclische Rest, durch welchen der Alkylrest, für welchen $R_6$ stehen kann, jeweils substituiert sein kann oder

e) der Hydroxyalkylrest, durch den der heterocyclische Rest, den $R_5$ und $R_6$ zusammen mit dem Stickstoffatom, an welchen sie gebunden sind, darstellen können, substituiert sein kann,

[ein] solche[r] mit 1 bis 3, insbesondere 1 oder 2, Kohlenstoffatom(en) ist beziehungsweise sind und/oder

f) der Alkylrest, für den $R_9$ stehen kann,

[ein] solche[r] mit 1 bis 4, insbesondere 1 bis 3, Kohlenstoffatomen ist und/oder

g) der Alkoxycarbonylrest, durch welchen der heterocyclische Rest, den $R_5$ und $R_6$ zusammen mit dem Stickstoffatom, an welches sie gebunden sind, darstellen können, substituiert sein kann, und/oder

h) der Alkoxycarbonylrest, durch welchen der Alkylrest, für den $R_9$ stehen kann, substituiert sein kann,

[ein] solche[r] mit 2 bis 4 Kohlenstoffatomen ist.

Es ist auch bevorzugt, daß der Cycloalkylrest, für welchen $R_6$ stehen kann, ein solcher mit 3 bis 5, insbesondere 3 oder 4, ganz besonders 3, Kohlenstoffatomen ist.

Ferner ist es bevorzugt, daß

a) das Halogenatom, für welches $R_2$ stehen kann oder

b) das Halogenatom, durch welches der Phenyl- oder Phenoxyrest, durch den der Alkylrest, für welchen $R_6$ stehen kann, substituiert sein kann, substituiert sein kann,

ein Fluor- oder Chloratom ist.

Weiterhin ist es bevorzugt, daß

a) der heterocyclische Rest, durch welchen der Alkylrest, für den $R_6$ stehen kann, substituiert sein kann, oder

b) der heterocyclische Rest, den $R_5$ und $R_6$ zusammen mit dem Stickstoffatom, an welches sie gebunden sind, darstellen können,

ein Morpholinylrest, insbesondere Morpholin-4-ylrest oder Morpholin-2-ylrest, ein Piperidylrest, insbesondere Piperid-1-ylrest, oder Piperazinylrest, insbesondere Piperazin-1-ylrest, ist.

Ganz besonders bevorzugt ist der Alkylrest, für den $R_1$ stehen kann, ein Methylrest.

Ganz besonders bevorzugt ist das Halogenatom, für welches $R_2$ stehen kann, ein Fluoratom.

Ganz besonders bevorzugt ist beziehungsweise sind der beziehungsweise die Alkylrest(e), für welche[n] $R_5$ und/oder $R_6$ stehen kann beziehungsweise können, sofern er beziehungsweise sie nicht substituiert ist beziehungsweise sind, [ein] Methylrest(e).

Ganz besonders bevorzugt ist beziehungsweise sind der beziehungsweise die Alkylrest(e), für welche-[n] $R_5$ und/oder $R_6$ stehen kann beziehungsweise können, sofern er beziehungsweise sie durch eine Hydroxygruppe substituiert ist beziehungsweise sind, [ein] Ethylrest(e). Ganz besonders bevorzugt liegt die Substitution in der 2-Stellung vor.

Ganz besonders bevorzugt ist der Alkylrest, für welchen $R_6$ stehen kann, sofern er durch eine Mercaptogruppe einen Di-(alkyl)-aminorest, einen, gegebenenfalls wie oben angegeben substituierten, Phenyl- oder Phenoxyrest oder einen, gegebenenfalls wie oben angegeben substituierten, heterocyclischen Rest substituiert ist, ein Ethylrest, wobei ganz besonders bevorzugt die Substitution in der 2-Stellung des Ethylrestes vorliegt. Im letztgenannten Fall der Substitution durch einen heterocyclischen Rest ist nächstfolgend ganz besonders bevorzugt der Alkylrest, für den $R_6$ stehen kann, ein Methylrest oder n-Propylrest, wobei ganz besonders bevorzugt die Substitution in der 3-Stellung des n-Propylrestes vorliegt. Ferner ist

es, sofern es sich beim heterocyclischen Rest um einen Morpholinyl-, Piperidyl- oder Piperazinylrest handelt, ganz besonders bevorzugt, daß die Substitution in dessen 4-Stellung und nächstfolgend bevorzugt in der 2-Stellung vorliegt. Sofern diese heterocyclischen Reste substituiert sind, sind sie ganz besonders bevorzugt ein Morpholin-2-yl-, Piperid-2-yl- beziehungsweise Piperazin-2-ylrest, während sie anderenfalls ganz besonders bevorzugt ein Morpholin-4-yl-, Piperid-4-yl- beziehungsweise Piperazin-4-ylrest sind, wobei aber auch im letztgenannten Fall eine Bevorzugung für den Morpholin-2-yl-, Piperidin-2-yl- beziehungsweise Piperazin-2-ylrest vorliegt.

Ganz besonders bevorzugt ist der Di-(alkyl)-aminorest, durch welchen der Alkylrest, für den $R_6$ stehen kann, substituiert sein kann, ein Di-(Ethyl)-aminorest.

Ganz besonders bevorzugt ist der Alkylrest, für welchen $R_6$ stehen kann, sofern er durch einen Aminocarbonyl-, Furyl- oder 2-Benzo[1,4]dioxanylrest substituiert ist, ein Methylrest.

Ganz besonders bevorzugt ist das Halogenatom, durch welches der Phenyl- oder Phenoxyrest, durch welchen der Alkylrest, für den $R_6$ stehen kann, substituiert sein kann, substituiert sein kann, ein Fluoratom.

Ganz besonders bevorzugt ist der Alkylrest, durch welchen der Phenyl- oder Phenoxyrest, durch welchen der Alkylrest, für den $R_6$ stehen kann, substituiert sein kann, substituiert sein kann, ein Methylrest. Nächstfolgend ganz besonders bevorzugt ist der Ethylrest, wobei ganz besonders bevorzugt die Substitution in der 2-Stellung des Ethylrestes vorliegt.

In den beiden letztgenannten Fallgruppen liegt die Substitution ganz besonders bevorzugt in der 4-Stellung des Phenyl- oder Phenoxyrestes vor.

Ganz besonders bevorzugt ist beziehungsweise sind der beziehungsweise die Alkoxyrest(e), durch welche[n] der Phenyl- oder Phenoxyrest, durch welchen der Alkylrest, für den $R_6$ stehen kann, substituiert sein kann, substituiert sein kann, [ein] Methoxyrest(e). In diesem Falle liegt die Substitution ganz besonders bevorzugt in der beziehungsweise den 4- und/oder 3-Stellung(en) vor.

Ganz besonders bevorzugt ist der Alkylrest, durch welchen der heterocyclische Rest, durch den der Alkylrest, für welchen $R_6$ stehen kann, substituiert sein kann, substituiert sein kann, ein Methylrest.

Ganz besonders bevorzugt ist der Alkoxycarbonylrest, durch welchen der heterocyclische Rest, für den $R_6$ stehen kann, substituiert sein kann, ein Ethoxycarbonylrest, vor allem 2-(Ethoxy)-carbonylrest.

Ganz besonders bevorzugt ist der Hydroxyalkylrest, durch welchen der heterocyclische Rest, für den $R_6$ stehen kann, substituiert sein kann, ein Hydroxyethylrest, vor allem 2-(Hydroxy)-ethylrest.

In den beiden letztgenannten Fällen liegt die Substitution, sofern es sich beim heterocyclischen Rest um einen Piperidyl- oder Piperazinylrest handelt, ganz besonders bevorzugt in dessen 4-Stellung vor. Sofern diese heterocyclischen Reste substituiert sind, sind sie ganz besonders bevorzugt ein Morpholin-2-yl-, Piperid-2-yl- beziehungsweise Piperazin-2-ylrest, während sie anderenfalls ganz besonders bevorzugt ein Morpholin-4-yl-, Piperid-4-yl- beziehungsweise Piperazin-4-ylrest sind, wobei aber auch im letztgenannten Fall eine Bevorzugung für den Morpholin-2-yl-, Piperidin-2-yl- beziehungsweise Piperazin-2-ylrest vorliegt.

Ganz besonders bevorzugt ist beziehungsweise sind der beziehungsweise die Alkylrest(e), für welche[n] $R_3$ und/oder $R_4$ stehen kann beziehungsweise können, [ein] Methylrest(e).

Ganz besonders bevorzugt ist der, gegebenenfalls wie oben angegeben substituierte, Alkylrest, für welchen $R_9$ stehen kann, ein Propylrest, vor allem n-Propylrest, oder Methyl- oder Ethylrest.

Ganz besonders bevorzugt ist der Alkylrest, für welchen $R_9$ stehen kann, sofern er durch eine Cyangruppe oder einen Aminocarbonyl-, Alkoxycarbonyl-, Pyridyl-, Morpholinylcarbonyl- oder Phenylrest substituiert ist, ein Methylrest. Nächstfolgend ganz besonders bevorzugt ist der Ethylrest, wobei die Substitution ganz besonders bevorzugt in der 1-Stellung des Ethylrestes vorliegt.

Ganz besonders bevorzugt ist der Alkylrest, für welchen $R_9$ stehen kann, sofern er durch eine Oxagruppe substituiert ist, ein Propylrest, vor allem n-Propylrest. In diesem Falle liegt die Substitution ganz besonders bevorzugt in der 2-Stellung des n-Propylrestes vor.

Sofern der Alkylrest, für welchen $R_9$ stehen kann, durch mehr als 1 Hydroxygruppe substituiert ist, ist deden Zahl ganz besonders bevorzugt 2.

Ganz besonders bevorzugt ist der Alkylrest, für welchen $R_9$ stehen kann, sofern er durch 1 oder mehr Hydroxygruppe(n) substituiert ist, ein Propylrest, vor allem n-Propylrest. In diesem Falle liegt die Substitution ganz besonders bevorzugt in der beziehungsweise den 3- und/oder 2-Stellung(en) des n-Propylrestes vor.

Ganz besonders bevorzugt ist der Alkoxycarbonylrest, durch welchen der Alkylrest, für welchen $R_9$ stehen kann, substituiert sein kann, ein Butoxycarbonylrest, vor allem n-Butoxycarbonylrest, oder Ethoxycarbonylrest. Dabei handelt es sich ganz besonders bevorzugt beim n-Butoxycarbonylrest um einen 4-(n-Butoxy)-carbonylrest und beim Ethoxycarbonylrest um einen 2-(Ethoxy)-carbonylrest.

Ganz besonders bevorzugt ist der Pyridylrest, durch welchen der Alkylrest, für den $R_9$ stehen kann,

substituiert sein kann, ein Pyrid-3-ylrest.

Ganz besonders bevorzugt ist der Morpholinylcarbonylrest, für welchen $R_9$ stehen kann, ein Morpholin-4-yl-carbonylrest.

Eine bevorzugte Gruppe der erfindungsgemäßen Pyrimido[5,4-b][1,4]oxazinderivate der allgemeinen Formel I sind diejenigen, bei welchen

| | |
|---|---|
| $R_1$ | für einen Alkylrest mit 1 bis 4 Kohlenstoffatom(en) steht, |
| $R_2$ | einen Rest der allgemeinen Formel II bedeutet, |
| $R_3$ und $R_4$ | unabhängig voneinander ein Wasserstoffatom oder einen Alkylrest mit 1 oder 2 Kohlenstoffatom(en) darstellt und |
| $R_9$ | für ein Wasserstoffatom oder einen, gegebenenfalls durch eine Oxogruppe, eine Cyanogruppe, einen Aminocarbonylrest oder einen Alkoxycarbonylrest mit 2 bis 5 Kohlenstoffatomen monosubstituierten oder durch 1 oder mehr Hydroxygruppe(n) mono- oder polysubstituierten, Alkylrest mit 1 bis 4 Kohlenstoffatom(en) steht. |

Die Säureadditionssalze der Pyrimido[5,4-b][1,4]oxazinderivate der allgemeinen Formel I sind zweckmäßig solche mit pharmazeutisch brauchbaren Säuren. Als solche kommen sowohl anorganische Säuren, wie Salzsäure, Bromwasserstoffsäure, Schwefelsäure und Phosphorsäure, als auch organische Säuren, wie Essigsäure, Weinsäure, Maleinsäure und Fumarsäure, in Frage. Bevorzugt sind Salzsäure und Fumarsäure.

Ganz besonders bevorzugte erfindungsgemäße Pyrimido[5,4-b][1,4]oxazinderivate sind

2-[Methyl]-4-[⟨2'-morpholin-4''-yl)-ethyl⟩-amino)-6,7-di-[hydro]-8H-pyrimido[5,4-b][1,4]oxazin-7-on,
4-[N-⟨Benyl⟩-N-⟨2'-(hydroxy)-ethyl⟩-amino]-2-[methyl]-6,7-di-[hydro]-8H-pyrimido[5,4-b[1,4]oxazin-7-on,
2-[Methyl]-4-[⟨3'-(morpholin-4''-yl)-propyl⟩-amino]-6,7-di-[hydro]-8H-pyrimido[5,4-b][1,4]oxazin-7-on,
4-[Chlor]-8-[2',3'-di-(hydroxy)-propyl]-2-[methyl]-6,7-di-[hydro]-8H-pyrimido[5,4-b][1,4]oxazin-7-on,
8-[2',3'-Di-(hydroxy)-propyl]-2-[methyl]-4-[morpholin-4''-yl]-6,7-di-[hydro]-8H-pyrimido[5,4-b][1,4]oxazin-7-on,
4-[⟨4'-(Benzyl)-morpholin-2'-yl)-methyl⟩-amino]-2-[methyl]-6,7-di-[hydro]-8H-pyrimido[5,4-b][1,4]oxazin-7-on,
8-[(Ethoxy)-carbonylmethyl]-4-[⟨2''-(hydroxy)-ethyl⟩-amino]-2-[methyl]-6,7-di-[hydro]-8H-pyrimido[5,4-b][1,4]-oxazin-7-on,
4-[⟨2'-(Hydroxy)-ethyl⟩-amino]-2-[methyl]-8-[2''-(oxo)-propyl]-6,7-di-[hydro]-8H-pyrimido[5,4-b][1,4]oxazin-7-on,
4-[⟨2'-(Hydroxy)-ethyl⟩-amino]-2-[methyl]-8-[morpholin-4''-ylcarbonylmethyl]-6,7-di-[hydro]-8H-pyrimido[5,4-b][1,4]oxazin-7-on,
8-[(n-Butoxy)-carbonylmethyl]-4-[⟨2'-(hydroxy)-ethyl⟩-amino]-2-[methyl]-6,7-di-[hydro]-8H-pyrimido[5,4-b]-[1,4]oxazin-7-on,
8-[Benzyl]-4-[⟨2'-(hydroxy))-ethyl⟩-amino]-2-[methyl]-6,7-di-[hydro]-8H-pyrimido[5,4-b][1,4]oxazin-7-on,
4-[Amino]-8-[(ethoxy)-carbonylmethyl]-2-[methyl]-6,7-di-[hydro]-8H-pyrimido[5,4-b][1,4]oxazin-7-on

sowie ihre Säureadditionssalze, insbesondere Hydrochloride und Fumarate. Von diesen sind überragend bevorzugt das 2-[Methyl]-4-[⟨2'-(morpholin-4''-yl)-ethyl⟩-amino]-6,7-di-[hydro]-8H-pyrimido[5,4-b][1,4]oxazin-7-on und sein Fumarat.

Gegenstand der Erfindung ist auch ein Verfahren zur Herstellung der erfindungsgemäßen Verbindungen, welches dadurch gekennzeichnet ist, daß

a) zur Herstellung der Pyrimido[5,4-b][1,4]oxazinderivate der allgemeinen Formel I, bei welchen $R_2$ für einen Rest der allgemeinen Formel II, in welcher $R_5$ und $R_6$ die oben angegebenen Bedeutungen haben steht und $R_1$ , $R_3$ , $R_4$ und $R_9$ die oben angegebenen Bedeutungen haben, Pyrimido[5,4-b][1,4]-oxazinderivate der allgemeinen Formel

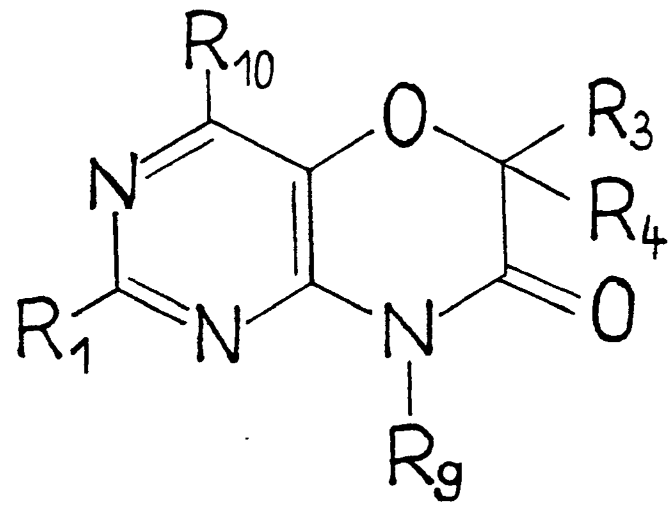

III ,

worin $R_1$, $R_3$, $R_4$ und $R_9$ die oben angegebenen Bedeutungen haben und $R_{10}$ für eine austretende Gruppe steht, mit, gegebenenfalls eine Schutzgruppe aufweisenden, Aminen der allgemeinen Formel

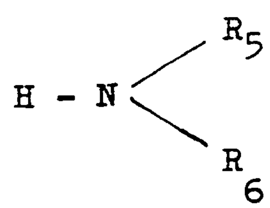

IV ,

worin $R_5$ und $R_6$ die oben angegebenen Bedeutungen haben, umgesetzt werden oder

b) zur Herstellung der Pyrimido[5,4-b][1,4]oxazinderivate der allgemeinen Formel I, bei welchen $R_2$ für eine Azidogruppe steht und $R_1$, $R_3$, $R_4$ und $R_9$ die oben angegebenen Bedeutungen haben, Pyrimido-[5,4-b][1,4]oxazinderivate der allgemeinen Formel I, bei welchen $R_1$, $R_3$, $R_4$ und $R_9$ die oben angegebenen Bedeutungen haben und $R_2$ für eine Hydrazinogruppe steht, diazotiert werden und/oder

c) zur Herstellung der Pyrimido[5,4-b][1,4]oxazinderivate der allgemeinen Formel I, bei welchen $R_1$, $R_2$, $R_3$ und $R_4$ die oben angegebenen Bedeutungen haben und $R_9$ für einen 2,3-Di-(hydroxy)-propylrest steht, Pyrimido[5,4-b][1,4]oxazinderivate der allgemeinen Formel I, bei welchen $R_1$, $R_2$, $R_3$ und $R_4$ die oben angegebenen Bedeutungen haben und $R_9$ für Wasserstoff steht, mit 2,3-(Epoxy)-propanol umgesetzt werden und

α) gegebenenfalls die nach a) oder b) erhaltenen Pyrimido[5,4-b][1,4}oxazinderivate der allgemeinen Formel I, bei welchen $R_1$, $R_2$, $R_3$ und $R_4$ die oben angegebenen Bedeutungen haben und $R_9$ für Wasserstoff steht, mit, gegebenenfalls eine Schutzgruppe aufweisenden, Verbindungen der allgemeinen Formel

$R_9$ - X V ,

worin

$R_9$ die oben angegebenen Bedeutungen mit Ausnahme von Wasserstoff hat und

X für eine austretende Gruppe steht, umgesetzt werden und/oder

β) gegebenenfalls von den nach a), b) oder c) und gegebenenfalls α) erhaltenen Pyrimido[5,4-b]-[1,4]oxazinderivate der allgemeinen Formel I die Schutzgruppe(n) entfernt wird bzw. werden und/oder

γ) gegebenenfalls nach a), b) oder c) und gegebenenfalls α) und/oder β) erhaltene Pyrimido[5,4-b]-[1,4]oxazinderivate der allgemeinen Formel I in andere Pyrimido[5,4-b][1,4]oxazinderivate der allgemeinen Formel I überführt werden und/oder

δ) gegebenenfalls nach a), b) oder c) und gegebenenfalls α), β) und/oder γ) erhaltene Pyrimido[5,4-b][1,4]oxazinderivatbasen der allgemeinen Formel I in an sich bekannter Weise in Säureadditionssalze überführt werden und/oder gegebenenfalls erhaltene Säureadditionssalze der Pyrimido[5,4-b][1,4]oxazinderivate der allgemeinen Formel I in die freien Pyrimido[5,4-b][1,4]-oxazinderivatbasen der allgemeinen Formel I oder in andere Säureadditionssalze überführt werden.

Unter den "austretenden Gruppen", für welche $R_{10}$ und X stehen, sind gemäß einer vom Schrifttum angenommenen Definition (siehe zum Beispiel T. A. Geissman: Principles of Organic Chemistry, 3. Ausgabe, Ed. W. H. Freeman, London, 1968) solche Gruppen, welche beim Angriff eines nucleophilen Partners relativ leicht abgespalten werden, zu verstehen. Solche Gruppen sind beispielsweise die Halogenatome, Sulfonyloxyreste, wie niedere Alkylsulfonyloxyreste und, gegebenenfalls substituierte, Arylsulfonyloxyreste, zum Beispiel Phenylsulfonyloxyreste, sowie niedere Alkansulfonyl- und Alkansulfinylreste, zum Beispiel der Methylsulfonylrest. Im erfindungsgemäßen Verfahren sind für $R_{10}$ das Chloratom oder der 4-Toluolsulfonyloxyrest und für X das Chlor- oder Bromatom besonders bevorzugt.

Die Variante c) des erfindungsgemäßen Verfahrens kann vor oder nach der Variante a) oder b) des erfindungsgemäßen Verfahrens durchgeführt werden.

Gemäß einer vorteilhaften Ausführungsform der Variante a) des erfindungsgemäßen Verfahrens werden die Amine der allgemeinen Formel IV mit solchen Pyrimido[5,4-b][1,4]oxazinderivaten der allgemeinen Formel III, bei welchen $R_{10}$ = Chlor als austretende Gruppe bedeutet, umgesetzt. Diese Umsetzung wird in organischen Lösungs- und/oder Verdünnungsmitteln in Gegenwart von säurebindenden Mitteln bei Temperaturen von 50 bis 150°C, vorzugsweise bei der Siedetemperatur des Lösungs- oder Verdünnungsmittels, durchgeführt. Geeignete Lösungs- beziehungsweise Verdünnungsmittel sind zum Beispiel aliphatische Alkohole, wie n-Butanol, und/oder aromatische Kohlenwasserstoffe, wie Benzol. Zweckmäßig wird als säurebindendes Mittel ein Überschuß des Amines der allgemeinen Formel IV oder ein organisches tertiäres Amin, wie Triethylamin, oder eine anorganische Base, wie Kaliumcarbonat, verwendet.

Gemäß einer anderen vorteilhaften Ausführungsform der Variante a) des erfindungsgemäßen Verfahrens werden die Amine der allgemeinen Formel III mit solchen Pyrimido[5,4-b][1,4]oxazinderivaten der allgemeinen Formel IV, bei welchen $R_{10}$ für einen Arylsulfonyloxyrest, zum Beispiel 4-Toluolsulfonyloxyrest, steht, umgesetzt. Die Reaktionsteilnehmer werden miteinander in Gegenwart von organischen Lösungs- und/oder Verdünnungsmitteln und säurebindenden Mitteln umgesetzt. Vorzugsweise werden als Lösungsmittel Ester, wie Ethylacetat, und als säurebindendes Mittel ein Überschuß des Amines der allgemeinen Formel IV oder eine organische tertiäre Base, wie Triethylamin, oder eine anorganische Base, wie Kaliumcarbonat, verwendet. Diese Umsetzung wird zweckmäßig bei Temperaturen von 10°C bis zur Siedetemperatur des Reaktionsgemisches durchgeführt.

Die Variante b) des erfindungsgemäßen Verfahrens wird vorteilhaft in der Weise durchgeführt, daß die Pyrimido[5,4-b][1,4]oxazinderivate der allgemeinen Formel I, bei welchen $R_2$ eine Hydrazinogruppe bedeutet, unter den wohlbekannten Bedingungen der Diazotierung, zum Beispiel in einem wäßrig-essigsauren Medium mit Natriumnitrit behandelt werden. Diese Umsetzung wird zweckmäßig bei Temperaturen von 0 bis 5°C durchgeführt.

Gemäß einer bevorzugten Ausführungsform der Variante c) des erfindungsgemäßen Verfahrens werden die Pyrimido[5,4-b][1,4]oxazinderivate der allgemeinen Formel I, bei welchen $R_9$ Wasserstoff bedeutet, in Gegenwart von Lösungsmitteln und quaternären Ammoniumsalzen als Katalysator mit dem 2,3-Epoxypropanol umgesetzt. Zweckmäßig werden als Lösungsmittel aromatische Kohlenwasserstoffe, wie Benzol, und als Katalysator Tetra-(n-butyl)-ammoniumbromid verwendet. Die Umsetzung wird bevorzugt bei der Siedetemperatur des Lösungsmittels, zum Beispiel des Benzols, durchgeführt.

Das im erfindungsgemäßen Verfahren gewonnene Reaktionsgemisch kann in an sich bekannter Weise aufgearbeitet werden, zum Beispiel in der Weise, daß es, gegebenenfalls nach dem Entfernen der anorganischen Salze und/oder des Katalysators, unter Vakuum eingedampft, der Rückstand mit Wasser vermischt und alkalisch gemacht und das so gewonnene Produkt, zum Beispiel durch Filtrieren oder Extrahieren abgetrennt wird. Gegebenenfalls kann die (so gewonnene) Pyrimido[5,4-b][1,4]oxazinderivatbase der allgemeinen Formel I gereinigt und/oder in ein Säureadditionssalz überführt werden, das gegebenenfalls durch Umkristallisieren gereinigt werden kann.

Wenn nach Durchführung der obigen Varianten a) oder b), des erfindungsgemäßen Verfahrens Pyrimido[5,4-b][1,4]oxazinderivate der allgemeinen Formel I, bei welchen $R_9$ für Wasserstoff steht, erhalten werden, wird die gegebenenfalls erfolgende Überführung derselben in Pyrimido[5,4-b][1,4]oxazinderivate der allgemeinen Formel I, bei welchen $R_9$ von Wasserstoff verschieden ist, nach der Gegebenenfallsvariante $\alpha$) des erfindungsgemäßen Verfahrens vorzugsweise in der Weise durchgeführt, daß die Pyrimido[5,4-b]-[1,4]oxazinderivate der allgemeinen Formel I, bei welchen $R_9$ Wasserstoff bedeutet, in Gegenwart von Lösungsmitteln und säurebindenden Mitteln mit den Verbindungen der allgemeinen Formel V, bei welchen X bevorzugt ein Chlor- oder Bromatom oder einen 4-Toluolsulfonyloxyrest bedeutet, umgesetzt werden. Als Lösungsmittel werden zweckmäßig Ketone, wie Aceton und insbesondere Butanon, und/oder niedere

Alkanole, wie Ethanol und/oder Isopropanol, verwendet. Als basische Mittel können zweckmäßig Alkalimetallalkoholate, wie Natriumalkoholat, verwendet werden. Bei Verwendung von Ketonen als Lösungsmitteln, ist es besonders vorteilhaft, Kaliumcarbonat als säurebindendes Mittel einzusetzen. Diese Umsetzung wird zweckmäßig bei der Siedetemperatur des Reaktionsgemisches durchgeführt.

Es ist klar, daß die bei den Varianten a) bis c) des erfindungsgemäßen Verfahrens verwendeten Verbindungen gegebenenfalls mit einer Schutzgruppe (oder mit Schutzgruppen) zu versehen sind, um Nebenreaktionen zu vermeiden. Solche Schutzgruppen sind wohlbekannt; von ihnen ist die Benzylgruppe bei der Herstellung der erfindungsgemäßen Pyrimido[5,4-b][1,4]oxazinderivate besonders günstig anwendbar, da sie nach der gewünschten Umsetzung in bekannter Weise, zweckmäßig durch Hydrogenolyse, nach der Gegebenenfallsvariante β) des erfindungsgemäßen Verfahrens entfernt werden kann.

Die Gegebenenfallsvarianten α), β) und γ) des erfindungsgemäßen Verfahrens können auch in vertauschter Reihenfolge durchgeführt werden. Ein Beispiel für die Durchführung zunächst der Gegebenenfallsvariante γ), dann der Gegebenenfallsvariante β) und schließlich Gegebenenfalls variante α) ist das Umsetzen von 4-[Azido]-2-[alkyl]-6,7-di-[hydro]-8H-pyrimido[5,4-b][1,4]oxazin-7-onen mit Triphenylphosphin, Umsetzen der erhaltenen 2-[Alkyl]-4-[(triphenylphosphoranyliden)-amino]-6,7-di-[hydro]-8H-pyrimido[5,4-b][1,4]-oxazin-7-one mit Mineralsäuren, wie Salzsäure, zu 4-[Amino]-2-[alkyl]-6,7-di-[hydro]-8H-pyrimido[5,4-b][1,4]-oxazin-7-onen und Umsetzen der letzteren mit Chloressigsäureethylester zu 4-[Amino]-8-[(ethoxy)-carbonylmethyl]-2-[alkyl]-6,7-di-[hydro]-8H-pyrimido[5,4-b][1,4]oxazin-7-onen.

Die Überführung der Pyrimido[5,4-b][1,4]oxazinderivatbasen der allgemeinen Formel I in Säureadditionssalze nach der Gegebenenfallsvariante δ) des erfindungsgemäßen Verfahrens kann in an sich bekannter Weise durchgeführt werden, zum Beispiel in der Weise, daß die Pyrimido[5,4-b][1,4]oxazinderivatbase in einem organischen Lösungsmittel gelöst und die entsprechende Säure oder eine Lösung dieser Säure in einem organischen Lösungsmittel zugegeben wird. Das so erhaltene Salzprodukt kann durch Filtrieren oder Eindampfen abgetrennt und gegebenenfalls in an sich bekannter Weise, zum Beispiel durch Umkristallisieren, gereinigt werden. Wenn als Säure eine anorganische Mineralsäure verwendet wird, können als Lösungsmittel vorteilhaft niedere Alkanole, wie Ethanol und/oder Isopropanol und in einigen Fällen Aceton verwendet werden. Wenn als Säure eine organische verwendet wird, können als Lösungsmittel zweckmäßig niedere Alkanole, wie Ethanol und/oder Isopropanol, und/oder niedrigsiedende (50 bis 90°C) aliphatische Ketone, wie Aceton und/oder Butanon, und/oder Ether, vorzugsweise Diethyl- oder Diisopropylether, verwendet werden.

Gegenstand der Erfindung sind auch Arzneimittel, welche 1 oder mehr der erfindungsgemäßen Verbindungen als Wirkstoff(e), zweckmäßig zusammen mit 1 oder mehr üblichen pharmazeutischen Träger- und/oder Hilfsstoff(en), enthalten.

Die erfindungsgemäßen Pyrimido[5,4-b][1,4]oxazinderivate haben nämlich wie bereits gesagt wertvolle pharmakodynamische Wirkungen, insbesondere eine den Kreislauf verstärkende und dabei vor allem eine die Leistung der ungenügend wirkenden Herzmuskulatur erhöhende, sogenannte cardiotonische (positiv inotrope) Wirkung und zum Teil auch den Kreislauf der Koronargefäße günstig beeinflussende Wirkung.

Der die Herzkontraktionskraft steigernde Effekt ("Myocardial Contractile Force", im folgenden abgekürzt: MCF) wurde in *in vivo* Versuchen mittels der folgenden Methoden bestimmt.

A) Prüfung an narkotisierten Katzen

Die Tiere wurden intravenös (i.v.) mit 30 mg/kg Pentobarbital-Natrium narkotisiert und ihr Brustkorb wurde unter künstlicher Beatmung geöffnet. Nach der Eröffnung des Perikardiums wurde ein "strain gauge" auf die epikardiale Oberfläche der linken Kammer genäht [J. Pharm. Exp. Ther. 90, 26 (1947)]. Der Blutdruck wurde so gemessen, dass ein mit einem Drucküberträger und Elektromanometer verbundener Katheter in die Oberschenkelarterie eingeführt wurde. Die Wirkstoffe wurden entweder i.v. durch eine in die Oberschenkelvene gelegte Kanüle oder intraduodenal (i.d.) durch eine i.d. Kanüle verabreicht. Am Versuchsbeginn wurde 0,2 µg/kg Isoproterenol (abgekürzt: IS) als ein innerer Standard gegeben, um die myokardiale Reaktivität zu kontrollieren; das höchste ("peak") MCF-Ansprechen beim Tierindividuum kann sich nämlich bei allen positiv inotropen Mitteln verändern. So wurde IS als keine eigentliche Vergleichssubstanz verwendet.

Das MCF-Ansprechen wurde in den prozentualen Änderungen der Anfangswerte ausgedrückt: die Wirkung von 5 mg/kg i.v. der Prüfsubstanz wurde mit der von 0,2 µg/kg i.v. IS verglichen und in einem Quotienten ausgedrückt. Die Ergebnisse sind in der Tabelle 1 zusammengefasst.

Tabelle 1

MCF-steigernde Wirkung der Verbindungen der allgemeinen Formel I in Katzen mit eröffnetem Brustkorb nach i.v. Verabreichung von 5 mg/kg

| Verbindung (Beispiel Nr.) | MCF-Steigerung als % des Anfangswertes | Wirksamkeit, ausgedrückt durch Vergleich mit 0,2 µg/kg i.v. IS |
|---|---|---|
| 22 (23, 26) | 70 | 1,2 |
| 11 | 83 | 1,2 |
| 60 | 51 | 1,2 |
| 56 | 100 | 1,5 |
| 57 | 45 | 1,3 |
| 27 | 130 | 3,0 |
| 37 | 55,5 | 1,2 |
| 29 | 40 | 0,9 |
| 66 | 58,3 | 0,82 |
| 67 | 37 | 1,8 |
| 69 | 66 | 0,87 |
| 73 | 50 | 0,86 |

Die erfindungsgemässen Verbindungen übten eine ähnliche oder höhere MCF-steigernde Wirkung aus, als IS. Als ein starker β-Adrenozeptor-Agonist, löste IS eine messbare MCF-Steigerung schon in einer i.v. Dosis von 0,2 µg/kg aus: dieser Effekt war aber kurzfristig, völlig reversibel und beeinflusste die Kreislaufparameter nicht. Überraschenderweise wurde die Pulsfrequenz durch die Verbindungen der Tabelle 1 kaum oder durchaus nicht erhöht.

Die Wirkung mehrerer Verbindungen wurde auch nach i.d. Verabreichung untersucht. Die Verbindungen Nr. 27 und 32 wiesen einen starken positiv inotropen Effekt in einer Dosis von 20 mg/kg oder auch nach 5 mg/kg auf. Die Substanz Nr. 22 übte eine kraftvolle und langdauernde Wirkung schon in einer i.v. Dose von 1 mg/kg aus.

B) Prüfung an narkotisierten Hunden

Die Tiere wurden i.v. mit 35 mg/kg Pentobarbital-Natrium narkotisiert. Nach Eröffnung des Brustkorbes und Perikardiums wurde der untere aufsteigende Teil der Arteria coronaria circumflexa präpariert und mit einer elektromagnetischen Blutströmungssonde versehen, um die Blutströmung in der Koronararterie zu messen. Ein "strain gauge" wurde auf die durch dieses Gefäss versehene Oberfläche gelegt, um die MCF zu bestimmen. Zur Messung des systemischen arteriellen Blutdruckes diente eine Oberschenkelarterien-Kanüle, zur i.v. Verabreichung der Wirkstoffe (Prüfsubstanzen) eine Oberschenkelvenen-Kanüle. Der dp/dt-Wert wurde aus dem Druck berechnet, der mittels eines in die linke Kammer eingeführten Drucküberträgers

des Typs "Millar" gemessen wurde. Alle Parameter wurden an einem Beckman R 12 Dynograph (mit 8 Kanälen) registriert.

C) Prüfung an wachen Katzen

Diese Tiere wurden nach der Methode von Rabloczky und Mader ("Measurement of Systemic and Pulmonary Arterial Pressure in Conscious Animals", Vortrag an der International Union of the Pharmacologists, Congress, Budapest, 1980) oder nach mancher Modifizierung derselben verwendet. Die Aorta und Lungenarterie wurden chronisch zur Messung des Blutdruckes katheterisiert. Gemäss der Modifizierung wurde auch die rechte Kammer katheterisiert, um den $dp/dt_{max}$-Wert zu bestimmen.

Die positiv inotrope und koronarerweiternde Wirkung der erfindungsgemässen Verbindungen werden im folgenden an den durch eingehende Prüfung der Verbindungen Nr. 22, 27 und 37 gewonnenen Ergebnissen gezeigt, die mittels der obigen Methoden erhalten wurden.

Prüfung der Verbindung Nr. 22

Die positiv inotrope Wirkung dieser Substanz wurde an narkotisierten Hunden mit eröffnetem Brustkorb schon in einer Dosis von 0,025-1,6 mg/kg beobachtet. Die Stärke und Dauer der MCF-Steigerung waren dosisabhängig. Die positiv inotrope und koronarerweiternde Wirkung der i.v. Dosen von 0,2, 0,4 und 0,8 mg/kg dieser Verbindung wurde mit Dosen von 0,5, 1,0 und 2,0 mg/kg Amrinon [5-Amino- 3,4'-bipyridin -6-(1H)-on] verglichen.

In diesem Dosenintervall löste die Verbindung Nr. 22 eine dosisabhängige MCF-Steigerung aus. Die Wirkung von 2 mg/kg i.v. Amrinon war nicht stärker als die von 0,4 mg/kg Verbindung Nr. 22. Offensichtlich wurde die positiv inotrope Wirkung beider Verbindungen durch den koronarerweiternden Effekt gefördert; die MCF-steigernde Wirkung war aber dauerhafter als die Koronarwirkung.

Die günstige Herzmuskelwirkung der Verbindung Nr. 22 wurde auch nach einer i.d. Applikation bewiesen. Nach einer Dosis von 1 mg/kg wurde die MCF um 35-40 % ohne Veränderung der Koronarströmung gesteigert. Der MCF-steigernde Effekt von Amrinon war mindestens fünfmal schwächer als der der Substanz Nr. 22.

An narkotisierten Hunden mit eröffnetem Brustkorb nach einer akuten Ischämie verminderte sich wesentlich die positive inotrope Wirkung von Amrinon; die Wirkung der Verbindung Nr. 22 blieb aber statistisch unverändert.

Weiterhin wurde die negativ inotrope Wirkung einer Dosis von 0,2 IU/kg Vasopressin bedeutsam durch eine i.v. Dosis von 0,8 mg/kg der Verbindung Nr. 22 vermindert; die vasokonstriktorische Wirkung derselben wurde geradezu umgekehrt. Demnach konnte die Verbindung Nr. 22 die Entwicklung einer chemisch induzierten Ischämie verhindern.

Wenn orale Dosen von 0,5, 2,0, 4,0 und 8,0 mg/kg der Verbindung Nr. 22 chronisch katheterisierten wachen Katzen verabreicht wurden, blieben der systemische arterielle Blutdruck und die Pulsfrequenz praktisch unverändert, die MCF wurde aber nach einer jeden Dosis wesentlich gesteigert. Je höher die Dosis war, umso länger war die Dauer der positiv inotropen Wirkung. Die höchste ("peak") Steigerung im dp/dt-Wert war 60 %.

Es ist überraschend, dass die positiv inotrope Wirkung der Verbindung Nr. 22 durch eine Vorbehandlung mit $\beta$-Adrenozeptor-Antagonisten, Histamin-H-2-Antagonisten oder Reserpin unverändert blieb.

Prüfung der Verbindung Nr. 27

Diese Verbindung wurde an narkotisierten Hunden mit eröffnetem Brustkorb in einer i.v. Dosis von 1 mg/kg oder in einer i.d. Dosis von 5 mg/kg verabreicht.

Nach der i.v. Dosis wurden der MCF-Wert um 40 %, der Koronarkreislauf um 20 % gesteigert; die Pulsfrequenz in einem sehr niedrigen Maße (um 10 %) erhöht; der systemische arterielle systolische Blutdruck um 10 % und der diastolische Blutdruck um 20 % vermindert. Die Halbwertzeit des MCF-Effektes ergab sich als 10 Minuten; das nahezu unveränderte Ansprechen der Koronararterien dauerte 20 Minuten lang.

Nach der i.d. Dosis wurde der MCF-Wert um etwa 30 % erhöht und blieb 30 Minuten lang unverändert.

Biochemische-pharmakologische Untersuchungen

Die folgenden Prüfungen wurden ausgeführt, um die Wirkung der Verbindung Nr. 22 auf verschiedene

Enzyme, vor allem auf die membrangebundenen Enzyme der Herzmuskelzelle aufzuklären.

Wirkung der Verbindung Nr. 22 auf die Na-K-ATP-ase- und NADH-Oxydase Enzyme

Ein Sarkolemm-Präparat wurde aus der Herzmuskelzelle nach der Methode von D. M. Bers [Biochem. Biophys. Acta 555, 131 (1979)] hergestellt. NADH-Dichlorphenol-Reduktase wurde mittels eines enzymanalysierenden Gerätes (Centrifi-CHEM®) bestimmt.

Die Aktivität von Na-K-ATP-ase wurde in einer 50 mM Tris-HCl Lösung bei pH 7,5 gemessen. Ouabain, das als Vergleichssubstanz verwendet wurde, hemmte stark das Na-K-ATP-ase und verminderte nur schwach die Aktivität der NADH-Indophenol-Reduktase. Die Aktivität dieser Enzyme wurde sogar durch eine Konzentration von $10^{-4}$ M der Verbindung Nr. 22 nicht beeinflusst.

Wirkung der Verbindung Nr. 22 auf das die Hinausströmung der $Ca^{2+}$-Ionen regulierende Ca-ATP-ase-Enzym

Die Aktivität der membrangebundenen Ca-ATP-ase der Herzmuskelzelle wurde nach der Methode von McNamara [J. Biochem. 75, 795 (1974)] bestimmt. Im Laufe von in vitro Versuchen wurde die Enzymaktivität von einem Kontrollwert von $K_a$ = 0,83 mM zu $K_a$ = 1,8 mM durch eine $10^{-4}$ M Konzentration der Verbindung Nr. 22 verändert; dies bedeutet einen niedrigen Effekt.

Im Laufe weiterer Versuche wurde die Verbindung Nr. 22 für 5 Tage in einer täglichen i.p. Dosis von 2 mg/kg verabreicht. Aufgrund der aus den behandelten Tieren genommenen ex vivo Proben wurde gefunden, dass die Aktivität der Ca-ATP-ase unter Wirkung der obigen Behandlung um 30 % (korrigiert auf 1 g Herzgewebe) erniedrigt wurde.

Wirkung der Verbindung Nr. 22 auf die Aufnahme von $^{45}Ca^{2+}$-Ionen in das Sarkoplasma-Reticulum in der Gegenwart von ATP

Die Methode von S. Harigaya [Circ. Res. 25, 761 (1969)] wurde verwendet.

Die Aufnahme der $Ca^{2+}$-Ionen in das Sarkoplasma-Reticulum wurde um 25 % durch eine Konzentration von 5 x $10^{-3}$ M der Verbindung Nr. 22 vermindert. Das entspricht derselben Wirkung, die Coffein in einer Konzentration von $10^{-2}$ M ausübt.

Wirkung der Verbindung Nr. 22 auf den Gehalt an cyclischem Adenosin-monophosphat (cAMP) der Herzmuskelzellen

[Für die Methode siehe: Amer. J. Sci. 179, 807 (1973); sowie Clin. Chim. Acta 66, 221 (1974)].

Eine i.p. Dosis von 5 mg/kg der Verbindung Nr. 22 wurde Ratten verabreicht. 0, 2, 5, 15 und 30 Minuten nach dieser Behandlung wurden die Tiere getötet. Ihr Herz wurde mit Trichloressigsäure homogenisiert und der cAMP-Gehalt mittels eines spezifischen Bindeproteins durch radioaktive Methode bestimmt. Der cAMP-Gehalt des Herzens wurde durch die Verbindung Nr. 22 nicht wesentlich beeinflusst.

Wirkung der Verbindung Nr. 22 auf die Phosphodiesterase(PDE-)-Aktivität des Herzmuskels

$PDE_1$-Enzym wurde mittels der Methode von Sharma und Wang [Adv. Cycl. Nucleotide Res. 10, 187 (1979)] isoliert. $PDE_2$ [Adv. Cycl. Nucl. Res. 5, 159 (1975)] und $PDE_3$ [Adv. Cycl. Nucl. Res. 10, 69 (1979)] wurden gemäss Thompson und Mitarb. isoliert. Die Enzymaktivität wurde mittels der Methode derselben Autoren [Adv. Cycl. Nucl. Res. 5, 161 (1975)] bestimmt.

Die Aktivität von keinem der PDE-Enzyme wurde durch eine Konzentration von 100 $\mu$M der Verbindung Nr. 22 beeinflusst.

Aufgrund der Ergebnisse der obigen Versuche übt die Verbindung Nr. 22 keine für die Herzglykoside typisch charakteristischen Enzymwirkungen (auf die Na-K-ATP-ase- oder NADH-Oxydase Enzyme) aus und beeinflußt nicht die Wirkungsweise der vegetativen Rezeptor-Agonisten (d. h. die Funktion des Adenylatzyclase -Enzyms).

Abweichend von einigen Xanthinderivaten oder von den neuen positiv inotropen Verbindungen des Bipyridin-Typs (z. B. Amrinon) beeinflusst die Verbindung Nr. 22 die Funktion der PDE-Isoenzyme nicht.

Die akute Toxizität der Verbindung Nr. 22 wurde an Mäusen und Ratten geprüft. Die $LD_{50}$-Werte sind in der Tabelle 2 zusammengefasst.

Tabelle 2

| | | LD$_{50}$ Werte in mg/kg | |
|---|---|---|---|
| | | männliche Tiere | weibliche Tiere |
| An Mäusen | i.v. | 323,9 (312,08-336,29) | 339,89 (331,7-348,2) |
| | i.p. | 572,23 (524,17-624,7) | 573,08 (520,09-631,47) |
| | p.o. | 1355,9 (1192,7-1541,4) | 1419,6 (1313,3-1534,4) |
| An Ratten | i.v. | 253,45 (235,18-273,14) | 232,3 (209,4-257,7) |
| | i.p. | 315,0 (277,28-357,8) | 260,0 (201,55-335,4) |
| | p.o. | 829,6 (722,5-952,6) | 809,9 (724,7-901,4) |

Bemerkung: Die Zahlen in den Klammern sind die Grenzen der Zuverlässigkeit.

Aufgrund der Angaben der Tabelle 2 ist die Toxizität der Verbindung Nr. 22 niedrig.

Gemäss den Ergebnissen der pharmakologischen Versuche sind die Verbindungen der allgemeinen Formel I zur Behandlung von Kreislaufkrankheiten, vor allem zur Steigerung der Leistung der ungenügend wirkenden Herzmuskulatur (d. h. zur Behandlung der chronischen Herzinsuffizienz) und zur Verbesserung des Koronarkreislaufes des Herzens geeignet. Ihre Toxizität ist im allgemeinen niedrig. Alle diese Eigenschaften bedeuten ein wertvolles Wirkungsspektrum und therapeutische Sicherheit.

Für therapeutische Zwecke werden die erfindungsgemässen Wirkstoffe im allgemeinen in einer täglichen Dosis zwischen 0,2 mg/kg und 250 mg/kg, Körpergewicht, vorzugsweise zwischen 0,2 mg/kg und 50 mg/kg, gegebenenfalls täglich auf mehrere Teile verteilt und mit Rücksicht auf die Resorptionsbedingungen verwendet.

Zur therapeutischen Anwendung werden die erfindungsgemässen Wirkstoffe zweckmässig zu pharmazeutischen Präparaten zubereitet, indem sie mit den in der Arzneimittelherstellung üblichen, zur enteralen oder parenteralen Applikation geeigneten nichttoxischen, inerten, festen und/oder flüssigen Träger- und oder Hilfsstoffen vermischt werden. Geeignete Trägerstoffe sind z. B. Wasser, Gelatine, Lactose, Stärke Pektin, Magnesium-stearat, Stearinsäure, Talk und pflanzliche Öle. Als Hilfsstoffe können z. B Konservierungs- und Netzmittel (oberflächenaktive Substanzen) sowie Emulgier-, Dispergiermittel. Puffersubstanzen und Aromatisiermittel verwendet werden.

Die erfindungsgemässen Wirkstoffe können mittels der obigen Träger- und/oder Hilfsstoffe in die üblichen pharmazeutischen Präparate, z.B. in feste Arzneiformen (wie Tabletten, Kapseln, Pillen und Suppositorien) oder in flüssige Formen (wie wäßrige oder ölige Lösungen, Suspensionen, Emulsionen oder Sirupe) sowie in die zu Injektionszwecken geeignete Lösungen, Suspensionen und Emulsionen überführt werden.

Als bei der Variante a) des erfindungsgemäßen Verfahrens als Ausgangsverbindungen dienende Pyrimido[5,4-b][1,4]oxazinderivate der allgemeinen Formel III werden vorzugsweise solche, bei welchen $R_{10}$ für ein Chloratom oder einen 4-Toluolsulfonyloxyrest steht, eingesetzt.

Ein Teil dieser Pyrimido[5,4-b][1,4]oxazinderivate, bei welchen $R_{10}$ für ein Chloratom steht, ist aus dem Schrifttum bekannt (Him. Geterotsikl. Soed. 1972, 1 985). Die nicht bekannten von diesen Verbindungen sind mittels im Schrifttum beschriebener oder ähnlicher Verfahren herstellbar.

Die bei der Variante a) des erfindungsgemäßen Verfahrens als Ausgangsverbindungen dienenden Pyrimido[5,4-b][1,4]oxazinderivate der allgemeinen Formel III, bei welchen $R_{10}$ einen Alkylsulfonyloxy- oder Arylsulfonyloxyrest bedeutet, sind neu und können zum Beispiel in der Weise hergestellt werden, daß Pyrimido[5,4-b][1,4]oxazinderivate der allgemeinen Formel I mit der Abwandlung, daß $R_2$ für eine Hydroxygruppe steht, mit den entsprechenden Arylsulfonylchloriden in einem basischen Medium umgesetzt werden. Dieses Verfahren wird im weiter unten folgenden Beispiel 23 näher beschrieben.

Auch die bei der Variante a) des erfindungsgemäßen Verfahrens als Ausgangsverbindungen dienenden Pyrimido[5,4-b][1,4]oxazinderivate der allgemeinen Formel III, bei welchen $R_{10}$ einen Methylsulfonylrest bedeutet, sind neu und können in der Weise hergestellt werden, daß man Pyrimido[5,4-b][1,4]oxazinderivate der allgemeinen Formel III, bei welchen $R_{10}$ für einen Methylthiorest steht, in an sich bekannter Weise oxydiert werden. Dieses Verfahren wird im weiter unten folgenden Beispiel 26 näher beschrieben. Ein Teil dieser Methylthioderivate ist bekannt (Him. Geterotsikl. Soed. 1973, 171). Die neuen Methylthioverbindungen sind mittels bekannter Verfahren oder mittels den bekannten Verfahren ähnlicher Verfahren herstellbar.

Ein überwiegender Teil der gegebenenfalls eine Schutzgruppe aufweisenden, Verbindungen der allgemeinen Formel V, die als Ausgangsverbindungen zum gegebenenfalls erfolgenden Umsetzen der Pyrimido-[5,4-b][1,4]oxazinderivate der allgemeinen Formel I, bei welchen $R_9$ für ein Wasserstoffatom steht, nach der Gegebenenfallsvariante $\alpha$) des erfindungsgemäßen Verfahrens benötigt werden, ist bekannt (siehe zum Beispiel E. H. Rodd: Chemistry of Carbon Compounds, Elsevier Publishing Co., London, 1951, Bd. 1/A Seite 615). Die neuen von ihnen können mittels bekannter Verfahren oder mittels den bekannten Verfahren ähnlicher Verfahren hergestellt werden.

Gegenstand der Erfindung sind daher auch die neuen Pyrimido[5,4-b][1,4]oxazinderivate der allgemeinen Formel

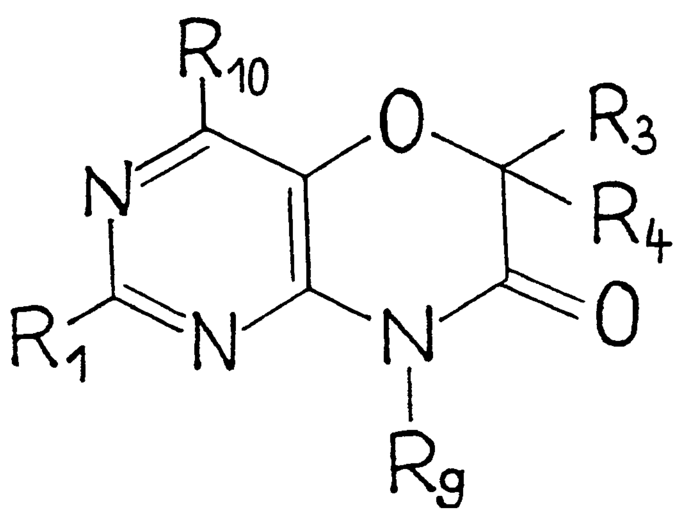

III ,

worin

$R_{10}$ für einen Alkylsulfonyloxy- oder Arylsulfonyloxyrest oder Methylsulfonylrest steht und

$R_1$, $R_3$, $R_4$ und $R_9$ die oben angegebenen Bedeutungen haben.

Die Eigenschaften dieser neuen Verbindungen sind nämlich die Ursache für die überlegenen pharmakologischen Wirkungen der aus ihnen herstellbaren erfindungsgemäßen Endprodukte Pyrimido[5,4-b][1,4]-

oxazinderivate der allgemeinen Formel I beziehungsweise Säureadditionssalze derselben.

Die Erfindung wird an Hand der folgenden Beispiele näher erläutert.

Beispiel 1

4-[4'-(Ethoxycarbonyl)-piperazin-1'-yl]-2-[methyl]-6,7-di-[hydro]-8H-pyrimido[5,4-b][1,4]oxazin-7-on

Das Gemisch von 1 g 4-[Chlor]-2-[methyl]-6,7-di-[hydro]-8H-pyrimido[5,4-b][1,4]oxazin-7-on (Him. Geterotsikl. Soed. 1976, 681), 1,58 g 1-(Ethoxycarbonyl)-piperazin und 10 ml n-Butanol wird 13 Stunden lang unter Schutz gegen Feuchtigkeit und Kohlendioxyd gekocht, dann eingedampft. Der Rückstand wird mit 20 ml Wasser zerrieben, über Nacht stehen gelassen, der Niederschlag wird abfiltriert, mit Wasser und Äther gewaschen und getrocknet. 1,4 g (87%) Produkt werden erhalten, Schmelzpunkt: 197-199°C.

Beispiel 2

2,6-Di-[methyl]-4-[4'-⟨2''(hydroxy)-ethyl⟩-piperazin-1'-yl]-6,7-di[hydro]-8H-pyrimido[5,4-b][1,4]oxazin-7-on

Das Gemisch von 0,86 g 4-Chlor-2,6-dimethyl-6,7-dihydro-8H-pyrimido[5,4-b][1,4]oxazin-7-on (Him. Geterotsikl. Soed. 1972, 1285), 1,04 g 1-[2'-(Hydroxy)-ethyl]-piperazin und 20 ml n-Butanol wird 7 Stunden lang gekocht und dann eingedampft. Der Rückstand wird mit 20 ml Wasser gemischt und mit Äthylacetat extrahiert. Die organische Phase wird getrocknet und eingedampft. Nach Umkristallisieren des Rückstandes aus Äthylacetat gewinnt man 0,90 g Produkt, Schmelzpunkt: 160-161 °C.

Die Verbindungen der allgemeinen Formel (I), worin $R_1$ Methylgruppe und $R_9$ Wasserstoff ist, wurden ähnlich wie im Beispiel 1 oder 2 beschrieben hergestellt und in der Tabelle 3 zusammengefasst.

Tabelle 3

| Beispiel Nr. | $R_2$ | $R_3$ | $R_4$ | Methode (Nr. des Typusbeispieles) | Ausbeute % | Schmelzpunkt (Base) °C |
|---|---|---|---|---|---|---|
| 3 | Cyclopropylamino | H | H | 1 | 60 | 224-226* |
| 4 | [2-(Hydroxy)-ethyl]-amino | $CH_3$ | H | 1 | 49 | 195-197 |
| 5 | bis-[2-(Hydroxy)-ethyl]-amino | $CH_3$ | H | 2 | 57 | 155-157 |
| 6 | N-[Methyl]-N-[2-(hydroxy)-ethyl]-amino | H | H | 1 | 92 | 156-158 |
| 7 | [2-(Mercapto)-ethyl]-amino | $CH_3$ | H | 2 | 52 | 185-187 |
| 8 | N-[Methyl]-N-[aminocarbonylmethyl]-amino | H | H | 1 | 82 | 255-257 |
| 9 | [2-(4'-⟨Fluor⟩-phenoxy)-ethyl]-amino | $CH_3$ | H | 1 | 64 | 140-141 |
| 10 | [2-(3',4'-Di-⟨methoxy⟩-phenyl)-ethyl]-amino | $CH_3$ | H | 1 | 74 | 148-150 |
| 11 | N-[Benzyl]-N-[2'-(hydroxy)-ethyl]-amino | H | H | 1 | 77 | 136-138 |
| 12 | [2-(Morpholin-4'-yl)-ethyl]-amino | $CH_3$ | H | 2 | 53 | 152-154 |
| 13 | [2-(Morpholin-4'-yl)-ethyl]-amino | $CH_3$ | $CH_3$ | 2 | 71 | 148-149 |
| 14 | 4-[2'-(Hydroxy)-ethyl]-piperazin-1-yl | H | H | 1 | 68 | 187-188 |
| 15 | [2-(Furyl)-methyl]-amino | $CH_3$ | H | 1 | 54 | 142-144 |
| 16 | {2-Benzo[1,4]dioxanyl}-methylamino | H | H | 2 | 37 | 165-167* |
| 17 | [4-(Chlor)-benzyl]-amino | $CH_3$ | H | 1 | 83 | 171-173 |
| 18 | [2-(Phenoxy)-ethyl]-amino | $CH_3$ | H | 1 | 57 | 162-164 |

Anmerkung: * Hydrochlorid

Beispiel 19

4-[(2'-(Hydroxy)-ethyl)-amino]-2-[methyl]-6,7-di-[hydro]-8H-pyrimido[5,4-b][1,4]oxazin-7-on

Eine Lösung von 2 g Produkt des Beispieles 11 in 60 ml Methanol werden in der Gegenwart von 1,2 g 10 gew.-%-iger Palladiumkohle bei Zimmertemperatur und Atmosphärendruck hydriert, dann 15 Minuten

lang gekocht und noch warm filtriert. Das Filtrat wird zu 35 ml eingedampft, über Nacht auf 0 bis 4 °C gekühlt, der Niederschlag abfiltriert, mit Methanol gewaschen und getrocknet. 1,26 g (90 %) Produkt werden erhalten, Schmelzpunkt: 192-194 °C.

Beispiel 20

4-[(2'-(Di-{ethyl}-amino)-ethyl)-amino]-2-[methyl]-6,7-di-[hydro]-8H-pyrimido[5,4-b][1,4]oxazin-7-on-dihydrochlorid

Das Gemisch von 2 g 4-[Chlor]-2-[methyl]-6,7-di-[hydro]-8H-pyrimido[5,4-b][1,4]oxazin-7-on, 1,52 g Triäthylamin, 1,16 g 2-Diäthylamino-äthylamin und 50 ml abs. Benzol wird 20 Stunden lang unter Rühren gekocht und dann eingedampft. Der Rückstand wird mit Wasser gemischt, auf pH-3 eingestellt und mit Chloroform extrahiert. Die wässrige Phase wird auf pH = 9 eingestellt, mit Chloroform extrahiert, die letztere organische Phase mit Wasser gewaschen, getrocknet und eingedampft. Der ölige Rückstand wird in wenig Äthanol aufgenommen und mit äthanolischer Chlorwasserstofflösung umgesetzt. Nach Filtrieren des Niederschlages gewinnt man 0,56 g (16 %) Produkt, Schmelzpunkt: 252-255 °C.

Beispiel 21

2-[Methyl]-4-[(2'-(piperid-1''yl)-ethyl)-amino]-6,7-di-[hydro]-8H-pyrimido[5,4-b][1,4]oxazin-7-on-dihydrochlorid

Man geht wie im Beispiel 20 beschrieben vor, doch verwendet man 1,23 g 2-(piperid-1'-yl)-ethylamin. So gewinnt man 0,91 g (25 %) Produkt, Schmelzpunkt: 135-140 °C.

Beispiel 22

2-Methyl-4-[(2'-(morpholin-4''-yl)-ethyl)-amino]-6,7-di-[hydro]-8H-pyrimido[5,4-b][1,4]oxazin-7-on-dihydrochlorid

Man geht wie im Beispiel 20 beschrieben vor, man verwendet aber 1 g 4-[Chlor]-2-[methyl]-6,7-di-[hydro]-8H-pyrimido[5,4-b][1,4]oxazin-7-on, 1,01 g Triäthylamin und 0,65 g 2-(Morpholin-4'-yl)-ethylamin und kocht das Gemisch 40 Stunden lang. 0,46 g (25 %) Produkt wird gewonnen, Schmelzpunkt: 233-237 °C (unter Zersetzung).

Beispiel 23

2-[Methyl]-4-[(2'-(morpholin-4''-yl)-ethyl)-amino]-6,7-di-[hydro]-8H-pyrimido[5,4-b][1,4]oxazin-7-on-dihydrochlorid

Methode A)

Das Gemisch von 50 g 2-[Methyl]-4-[4'-toluolsulfonyloxy]-6,7-di-[hydro]-8H-pyrimido[5,4-b][1,4]oxamin-7-on, 20,73 g wasserfreiem Kaliumcarbonat, 19,5 g 2-(Morpholin-4'-yl)-ethylamin und 1 250 ml abs. Ethylacetat wird 72 Stunden lang bei 20 °C gerührt, dann wird der unlösliche Teil abfiltriert und das Filtrat mit 1N wässriger Salzsäure extrahiert. Der pH-Wert der wässrigen Phase wird auf 5 eingestellt und mit Dichlormethan extrahiert. Der pH-Wert der wässrigen Phase wird dann auf 10 eingestellt und mit Dichlormethan extrahiert. Die aus der letzteren Extraktion erhaltene organische Phase wird getrocknet und eingedampft. Der Rückstand, d.h. die rohe Base wird in Aceton gelöst und unter Kühlung mit einer abs. äthanolischen Chlorwasserstofflösung umgesetzt. Der Niederschlag wird abfiltriert und mit Aceton gewaschen. 27,31 g (50 %) Produkt werden erhalten, Schmelzpunkt: 233-237 °C (unter Zersetzung).

Methode B)

Das Gemisch von 5 g 2-[Methyl]-4-[4'-toluolsulfonyloxy]-6,7-di-[hydro]-8H-pyrimido[5,4-b][1,4]oxazin-7-on, 2,99 g 2-(Morpholin-4'-yl)-ethylamin, 9,11 g Triäthylamin und 130 ml abs. Ethylacetat wird 5 Stunden lang gekocht und dann filtriert. Das Filtrat wird eingedampft, der Rückstand mit Wasser gemischt und der pH-Wert auf 2 eingestellt. Das Gemisch wird mit Ethylacetat extrahiert und der pH-Wert der wässrigen Phase wird auf 5 eingestellt. Im weiteren arbeitet man wie in der Methode A) beschrieben, und man erhält

3,28 g (60 %) Produkt, Schmelzpunkt: 233-237 °C (unter Zersetzung).

Das Fumarat-Salz der obigen Base wird wie folgt hergestellt.

Zur Lösung von 1 g nach der Methode A) oder B) erhaltener roher Base in 9 ml Ethanol gibt man eine auf 70 °C erwärmte Lösung von 0,2 g Fumarsäure in 5 ml Äthanol bei 70 °C unter Rühren und nach Abkühlung hält man das Gemisch über Nacht auf 0-4 °C. So gewinnt man das Fumarat mit einer fast quantitativer Ausbeute, Schmelzpunkt: 215-216 °C. Dieses Fumarat enthält die Base in einem Verhältnis 2:1 zur Fumarsäure.

| | | | | |
|---|---|---|---|---|
| Analyse: | für $C_{30}H_{42}N_{10}O_{10}$ | (M = 702,72) | | |
| | berechnet: | C 51,27; | H 6,03; | N 19,93 %; |
| | gefunden: | C 51,63; | H 5,96; | N 19,06 %. |

Der Fumarsäure-Gehalt wurde als 16,50 % gefunden (berechnet 16,52 %).

2-[Methyl]-4-[4'-toluolsulfonyloxy]-6,7-di-[hydro]-8H-pyrimido[5,4-b][1,4]-oxazin-7-on, die gemeinsame Ausgangsverbindung beider Methoden A) und B) kann z. B. wie folgt hergestellt werden:

Die Lösung von 157,2 g 4-Toluolsulfonylchlorid in 450 ml Aceton wird während 3 Stunden zur Lösung von 135,8 g 4-[Hydroxy]-2-[methyl]-6,7-di-[hydro]-8H-pyrimido-[5,4-b][1,4]oxazin-7-on (Him. Geterotsikl. Soed. 1976, 681) in 1205 ml 1N wässriger Natronlauge unter Rühren bei Raumtemperatur zugegeben und das Gemisch wird 5 Stunden lang bei Raumtemperatur weitergerührt. Die Kristalle werden abfiltriert, mit Wasser gewaschen und in 1800 ml Aceton aufgenommen. Der unlösliche Teil wird filtriert und mit Aceton gewaschen. Die Acetonlösung wird eingedampft, der Niederschlag abfiltriert, mit wenig Aceton gewaschen und getrocknet. 123,2 g (49 %) Produkt werden gewonnen, Schmelzpunkt: 185-186 °C.

Die in der folgenden Tabelle 4 aufgeführten Verbindungen wurden im wesentlichen gemäss der Methode A) des Beispieles 23 hergestellt.

## Tabelle 4

Verbindungen der allgemeinen Formel I, worin $R_1$ für Methylgruppe und $R_3$, $R_4$ sowie $R_5$ für Wasserstoff stehen

| Beispiel Nr. | $R_2$ | Ausbeute % | Schmelzpunkt °C (Salzform) |
|---|---|---|---|
| 24 | [2-⟨2'-(Methyl)-morpholin-4'-yl⟩-ethyl]-amino | 18 | 186-190 (Dihydrochlorid) |
| 25 | [2-⟨4'-(Methyl)-piperazin-4'-yl⟩-ethyl]-amino | 69 | 193-195 (Dimaleat) |

Beispiel 26

2-[Methyl]-4-[⟨2'-(morpholin-4''-yl)-ethyl⟩-amino]-6,7-di-[hydro]-8H-pyrimido[5,4-b][1,4]oxazin-7-on-dihydrochlorid

Das Gemisch von 0,34 g 4-[Methansulfonyl]-2-[methyl]-6,7-di-[hydro]-8H-pyrimido[5,4-b][1,4]oxazin-7-on und 0,40 g 2-(Morpholin-4'-yl)-ethylamin wird 10 Minuten lang auf 75-80 °C erwärmt, nach Abkühlung in 8 ml 1N Natronlauge aufgenommen und mit Dichlormethan erschöpfend extrahiert. Die organische Lösung wird mit Wasser ausgeschüttelt, getrocknet, filtriert und eingedampft. So erhält man 0,21 g (52,5 %) einheitliche Rohbase, die man auf die im Beispiel 22 oder 23 beschriebene Weise in das Dihydrochlorid umwandelt. Die Rohbase kann durch Rühren mit Äthanol oder Aceton kristallisiert werden, Schmelzpunkt: 143-145 °C.

Die Ausgangsverbindung 4-[Methansulfonyl]-2-[methyl]-6,7-di-[hydro]-8H-pyrimido[5,4-b][1,4]oxazin-7-on kann z. B. wie folgt hergestellt werden:

2 ml 30 %-ige Wasserstoffperoxyd-Lösung werden zum Gemisch von 0,83 g 2-[Methyl]-4-[methylthio]-6,7-di-[hydro]-8H-pyrimido[5,4-b][1,4]oxazin-7-on (Him. Geterotsikl. Soed. 1973, 171) und 8 ml Essigsäure zugegeben und 3 Stunden lang bei 60 °C gerührt. Die Lösung wird zur Trockne eingedampft, nach Entfernung von Spuren der Essigsäure wird der kristalline Rückstand mit 5 ml Wasser zerrieben und der pH-Wert auf 6,5 eingestellt. Der Niederschlag wird abfiltriert und getrocknet. So gewinnt man 0,36 g (38 %) der Ausgangsverbindung des Beispieles 26, Schmelzpunkt: 211 °C.

Beispiel 27

4-[⟨4'-(Benzyl)-morpholin-2'-yl)-methyl⟩-amino]-2-[methyl]-6,7-di-[hydro]-8H-pyrimido[5,4-b][1,4]oxazin-7-on-dihydrochlorid-dihydrat

Methode A)

Die Suspension von 16,1 g 2-[Methyl]-4-[4'-toluolsulfonyloxy]-6,7-di-[hydro]-8H-pyrimido[5,4-b][1,4]-oxazin-7-on (Beispiel 23), 8,93 g 2-[Aminomethyl]-4-[benzyl]-morpholin und 6,6 g wasserfreiem Kaliumcarbonat in 400 ml abs. Äthylacetat wird 80 Stunden lang bei Raumtemperatur gerührt, dann wird der unlösliche Teil abfiltriert, die Lösung mit 1N wässriger Salzsäure extrahiert, der pH-Wert der wässrigen Phase mit 1N Natronlauge auf 7 eingestellt und mit Äther extrahiert. Die Ätherlösung wird getrocknet und eingedampft, der Rückstand wird in abs. Äthanol gelöst und äthanolische Chlorwasserstofflösung wird in berechneter Menge zugetropft. Nach Kühlung bei 0-4 °C wird der kristalline Niederschlag abfiltriert, mit kaltem Äthanol gewaschen und getrocknet. So werden 2,33 g (15 %) Produkt gewonnen, Schmelzpunkt: 196-199 °C.

Methode B)

Die Suspension von 3 g 2-[Methyl]-4-[4'-toluolsulfonyloxy]-6,7-di-[hydro]-8H-pyrimido[5,4-b][1,4]-oxazin-7-on (Beispiel 23), 1,85 g 2-[Aminomethyl]-4-[benzyl]-morpholin 5,43 g Triäthylamin und 80 ml abs. Äthylacetat kocht man 8 Stunden lang unter Rühren und dampft ein. Den Rückstand rührt man mit 50 ml Wasser und stellt den pH-Wert mit 1N Salzsäure auf 1-2 ein. Nach Extraktion mit Äthylacetat stellt man den pH-Wert der wässrigen Phase mit 1N Natronlauge auf 7 ein und extrahiert mit Äther. Im weiteren geht man gemäss der Methode A) vor und so gewinnt man 1,0 g (13 %) Produkt, Schmelzpunkt: 195-198 °C.

2-[Aminomethyl]-4-benzyl]-morpholin, die gemeinsame Ausgangsverbindung beider Methoden A) und B) kann z. B. wie folgt hergestellt werden.

Schritt a): 4-[Benzyl]-2-[phthalimidomethyl]-morpholin

50,2 g Phthalimid-Kalium werden zur Lösung von 61,2 g 4-[Benzyl]-2-[chlormethyl]-morpholin [Synth. Com. 10, 59 (1980)] in 150 ml abs. Dimethylformamid zugegeben und das Gemisch wird 6 Stunden lang unter Rühren gekocht. Nach Abkühlen filtriert man die Kristalle ab, wäscht mit Dimethylformamid und dann mit Wasser und trocknet sie. So werden 68,95 g (76 %) Produkt gewonnen, Schmelzpunkt: 130-132 °C.

Schritt b): 4-[Benzyl]-2-[aminomethyl-morpholin-dihydrochlorid

Das Gemisch von 19,3 g Produkt des Schrittes a) mit 38 ml konzentrierter Salzsäure wird 14 Stunden lang unter Rühren gekocht. Nach Abkühlen wird die ausgeschiedene Phthalsäure abfiltriert und die wässrige Lösung durch Ausschütteln mit thylacetat von der Phthalsäure völlig befreit. Die wässrige Phase wird zur Trockne eingedampft, der Rückstand mit abs. Äthanol zerrieben, die Kristalle werden abfiltriert, mit abs. Äthanol gewaschen und getrocknet. So erhält man 10,3 g (65 %) Produkt, Schmelzpunkt: 244-246 °C.

Aus dem Dihydrochlorid kann die Base derart freigesetzt werden, dass man das Salz in Wasser löst, mit Natronlauge alkalisch macht und die Base mit Chloroform extrahiert. Nach Trocknung wird die organische Phase eingedampft und die Rohbase als Rückstand in quantitativer Ausbeute gewonnen, die in der Verfahrensweise dieses Beispieles 27 in dieser Form verwendbar ist.

Beispiel 28

8-[2',3'-Di-(hydroxy)-propyl]-4-[4'-(ethoxycarbonyl)-piperazin-1'-yl]-2-[methyl]-6,7-di-[hydro]-8H-pyrimido[5,4-b][1,4]oxazin-7-on

Die Suspension von 0,96 g 4-[4'-(Ethoxycarbonyl)-piperazin-1'-yl]-2-[methyl]-6,7-di-[hydro]-8H-pyrimi do[5,4-b][1,4]oxazin-7-on (Beispiel 1), 0,44g 2,3-Epoxypropanol, 0,0966 g Tetra-(n-butyl)-ammoniumbromid und 20 ml abs. Benzol wird 5 Stunden lang unter Rühren gekocht und dann über Nacht bei Raumtemperatur stehen gelassen. Der Niederschlag wird abfiltriert, mit Diisopropyl-äther gewaschen und getrocknet. So gewinnt man 0,98 g (83 %) Produkt, Schmelzpunkt: 146-148 °C.

Beispiel 29

4-[(2'-(Hydroxy)-ethyl)-amino]-2-[methyl]-8-[2''-(oxo)-propyl]-6,7-di-[hydro]-8H-pyrimido[5,4-b][1,4]oxazin-7-on

Die Lösung von 0,31 g Chloraceton in 5 ml Butanon wird einer Suspension von 0,68 g 4-[(2'-(Hydroxy)-ethyl)-amino]-2-[methyl]-6,7-di-[hydro]-8H-pyrimido[5,4-b][1,4]oxazin-7-on (Beispiel 19), 0,5 g wasserfreiem Kaliumcarbonat und 15 ml Butanon zugetropft, dann wird das Gemisch 8 Stunden lang unter Rühren gekocht und noch warm filtriert. Das Filtrat wird auf 12 ml eingedampft und über Nacht auf 0-4 °C gehalten. Der Niederschlag wird abfiltriert, mit Äther gewaschen und getrocknet. Man erhält 0,70 g (83 %) Produkt, Schmelzpunkt: 150-152 °C. Das Hydrochlorid schmilzt bei 185-187 °C.

Beispiel 30

8-[Cyanomethyl]-2-[methyl]-4-[(2'-(morpholin-4''-yl)-ethyl)- amino]-6,7-di-[hydro]-8H-pyrimido[5,4-b][1,4]-oxazin-7-on

Eine Lösung von 0,25 g Chloracetonitril in 50 ml Butanon wird einer Suspension von 0,88 g 2-[Methyl]-4-[(2'-(morpholin-4''-yl)-ethyl)-amino]-6,7-di-[hydro]-8H -pyrimido[5,4-b][1,4]oxazin-7-onbase (hergestellt z. B. wie im Beispiel 23), 0,5 g wasserfreiem Kaliumcarbonat und 15 ml Butanon während 15 Minuten unter Rühren bei Siedetemperatur zugetropft. Dann wird das Gemisch 8 Stunden lang unter Rühren gekocht, der Niederschlag wird noch warm abfiltriert und mit Butanon gewaschen. Das mit der Waschflüssigkeit vereinigte Filtrat wird eingedampft und der Rückstand mit Diisopropyl-äther gründlich durchgerührt. Das feste Produkt wird abfiltriert, mit Diisopropyl-äther gewaschen, und dann getrocknet. Man erhält 0,91 g (91%) Produkt, Schmelzpunkt: 126-128°C.

Beispiel 31

8-[Aminocarbonylmethyl]-4-[4'-(ethoxycarbonyl)-piperazin-1'-yl]-2-[methyl]-6,7-di-[hydro]-8H-pyrimido[5,4-b]-[1,4]oxazin-7-on

Die Suspension von 0,96 g 4-[4'-Ethoxycarbonyl)-piperazin-1'-yl]-2-[methyl]-6,7-di-[hydro]-8H-pyrimido-[5,4-b][1,4]-oxazin-7-on (Beispiel 1), 0,5g wasserfreiem Kaliumcarbonat, 0,31 g Chloracetamid und 25 ml Butanon wird 8 Stunden lang unter Rühren gekocht, noch warm filtriert, der Niederschlag wird mit Butanon gewaschen und das hier gewonnene Filtrat beiseite gelegt. Der Niederschlag wird mit Wasser und dann mit Äther gewaschen und getrocknet. So gewinnt man 0,65 g Produkt, Schmelzpunkt: 225-227 °C.

Das beiseite gelegte Filtrat wird auf 10 ml eingedampft und über Nacht auf 0-4 °C gehalten. Die

Kristalle werden abfiltriert, gewaschen und getrocknet. So gewinnt man noch 0,20 g Produkt, Schmelzpunkt: 225-227 °C. Die Gesamtausbeute beträgt 75 %.

Beispiel 32

4-[Chlor]-8-[ethoxycarbonylmethyl]-2-[methyl]-6,7-di-[hydro]-8H-pyrimido[5,4-b][1,4]oxazin-7-on

Die Lösung von 0,41 g Chloressigsäureethylester in 5 ml Butanon wird einer Suspension von 0,6 g 4-[Chlor]-2-[methyl]-6,7-di-[hydro]-8H-pyrimido[5,4-b][1,4]oxazin-7-on, 0,5 g wasserfreiem Kaliumcarbonat und 15 ml Butanon während 15 Minuten bei Siedetemperatur unter Rühren zugetropft, dann 8 Stunden lang unter Rühren gekocht, noch warm filtriert und der Niederschlag mit Butanon gewaschen. Das Filtrat wird auf 10 ml eingedampft und die nach Kühlung abgeschiedene unveränderte Ausgangssubstanz wird abfiltriert. Die Mutterlauge wird eingedampft, der Rückstand mit n-Hexan zerrieben, das Produkt abfiltriert, mit n-Hexan gewaschen und getrocknet. Man gewinnt 0,59 g (68 %) Produkt, Schmelzpunkt: 69-70 °C.

Die in der Tabelle 5 aufgeführten Verbindungen wurden mittels der in den Beispielen 28 bis 32 beschriebenen Methoden hergestellt.

Tabelle 5

Verbindungen der allgemeinen Formel I, worin $R_1$ für einen Methylrest steht

| Beispiel Nr. | $R_2$ | $R_3$ | $R_4$ | $R_9$ | Methode (Nr. des Typusbeispieles) | Ausbeute % | Schmelzpunkt °C |
|---|---|---|---|---|---|---|---|
| 33 | Cl | H | H | $CH_2CN$ | 30 | 79 | 147-149 |
| 34 | Cl | H | H | $CH_2COCH_3$ | 29 | 76 | 105-107 |
| 35 | Cl | $CH_3$ | $CH_3$ | $CH_2CN$ | 32 | 80 | 110-111 |
| 36 | [2-(Hydroxy)-ethyl]-amino | H | H | $CH_2CN$ | 30 | 99 | 138-140 |
| 37 | [2-(Hydroxy)-ethyl]-amino | H | H | $CH_2CO_2C_2H_5$ | 32 | 73 | 185-187 * |
| 38 | [2-(Hydroxy)-ethyl]-amino | H | H | $CH_2CH(OH)CH_2OH$ | 28 | 99 | 128-131 |
| 39 | [2-(Hydroxy)-ethyl]-amino | H | H | $CH_2CONH_2$ | 31 | 48 | 222-224 |
| 40 | N-[Methyl]-N-[2-(hydroxy)-ethyl]-amino | H | H | $CH_2CN$ | 30 | 96 | 90-92 |
| 41 | N-[Methyl]-N-[2-(hydroxy)-ethyl]-amino | H | H | $CH_2CO_2C_2H_5$ | 32 | 63 | 134-137 * |
| 42 | N-[Methyl]-N-[2-(hydroxy)-ethyl]-amino | H | H | $CH_2COCH_3$ | 29 | 39 | 190-192 |
| 43 | N-[Methyl]-N-[aminocarbonylmethyl]-amino | H | H | $CH_2CO_2C_2H_5$ | 32 | 25 | 142-145 |
| 44 | Cl | H | H | $CH_2CONH_2$ | 31 | 66 | 253-255 |
| 45 | N-(Morpholin-4-yl) | H | H | $CH_2CN$ | 30 | 69 | 158-161 |
| 46 | N-(Morpholin—-4-yl) | H | H | $CH_2CO_2C_2H_5$ | 32 | 75 | 117-119 |
| 47 | [2-(Moroholin-4'-yl)-ethyl]-amino | H | H | $CH_2COCH_3$ | 29 | 85 | 148-150 |
| 48 | [2-(Morpholin-4'-yl)-ethyl]-amino | H | H | $CH_2CO_2C_2H_5$ | 32 | 77 | 176-180 * |

(Fortsetzung der Tabelle 5)

| Beispiel Nr. | $R_2$ | $R_3$ | $R_4$ | $R_9$ | Methode (Nr. des Typusbeispieles) | Ausbeute % | Schmelzpunkt °C |
|---|---|---|---|---|---|---|---|
| 49 | 4-[2'-(Hydroxy)-ethyl]-piperazin-1-yl | H | H | $CH_2COC_2H_5$ | 32 | 69 | 125-128 |
| 50 | 4-[Ethoxycarbonyl]-piperazin-1-yl | H | H | $CH_2CN$ | 30 | 80 | 194-196 |
| 51 | 4-[Ethoxycarbonyl]-piperazin-1-yl | H | H | $CH_2COCH_3$ | 29 | 76 | 143-145 |
| 52 | 4-[Ethoxycarbonyl]-piperazin-1-yl | H | H | $CH_2CO_2C_2H_5$ | 32 | 79 | 105-106 |
| 53 | {(2-Benzo[1,4]dioxanyl)methyl}-amino | H | H | $CH_2CH(OH)-CH_2OH$ | 28 | 28 | 200-201 |
| 54 | N-[Benzyl]-N-[2'-(hydroxy)-ethyl]-amino | H | H | $-CH_2CN$ | 30 | 40 | 100-103 |
| 55 | N-[Benzyl]-N-[2'-(hydroxy)-ethyl]-amino | H | H | $CH_2CH(OH)-CH_2OH$ | 28 | 57 | 99-103 * |

Bemerkung: * Hydrochlorid

Beispiel 56

4-[Chlor]-8-[2',3'-di-(hydroxy)-propyl]-2-[methyl]-6,7-di-[hydro]-8H-pyrimido[5,4-b][1,4]oxazin-7-on

Das Gemisch von 10 g 4-[Chlor]-2-[methyl]-6,7-di-[hydro]-8H-pyrimido[5,4-b][1,4]oxazin-7-on, 0,7 g Triäthylamin-hydrochlorid, 7,4 g 2,3-(Epoxy)-propanol und 300 ml abs. Benzol wird 4 Stunden lang unter Rühren gekocht. Dann wird die Benzollösung zweimal mit je 100 ml Wasser gewaschen und die vereinigte wässrige Phase wird 4-mal mit je 70 ml Chloroform extrahiert. Die chloroformische Phase wird getrocknet

und das Lösungsmittel eingedampft. Der ölige Rückstand wird mit 10 ml Chloroform aufgenommen und ein Gemisch von Äther und Petroläther im Volumverhältnis von 1 : 1 wird zugegeben. Der ölige Niederschlag wird bald kristallin. Er wird abfiltriert, mit Petroläther gewaschen und getrocknet. 5,21g (38%) Produkt werden gewonnen, Schmelzpunkt: 117-118°C.

Beispiel 57

8-(2',3'-Di-(hydroxy)-propyl]-2-[methyl]-4-[morpholin-4"yl)-6,7-di[hydro]8H-pyrimido[5,4-b][1,4]oxazin-7-on

Das Gemisch von 1 g 2-[Methyl]-4-[morpholin-4'-yl)-6,7-di-[hydro]-8H-pyrimido[5,4-b][1,4]oxazin-7-on, 0,59 g 2,3-(Epoxy)-propanol, 0,128 g Tetra-(n-butyl)-ammoniumbromid und 60 ml abs. Benzol wird 7 Stunden lang unter Rühren gekocht. Nach Abkühlung wird der Niederschlag abfiltriert, mit Benzol und Äther gewaschen und getrocknet. Man gewinnt 1,04 g (77 %) Produkt, Schmelzpunkt: 153-155 °C.

Beispiel 58

8-[2',3'-Di-(hydroxy)-propyl]-2-[methyl]-4-[(2"-(morpholin-4"'-yl)-ethyl)-amino]-6,7-di-[hydro]-8H-pyrimido-[5,4-b][1,4]oxazin-7-on-dihydrochlorid

Ein Gemisch von 1 g 2-[Methyl]-4-[(2'-(morpholin-4"-yl)-ethyl)-amino]-6,7-di-[hydro]-8H-pyrimido[5,4-b]-[1,4]oxazin-7-onbase (hergestellt z.B. nach Beispiel 23), 0,50 g 2,3-(Epoxy)-propanol, 0,11 g Tetra-(n-butyl)-ammoniumbromid und 40 ml abs. Benzol wird 8 Stunden lang unter Rühren gekocht. Die Benzollösung wird mit Wasser gewaschen und die wässrige Phase mit Chloroform oder Ethylacetat extrahiert. Die organische Phase wird getrocknet und eingedampft. Der Rückstand wird in wenig abs. Ethanol gelöst und eine berechnete Menge äthanolischer Chlorwasserstofflösung wird zugegeben. Nach Kühlung wird der Niederschlag abfiltriert, gewaschen und getrocknet. Man gewinnt 0,45 g (30 %) Produkt, Schmelzpunkt: 158-160 °C.

Beispiel 59

8-[2',3'-Di-(hydroxy)-propyl]-2-[methyl]-6,7-di-[hydro]-8H-pyrimido[5,4-b][1,4]oxazin-7-on

Die Lösung von 2 g 4-[Chlor]-8-[2',3'-di(hydroxy)-propyl]-2-[methyl]-6,7-di-[hydro]-8H-pyrimido[5,4-b]-[1,4]-oxazin-7-on in 50 ml abs. Äthanol und 0,739g
Triethylamin wird in der Gegenwart von 0,20 g 5 gew`-%-iger Palladiumkohle bei Raumtemperatur und Atmosphärendruck hydriert. Dann wird der Katalysator entfernt, die Lösung wird eingedampft, der Rückstand in Wasser aufgenommen und mit Chloroform extrahiert. Die organische Phase wird getrocknet und eingedampft. Der Rückstand wird mit Petroläther gründlich durchgerührt und stehengelassen. Der Niederschlag wird abfiltriert, mit Petroläther gewaschen und getrocknet. Man gewinnt 0,33 g (19 %) Produkt Schmelzpunkt: 70-72 °C.

Beispiel 60

2-[Methyl]-4-[(3'-(morpholin-4"yl)-propyl)-amino]-6,7-di-[hydro]-8H-pyrimido[5,4-b][1,4]oxazin-7-on

Eine Lösung von 0,7 ml Triäthylamin und 1,44 g [3-(Morpholin-4'-yl)-prop-1-yl]-amin wird während 8 Stunden zum Gemisch von 2 g 4-[Chlor]-2-[methyl]-6,7-di-[hydro]-8H-pyrimido[5,4-b][1,4]oxazin-7-on, 1,4 ml Triäthylamin und 5 ml abs. Benzol unter Rühren bei Siedetemperatur zugetropft. Das Gemisch wird 8 Stunden lang unter Rühren gekocht, eingedampft, der Rückstand mit 16 ml Wasser gemischt, abfiltriert, mit Wasser und dann mit Äther gewaschen. Man erhält 1,1 g (36 %) Produkt, Schmelzpunkt: 165-168 °C.

Das Hydrochlorid gewinnt man durch Zugabe äthanolischer Chlorwasserstofflösung zu einer Lösung der Base in Ethylacetat, Schmelzpunkt: 250-251 °C.

Beispiel 61

4-[Chlor]-2-[methyl]-8-(pyrid-3'-ylmethyl)-6,7-di-[hydro]-8H-pyrimido[5,4-b][1,4]oxazin-7-on

Eine Lösung von aus 1 g 3-(Chlormethyl)-pyridin-hydrochlorid freigesetzter Base in 5 ml Butanon wird

unter Rühren zu einer Suspension von 1,0 g 4-[Chlor]-2-[methyl]-6,7-di-[hydro]-8H-pyrimido[5,4-b][1,4]-oxazin -7-on und 0,83 g wasserfreiem Kaliumcarbonat in 15 ml Butanon getropft.

Nach einem 8-stündigen Kochen filtriert man noch warm und dampft die Lösung ein. Der Rückstand wird mit Diisopropyl-äther zerrieben und abfiltriert. So gewinnt man 0,97 g (66 %) Base-Produkt, Schmelzpunkt: 116-118 °C. Das Hydrochlorid schmilzt bei 220-224 °C.

Beispiel 62

4[⟨2'-(Hydroxy)-äthyl⟩-amino]-2,6,6-tri-[methyl]-6,7-di-[hydro]-8H-pyrimido[5,4-b][1,4]oxazin-7-on

Man geht auf die im Beispiel 2 beschriebene Weise vor und gewinnt das Produkt in einer Ausbeute von 83 %, Schmelzpunkt: 168-170 °C. Das Hydrochlorid schmilzt bei 190-191 °C.

Beispiel 63

8-[Ethoxycarbonylmethyl]-4-[⟨2'-(hydroxy)-ethyl⟩-amino]-2,6-di-[methyl]-6,7-di-[hydro-8H-pyrimido[5,4-b]-[1,4]oxazin-7-on-hydrochlorid

Man geht auf die im Beispiel 32 beschriebene Weise vor und gewinnt das Produkt in einer Ausbeute von 41 %, Schmelzpunkt: 105-108 °C.

Beispiel 64

2,6-Di-[methyl)-4-[⟨1'-(methyl)-2'-(phenyl)-ethyl⟩-amino]-6,7-di-[hydro]-8H-pyrimido[5,4-b][1,4]oxazin-7-on

Man geht analog wie im Beispiel 2 beschrieben, jedoch unter Verwendung der äquivalenten Menge [1-(Methyl)-2-(phenyl)-ethyl]-amin statt des 1-[2'-(Hydroxy)-ethyl]-piperazines vor und gewinnt das Produkt in einer Ausbeute von 31 %, Schmelzpunkt: 126-130 °C. Das Hydrochlorid schmilzt bei 181-184 °C.

Beispiel 65

2,6-Di-[methyl]-8-[1'-(ethoxycarbonyl)-eth-1'-yl]-4-[morpholin-4''-yl]-6,7-di-[hydro]-8H-pyrimido[5,4-b][1,4]-oxazin-7-on

Ausgehend von 4-[Chlor]-2,6-di-[methyl]-6,7-di-[hydro]-8H-pyrimido[5,4-b][1,4]oxazin-7-on (Him. Geterotsikl. Soed. 1972, 1285) und 2-(Brom)-propionsäureethylester geht man wie im Beispiel 32 beschrieben vor, aber man kocht das Gemisch 12 Stunden lang. Nach Eindampfen setzt man den öligen Rückstand (der grösstenteils 4-[Chlor]-2,6-di-[methyl]-8-[1'-(ethoxycarbonyl)-eth-1'-yl]-6,7-di-[hydro]-8H-pyrimido[5,4-b][1,4]-oxazin-7-on enthält) analog dem Beispiel 1 mit Morpholin um und arbeitet das Reaktionsgemisch ähnlich auf mit der Ausnahme, dass der Rückstand mit Äther zerrieben und filtriert wird. Das Filtrat wird eingedampft und der Rückstand aus n-Hexan umkristallisiert. So erhält man das Produkt in einer Ausbeute von 42 %, Schmelzpunkt: 80-82 °C.

Beispiel 66

4-[⟨2'-(Hydroxy)-ethyl⟩-amino]-2-[methyl]-8-[morpholin-4''-ylcarbonylmethyl]-6,7-di-[hydro]-8H-pyrimido[5,4-b][1,4]oxazin-7-on

Man geht analog wie im Beispiel 32 beschrieben, jedoch ausgehend von der äquivalenten Menge 4-[⟨2'-(Hydroxy)-ethyl⟩- amino]-2-[methyl]-6,7-di-[hydro]-8H-pyrimido[5,4-b][1,4]oxazin-7-on statt des 4-[Chlor]-2-[methyl]-6,7-di-[hydro]-8H-pyrimido[5,4-b][1,4]oxa zin-7-ones und der äquivalenten Menge 4-(Chloracetyl)-morpholin statt des Chloressigsäureethylesters vor und gewinnt das Produkt in einer Ausbeute von 89%, Schmelzpunkt: 162-165°C.

Beispiel 67

8-[n-(Butoxy)-carbonylmethyl]-4-[⟨2'-(hydroxy)-ethyl⟩-amino]-2-[methyl]-6,7-di-[hydro]-8H-pyrimido[5,4-b]-[1,4]oxazin-7-on-hydrochlorid

Man geht analog wie in Beispiel 32 beschrieben, jedoch ausgehend von der äquivalenten Menge 4-[⟨2'-Hydroxy)-ethyl⟩-amino]-2-[methyl]-6,7-di-[hydro]-8H-pyrimido[5,4-b][1,4]oxazin-7-on statt des 4-[Chlor]-2-[methyl]-6,7-di-[hydro]-8H-pyrimido[5,4-b][1,4]oxazin-7-ones und der äquivalenten Menge Chloressigsäuren-butylester vor und gewinnt das Produkt in einer Ausbeute von 62%, Schmelzpunkt: 142-145°C.

Beispiel 68

4-[⟨4'-(Chlor)-benzyl⟩-amino]-2,6-di-[methyl]-8-ethoxycarbonylmethyl]-6,7-di-[hydro]-8H-pyrimido[5,4-b][1,4]-oxazin-7-on

Man geht analog wie im Beispiel 32 beschrieben, jedoch ausgehend von der äquivalenten Menge 4-[⟨4'-(Chlor)-benzyl⟩-amino]-2,6-di-[methyl]-8H-pyrimi do[5,4-b][1,4]oxazin-7-on statt des 4-[Chlor-2-[methyl]-6,7-di-[hydro]-8H-pyrimido[5,4-b][1,4]oxazin-7-ones vor und gewinnt das Produkt in einer Ausbeute von 62%, Schmelzpunkt: 101-104°C.

Beispiel 69

8-[Benzyl]-4-[⟨2'-(hydroxy)-ethyl⟩-amino]-2-[methyl]-6,7-di-[hydro]-8H-pyrimido[5,4-b][1,4]oxazin-7-on

Man geht wie im Beispiel 1 beschrieben, jedoch ausgehend von der äquivalenten Menge 8-[Benzyl]-4-[chlor]-2-[methyl]-6,7-di-[hydro]-8H-pyrimido[5,4-b][1,4]oxazin-7-on statt des 4-[Chlor]-2-[methyl]-6,7-di-[hydro]-8H-pyrimido[5,4-b][1,4]oxazin-7-ones und der äquivalenten Menge [2-(Hydroxy)-ethyl]-amin statt des 1-(Ethoxycarbonyl)-piperazines vor und gewinnt das Produkt in einer Ausbeute von 84%, Schmelzpunkt: 124-126°C. Das Hydrochlorid schmilzt bei 153-157°C.

Beispiel 70

4-[Hydrazino]-2-[methyl]-6,7-di-[hydro]-8H-pyrimido[5,4-b][1,4]oxazin-7-on

Eine Lösung von 12,8 ml 98 gew.-%-igem Hydrazinhydrat in 40 ml n-Butanol wird während 30 Minuten einer Lösung von 20 g 4-[Chlor]-2-[methyl]-6,7-di-[hydro]-8H-pyrimido[5,4-b][1,4]oxazin-7-on in 320 ml n-Butanol unter Rühren bei 117 °C zugetropft und dann wird das Gemisch bei derselben Temperatur 45 Minuten lang gerührt und auf Zimmertemperatur abgekühlt. Der Niederschlag wird abfiltriert und mit Wasser gewaschen. Man gewinnt 11,66 g (56,3 %) Produkt, Schmelzpunkt: 262-264 °C.

Beispiel 71

4-[Azido]-2-[methyl]-6,7-di-[hydro]-8H-pyrimido[5,4-b][1,4]oxazin-7-on

Die Lösung von 1,75 g Natriumnitrit in 14 ml Wasser wird zur Suspension von 4,90 g 4-[Hydrazino]-2-[methyl]-6,7-di-[hydro]-8H-pyrimido[5,4-b][1,4]oxazin-7-on (Beispiel 70), 55 ml Wasser und 9 ml Eisessig unter Rühren bei 0-5 °C getropft und dann wird das Gemisch 20 Minuten lang bei derselben Temperatur und 30 Minuten lang bei Zimmertemperatur gerührt. Der Niederschlag wird abfiltriert, mit Wasser, dann mit Äthanol und Äther gewaschen und getrocknet. Man erhält 4,80 g (93,2 %) Produkt, Schmelzpunkt: 214-217 °C.

Beispiel 72

4-[Amino]-2-[methyl]-6,7-di-[hydro]-8H-pyrimido[5,4-b][1,4]oxazin-7-on-hydrochlorid

25 ml 20 gew.-%-ige, auf 10°C abgekühlte Salzsäure werden während 15 Minuten zur Suspension von 4,0 g 2-[Methyl]-4-[⟨triphenylphosphoranyliden⟩-amino]-6,7-di-[hydro]-8H-pyrimido[5,4-b][1,4]oxazin-7-on in 25 ml Äthanol unter Rühren getropft. Das Gemisch wird 5 Stunden lang bei Raumtemperatur gerührt, dann wird der Niederschlag abfiltriert, mit Äthanol gewaschen und getrocknet. Man gewinnt 1,80 g (91 %) Produkt, das bis 260 °C nicht schmilzt.

Aus diesem Salz kann man die Base mittels wässriger Natriumbicarbonatlösung freisetzen.

Die Ausgangsverbindung 2-[Methyl]-4-[⟨triphenylphosphoranyliden⟩-amino]-6,7-di-[hydro]-8H-pyrimido-[5,4-b][1,4]oxazin-7-on stellt man wie folgt her:

3,50 g 4-[Azido]-2-[methyl]-6,7-di-[hydro]-8H-pyrimido[5,4-b][1,4]oxazin-7-on (Beispiel 71) werden portionsweise während 20 Minuten unter Rühren zu einer Lösung von 4,48 g Triphenylphosphin in 140 ml trockenem Dichlormethan bei Raumtemperatur zugegeben. Das Gemisch wird über Nacht bei Raumtemperatur gerührt, dann filtriert, und das Filtrat wird zur Trockne eingedampft. Der Rückstand wird in 70 ml Äther suspendiert, der Niederschlag wird abfiltriert und getrocknet. So erhält man 6,73 g (95 %) Produkt, Schmelzpunkt: 245 °C.

Beispiel 73

4-[Amino]-8-[(ethox)-carbonylmethyl]-2-[methyl]-6,7-di-[hydro]-8H-pyrimido[5,4-b][1,4]oxazin-7-on

Die Suspension von 0,90 g 4-[Amino]-2-[methyl]-6,7-di-[hydro]-8H-pyrimido[5,4-b][1,4]oxazin-7-on (Beispiel 72), 0,69 g wasserfreiem Kaliumcarbonat, 0,65 ml Chloressigsäureethylester und 20 ml Butanon wird 12 Stunden lang gekocht. Die anorganischen Salze werden abfiltriert die Lösung wird zur Trockne eingedampft und der Rückstand aus Isopropanol umkristallisiert. Man gewinnt 0,43 g (32,3 %) Produkt, Schmelzpunkt: 149-150 °C. Aus den Mutterlaugen der Umkristallisierungen kann man noch 0,22 g 16,2 % Produkt gewinnen.

Beispiel 74

Herstellung von Tabletten

| Zusammensetzung (berechnet für 1000 Tabletten) | g |
|---|---|
| Fumarat der Base gemäss dem Beispiel 22 | 10 |
| Lactose | 185 |
| Mikrokristalline Cellulose | 25 |
| Talk | 5 |
| Maisstärke | 73 |
| Magnesium-stearat | 2 |
| Insgesamt: | 300 |

Die Komponenten werden vermischt, homogenisiert und zu Tabletten gepresst, die je 10 mg des Wirkstoffes enthalten.

Beispiel 75
Herstellung einer Injektionslösung

| Zusammensetzung (berechnet für 2 Liter Lösung) | |
|---|---|
| Fumarat der Base gemäss dem Beispiel 22 | 2 g |
| Natriumchlorid | 20 g |
| Wasser für Injektionszwecke q.s. | ad 2000 ml |

Die obigen Komponenten werden gelöst, in Ampullen gefüllt und sterilisiert. Eine Ampulle enthält 2 ml Lösung.

**Patentansprüche**
**Patentansprüche für folgende Vertragsstaaten: BE,CH,DE,FR,GB,IT, LI,LU,NL,SE**

**1.** Pyrimido[5,4-b][1,4]oxazinderivate der allgemeinen Formel

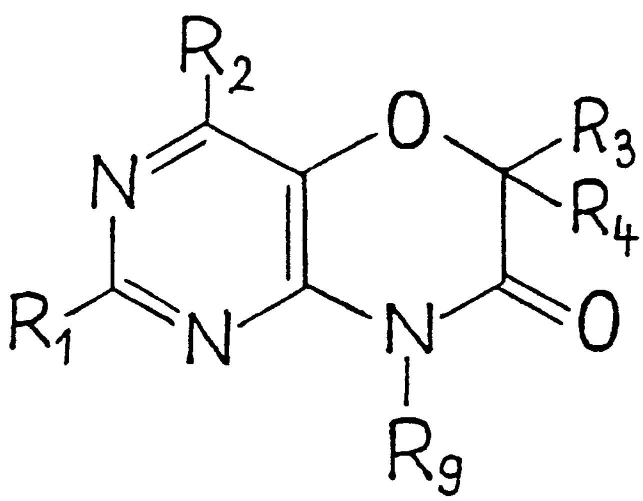

I ,

worin

$R_1$ für einen Alkylrest mit 1 bis 4 Kohlenstoffatom(en) steht,

$R_2$ ein Wasserstoffatom, ein Halogenatom, eine Azidogruppe, eine Hydrazinogruppe oder einen Rest der allgemeinen Formel

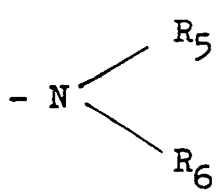

II ,

in welchletzterer

$R_5$ für ein Wasserstoffatom, einen, gegebenenfalls durch eine Hydroxygruppe substituierten, Alkylrest mit 1 bis 4 Kohlenstoffatom(en) oder Benzylrest steht und

$R_6$ ein Wasserstoffatom, eine Aminogruppe, einen Cycloalkylrest mit 3 bis 6 Kohlenstoffatomen, einen, gegebenenfalls durch eine Hydroxygruppe, eine Mercaptogruppe, einen Aminocarbonylrest, einen Furylrest, einen 2-Benzo[1,4]dioxanylrest, einen Di-(alkyl)-aminorest mit 1 bis 4 Kohlenstoffatom(en) in jedem Alkylteil oder einen, gegebenenfalls durch ein Halogenatom oder einen Alkylrest mit 1 bis 4 Kohlenstoffatom(en) oder 1 oder mehr Alkoxyrest(e) mit 1 bis 4 Kohlenstoffatom(en) substituierten, Phenyl- oder Phenoxyrest oder einen, gegebenenfalls durch einen, gegebenenfalls durch einen Alkylrest mit 1 bis 4 Kohlenstoffatom(en) oder Benzylrest substituierten, 6-gliedrigen gesättigten Stickstoff aufweisenden und gegebenenfalls außer dem Stickstoffatom noch 1 weiteres Stickstoffatom und/oder 1 Sauerstoffatom aufweisenden heterocyclischen Rest substituierten, Alkylrest mit 1 bis 4 Kohlenstoffatom(en) darstellt oder

$R_5$ und $R_6$ zusammen mit dem Stickstoffatom, an welches sie gebunden sind, einen, gegebenenfalls durch einen Alkoxycarbonylrest mit 2 bis 4 Kohlenstoffatomen oder einen Hydroxyalkylrest mit 1 bis 4 Kohlenstoffatom(en) substituierten, 6-gliedrigen gesättigten und gegebenenfalls außer dem Stickstoffatom noch 1 weiteres Stickstoffatom und/oder 1 Sauerstoffatom aufweisenden heterocyclischen Rest darstellen,

bedeutet,

$R_3$ und $R_4$, die gleich oder verschieden sein können, voneinander unabhängig für ein Wasserstoffatom oder einen Alkylrest mit 1 bis 4 Kohlenstoffatom(en) stehen und

$R_9$ ein Wasserstoffatom, einen, gegebenenfalls durch eine Oxogruppe, eine Cyanogruppe, einen Aminocarbonylrest. einen Alkoxycarbonylrest mit 2 bis 5 Kohlenstoffatomen, einen Pyridylrest, einen Morpholinylcarbonylrest oder einen Phenylrest monosubstituierten oder durch 1 oder mehr Hydroxygruppe(n) mono- oder polysubstituierten, Alkylrest bedeutet,

mit den weiteren Maßgaben, daß

a)

$R_2$ von einem Wasserstoffatom, Chloratom, einem 4-Morpholinylrest oder einem Piperidylrest verschieden ist, wenn $R_9$ für ein Wasserstoffatom steht, und

b)

$R_2$ von einem Chloratom oder einem 4-Morpholinylrest verschieden ist, wenn $R_9$ für einen Methyl- oder Benzylrest steht,

einschließlich ihrer Tautomere und ihrer Gemische sowie ihre Säureadditionssalze.

**2.** Pyrimido[5,4-b][1,4]oxazinderivate nach Anspruch 1, dadurch gekennzeichnet, daß

a) der Alkylrest, für den $R_1$ und/oder $R_3$ und/oder $R_4$ und/oder $R_5$ und/oder $R_6$ stehen kann beziehungsweise können, und/oder

b) die Alkylteile des Di-(alkyl)-aminorestes, durch welchen der Alkylrest, für welchen $R_6$ stehen kann, substituiert sein kann, oder

c) der Alkylrest oder Alkoxyrest, durch welchen der Phenyl- oder Phenoxyrest, durch den der Alkylrest, für welchen $R_6$ stehen kann, substituiert sein kann, substituiert sein kann, oder

d) der Alkylrest, durch welchen der Benzylrest, durch den der heterocyclische Rest, durch welchen der Alkylrest, für welchen $R_6$ stehen kann, jeweils substituiert sein kann oder

e) der Hydroxyalkylrest, durch den der heterocyclische Rest, den $R_5$ und $R_6$ zusammen mit dem Stickstoffatom, an welchen sie gebunden sind, darstellen können, substituiert sein kann,

[ein] solche[r] mit 1 bis 3, insbesondere 1 oder 2, Kohlenstoffatom(en) ist beziehungsweise sind und/oder

f) der Alkylrest, für den $R_9$ stehen kann,

[ein] solche[r] mit 1 bis 4, insbesondere 1 bis 3, Kohlenstoffatomen ist und/oder

g) der Alkoxycarbonylrest, durch welchen der heterocyclische Rest, den $R_5$ und $R_6$ zusammen mit dem Stickstoffatom, an welches sie gebunden sind, darstellen können, substituiert sein kann, und/oder

h) der Alkoxycarbonylrest, durch welchen der Alkylrest, für den $R_9$ stehen kann, substituiert sein

kann,
[ein] solche[r] mit 2 bis 4 Kohlenstoffatomen ist.

3. Pyrimido[5,4-b][1,4]oxazinderivate nach Anspruch 1 oder 2, dadurch gekennzeichnet, daß der Cycloalkylrest, für welchen $R_6$ stehen kann, ein solcher mit 3 bis 5, insbesondere 3 oder 4, Kohlenstoffatomen ist.

4. Pyrimido[5,4-b][1,4]oxazinderivate nach Anspruch 1 bis 3, dadurch gekennzeichnet, daß
   a) das Halogenatom, für welches $R_2$ stehen kann, oder
   b) das Halogenatom, durch welches der Phenyl- oder Phenoxyrest, durch den der Alkylrest, für welchen $R_6$ stehen kann, substituiert sein kann, substituiert sein kann,
   ein Fluor- oder Chloratom ist.

5. Pyrimido[5,4-b][1,4]oxazinderivate nach Anspruch 1 bis 3, dadurch gekennzeichnet, daß
   a) der heterocyclische Rest, durch welchen der Alkylrest, für den $R_6$ stehen kann, substituiert sein kann, oder
   b) der heterocyclische Rest, den $R_5$ und $R_6$ zusammen mit dem Stickstoffatom, an welches sie gebunden sind, darstellen können,
   ein Morpholinylrest, ein Piperidylrest oder Piperazinylrest ist.

6. 2-[Methyl]-4-[⟨2'-(morpholin-4''-yl)-ethyl⟩-amino]-6,7-di-[hydro]-8H-pyrimido[5,4-b][1,4]oxazin-7-on,

   4-[N-⟨Benzyl⟩-N-⟨2'-(hydroxy)-ethyl⟩-amino]-2-[methyl]-6,7-di-[hydro]-8H-pyrimido[5,4-b][1,4]oxazin-7-on,

   2-[Methyl]-4-[⟨3'-(morpholin-4''-yl)-propyl⟩-amino]-6,7-di-[hydro]-8H-pyrimido[5,4-b][1,4]oxazin-7-on,

   4-[Chlor]-8-[2',3'-di-(hydroxy)-propyl]-2-[methyl]-6,7-di-[hydro]-8H-pyrimido[5,4-b][1,4]oxazin-7-on,

   8-[2',3'-Di-(hydroxy)-propyl]-2-[methyl]-4-[morpholin-4''-yl]-6,7-di-[hydro]-8H-pyrimido[5,4-b][1,4]oxazin-7-on,

   4-[⟨4'-(Benzyl)-morpholin-2'-yl)-methyl⟩-amino]-2-[methyl]-6,7-di-[hydro]-8H-pyrimido[5,4-b][1,4]oxazin-7-on,

   8-[(Ethoxy-carbonylmethyl]-4-[⟨2''-(hydroxy)-ethyl⟩-amino]-2-[methyl]-6,7-di-[hydro]-8H-pyrimido[5,4-b]-[1,4]oxazin-7-on,

   4-[⟨2'-(Hydroxy)-ethyl⟩-amino]-2-[methyl]-8-[2''-(oxo)-propyl]-6,7-di-[hydro]-8H-pyrimido[5,4-b][1,4]-oxazin-7-on,

   4-[⟨2'-(Hydroxy)-ethyl⟩-amino]-2-[methyl]-8-[morpholin-4''-ylcarbonylmethyl]-6,7-di-[hydro]-8H-pyrimido-[5,4-b][1,4]oxazin-7-on,

   8-[(n-Butoxy)-carbonylmethyl]-4-[⟨2'-(hydroxy)-ethyl⟩-amino]-2-[methyl]-6,7-di-[hydro]-8H-pyrimido[5,4-b][1,4]oxazin-7-on,

   8-[Benzyl]-4-[⟨2'-(hydroxy)-ethyl⟩-amino]-2-[methyl]-6,7-di-[hydro]-8H-pyrimido[5,4-b][1,4]oxazin-7-on,

   4-[Amino]-8-[(ethoxy)-carbonylmethyl]-2-[methyl]-6,7-di-[hydro]-8H-pyrimido[5,4-b][1,4]oxazin-7-on sowie ihre Säureadditionssalze.

7. Verfahren zur Herstellung der Verbindungen nach Anspruch 1 bis 6, dadurch gekennzeichnet, daß man
   a) zur Herstellung der Pyrimido[5,4-b][1,4]oxazinderivate der allgemeinen Formel I, bei welchen $R_2$ für einen Rest der allgemeinen Formel II, in welcher $R_5$ und $R_6$ die in den Ansprüchen 1 bis 5 angegebenen Bedeutungen haben, steht und $R_1$ , $R_3$ , $R_4$ und $R_9$ die im Anspruch 1 oder 2 angegebenen Bedeutungen haben, Pyrimido[5,4-b][1,4]oxazinderivate der allgemeinen Formel

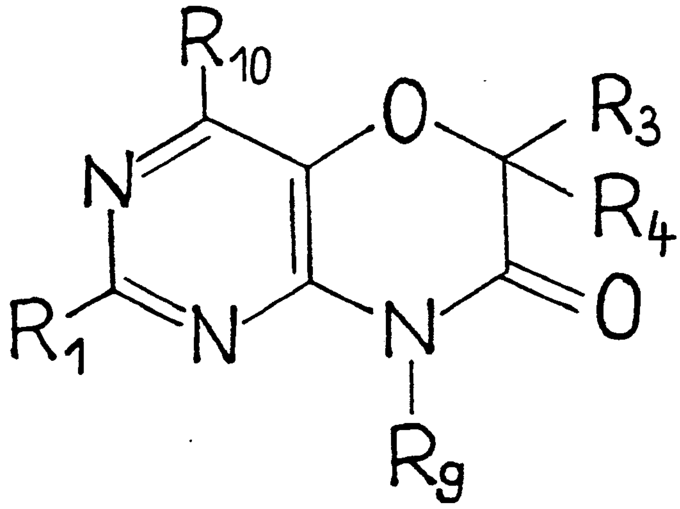

III ,

worin $R_1$, $R_3$, $R_4$ und $R_9$ die im Anspruch 1 oder 2 angegebenen Bedeutungen haben und $R_{10}$ für eine austretende Gruppe steht, mit, gegebenenfalls eine Schutzgruppe aufweisenden, Aminen der allgemeinen Formel

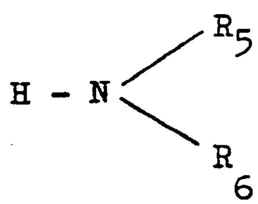

IV ,

worin $R_5$ und $R_6$ die in den Ansprüchen 1 bis 5 angegebenen Bedeutungen haben,
umsetzt oder

b) zur Herstellung der Pyrimido[5,4-b][1,4]oxazinderivate der allgemeinen Formel I, bei welchen $R_2$ für eine Azidogruppe steht und $R_1$, $R_3$, $R_4$ und $R_9$ die im Anspruch 1 oder 2 angegebenen Bedeutungen haben, Pyrimido[5,4-b][1,4]oxazinderivate der allgemeinen Formel I, bei welchen $R_1$, $R_3$, $R_4$ und $R_9$ die im Anspruch 1 oder 2 angegebenen Bedeutungen haben und $R_2$ für eine Hydrazinogruppe steht, diazotiert und/oder

c) zur Herstellung der Pyrimido[5,4-b][1,4]oxazinderivate der allgemeinen Formel I, bei welchen $R_1$, $R_2$, $R_3$ und $R_4$ die im Anspruch 1 oder 2 angegebenen Bedeutungen haben und $R_9$ für einen 2,3-Di-(hydroxy)-propylrest steht, Pyrimido[5,4-b][1,4]oxazinderivate der allgemeinen Formel I, bei welchen $R_1$, $R_2$, $R_3$ und $R_4$ die im Anspruch 1, 2 oder 4 angegebenen Bedeutungen haben und $R_9$ für Wasserstoff steht, mit 2,3-(Epoxy)-propanol umsetzt, und

α) gegebenenfalls die nach a) oder b) erhaltenen Pyrimido[5,4-b][1,4]oxazinderivate der allgemeinen Formel I, bei welchen $R_1$, $R_2$, $R_3$ und $R_4$ die im Anspruch 1, 2 oder 4 angegebenen Bedeutungen haben und $R_9$ für Wasserstoff steht, mit, gegebenenfalls eine Schutzgruppe aufweisenden, Verbindungen der allgemeinen Formel

$R_9$ - X V ,

worin

$R_9$ die im Anspruch 1 oder 2 angegebenen Bedeutungen mit Ausnahme von Wasserstoff hat und

X für eine austretende Gruppe steht, umsetzt und/oder

β) gegebenenfalls von den nach a), b) oder c) und gegebenenfalls α) erhaltenen Pyrimido[5,4-b][1,4]oxazinderivate der allgemeinen Formel I die Schutzgruppe(n) entfernt und/oder

γ) gegebenenfalls nach a), b) oder c) und gegebenenfalls α) und/oder β) erhaltene Pyrimido[5,4-b][1,4]oxazinderivate der allgemeinen Formel I in andere Pyrimido[5,4-b][1,4]oxazinderivate der allgemeinen Formel I überführt und/oder

δ) gegebenenfalls nach a), b) oder c) und gegebenenfalls α), β) und/oder γ) erhaltene Pyrimido-

[5,4-b][1,4]oxazinderivatbasen der allgemeinen Formel I in an sich bekannter Weise in Säureadditionssalze überführt und/oder gegebenenfalls erhaltene Säureadditionssalze der Pyrimido[5,4-b][1,4]oxazinderivate der allgemeinen Formel I in die freien Pyrimido[5,4-b][1,4]-oxazinderivatbasen der allgemeinen Formel I oder in andere Säureadditionssalze überführt.

**8.** Arzneimittel, gekennzeichnet durch einen Gehalt an 1 oder mehr Verbindung(en) nach Anspruch 1 bis 6 als Wirkstoff(en), zweckmäßig zusammen mit 1 oder mehr üblichen pharmazeutischen Träger- und/oder Hilfsstoff(en).

**9.** Pyrimido[5,4-b][1,4]oxazinderivate der allgemeinen Formel

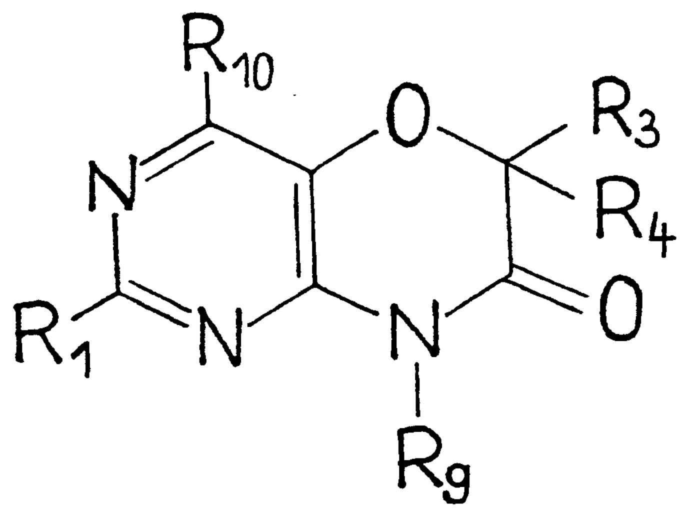

III ,

worin

| | |
|---|---|
| $R_{10}$ | für einen Alkylsulfonyloxy- oder Arylsulfonyloxyrest oder Methylsulfonylrest steht und |
| $R_1$ , $R_3$ , $R_4$ und $R_9$ | die im Anspruch 1 oder 2 angegebenen Bedeutungen haben. |

**Patentansprüche für folgende Vertragsstaat: AT**

**1.** Verfahren zur Herstellung von Pyrimido[5,4-b][1,4]oxazinderivaten der allgemeinen Formel

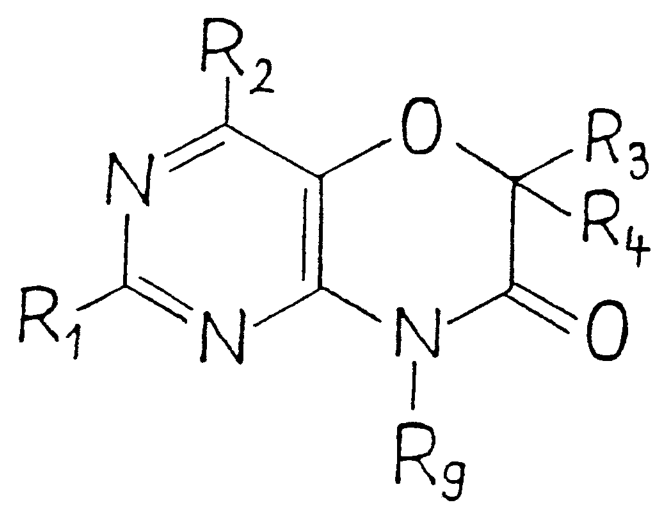

I ,

worin

| | |
|---|---|
| $R_1$ | für einen Alkylrest mit 1 bis 4 Kohlenstoffatom(en) steht, |
| $R_2$ | ein Wasserstoffatom, ein Halogenatom, eine Azidogruppe, eine Hydrazinogruppe oder |

einen Rest der allgemeinen Formel

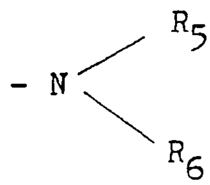

II ,

in welchletzterer

$R_5$ für ein Wasserstoffatom, einen, gegebenenfalls durch eine Hydroxygruppe substituierten, Alkylrest mit 1 bis 4 Kohlenstoffatom(en) oder Benzylrest steht und

$R_6$ ein Wasserstoffatom, eine Aminogruppe, einen Cycloalkylrest mit 3 bis 6 Kohlenstoffatomen, einen, gegebenenfalls durch eine Hydroxygruppe, eine Mercaptogruppe, einen Aminocarbonylrest, einen Furylrest, einen 2-Benzo[1,4]dioxanylrest, einen Di-(alkyl)-aminorest mit 1 bis 4 Kohlenstoffatom(en) in jedem Alkylteil oder einen, gegebenenfalls durch ein Halogenatom oder einen Alkylrest mit 1 bis 4 Kohlenstoffatom(en) oder 1 oder mehr Alkoxyrest(e) mit 1 bis 4 Kohlenstoffatom(en) substituierten, Phenyl- oder Phenoxyrest oder einen, gegebenenfalls durch einen, gegebenenfalls durch einen Alkylrest mit 1 bis 4 Kohlenstoffatom(en) oder Benzylrest substituierten, 6-gliedrigen gesättigten Stickstoff aufweisenden und gegebenenfalls außer dem Stickstoffatom noch 1 weiteres Stickstoffatom und/oder 1 Sauerstoffatom aufweisenden heterocyclischen Rest substituierten, Alkylrest mit 1 bis 4 Kohlenstoffatom(en) darstellt oder

$R_5$ und $R_6$ zusammen mit dem Stickstoffatom, an welches sie gebunden sind, einen, gegebenenfalls durch einen Alkoxycarbonylrest mit 2 bis 4 Kohlenstoffatomen oder einen Hydroxyalkylrest mit 1 bis 4 Kohlenstoffatom(en) substituierten, 6-gliedrigen gesättigten und gegebenenfalls außer dem Stickstoffatom noch 1 weiteres Stickstoffatom und/oder 1 Sauerstoffatom aufweisenden heterocyclischen Rest darstellen,

bedeutet,

$R_3$ und $R_4$ , die gleich oder Verschieden sein können, voneinander unabhängig für ein Wasserstoffatom oder einen Alkylrest mit 1 bis 4 Kohlenstoffatom(en) stehen und

$R_9$ ein Wasserstoffatom, einen, gegebenenfalls durch eine Oxogruppe, eine Cyanogruppe, einen Aminocarbonylrest. einen Alkoxycarbonylrest mit 2 bis 5 Kohlenstoffatomen, einen Pyridylrest, einen Morpholinylcarbonylrest oder einen Phenylrest monosubstituierten oder durch 1 oder mehr Hydroxygruppe(n) mono- oder polysubstituierten, Alkylrest bedeutet, mit den weiteren Maßgaben, daß

a)

$R_2$ von einem Wasserstoffatom, Chloratom, einem 4-Morpholinylrest oder einem Piperidylrest verschieden ist, wenn $R_9$ für ein Wasserstoffatom steht, und

b)

$R_2$ von einem Chloratom oder einem 4-Morpholinylrest verschieden ist, wenn

$R_9$ für einen Methyl- oder Benzylrest steht,

einschließlich ihrer Tautomere und ihrer Gemische sowie ihren Säureadditionssalzen, dadurch gekennzeichnet, daß man

a) zur Herstellung der Pyrimido[5,4-b][1,4]oxazinderivate der allgemeinen Formel I, bei welchen $R_2$ für einen Rest der allgemeinen Formel II, in welcher $R_5$ und $R_6$ die oben angegebenen Bedeutungen haben, steht und $R_1$ , $R_3$ , $R_4$ und $R_9$ die oben angegebenen Bedeutungen haben, Pyrimido[5,4-b]-[1,4]oxazinderivate der allgemeinen Formel

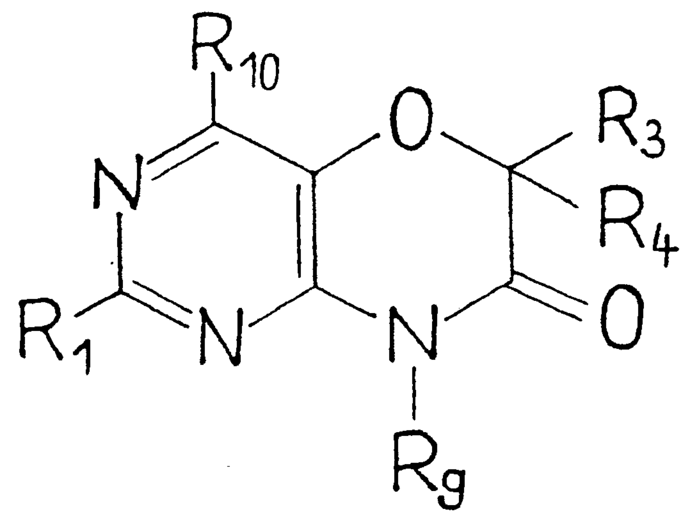

III ,

worin $R_1$, $R_3$, $R_4$ und $R_9$ die oben angegebenen Bedeutungen haben und $R_{10}$ für eine austretende Gruppe steht, mit, gegebenenfalls eine Schutzgruppe aufweisenden, Aminen der allgemeinen Formel

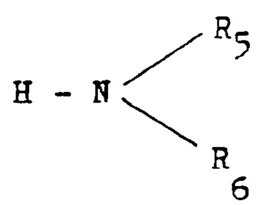

IV ,

worin $R_5$ und $R_6$ die oben angegebenen Bedeutungen haben,
umsetzt oder

b) zur Herstellung der Pyrimido[5,4-b][1,4]oxazinderivate der allgemeinen Formel I, bei welchen $R_2$ für eine Azidogruppe steht und $R_1$, $R_3$, $R_4$ und $R_9$ die oben angegebenen Bedeutungen haben, Pyrimido[5,4-b][1,4]oxazinderivate der allgemeinen Formel I, bei welchen $R_1$, $R_3$, $R_4$ und $R_9$ die oben angegebenen Bedeutungen haben und $R_2$ für eine Hydrazinogruppe steht, diazotiert und/oder

c) zur Herstellung der Pyrimido[5,4-b][1,4]oxazinderivate der allgemeinen Formel I, bei welchen $R_1$, $R_2$, $R_3$ und $R_4$ die oben angegebenen Bedeutungen haben und $R_9$ für einen 2,3-Di-(hydroxy)-propylrest steht, Pyrimido[5,4-b][1,4]oxazinderivate der allgemeinen Formel I, bei welchen $R_1$, $R_2$, $R_3$ und $R_4$ die oben angegebenen Bedeutungen haben und $R_9$ für Wasserstoff steht, mit 2,3-(Epoxy)-propanol umsetzt, und

α) gegebenenfalls die nach a) oder b) erhaltenen Pyrimido[5,4-b][1,4]oxazinderivate der allgemeinen Formel I, bei welchen $R_1$, $R_2$, $R_3$ und $R_4$ die oben angegebenen Bedeutungen haben und $R_9$ für Wasserstoff steht, mit, gegebenenfalls eine Schutzgruppe aufweisenden, Verbindungen der allgemeinen Formel

$R_9$ - X    V ,

worin

$R_9$ die oben angegebenen Bedeutungen mit Ausnahme von Wasserstoff hat und

X für eine austretende Gruppe steht, umsetzt und/oder

β) gegebenenfalls von den nach a), b) oder c) und gegebenenfalls α) erhaltenen Pyrimido[5,4-b][1,4]oxazinderivate der allgemeinen Formel I die Schutzgruppe(n) entfernt und/oder

γ) gegebenenfalls nach a), b) oder c) und gegebenenfalls α) und/oder β) erhaltene Pyrimido[5,4-b][1,4]oxazinderivate der allgemeinen Formel I in andere Pyrimido[5,4-b][1,4]oxazinderivate der allgemeinen Formel I überführt und/oder

δ) gegebenenfalls nach a), b) oder c) und gegebenenfalls α), β) und/oder γ) erhaltene Pyrimido-[5,4-b][1,4]oxazinderivatbasen der allgemeinen Formel I in an sich bekannter Weise in Säureadditionssalze überführt und/oder gegebenenfalls erhaltene Säureadditionssalze der

Pyrimido[5,4-b][1,4]oxazinderivate der allgemeinen Formel I in die freien Pyrimido[5,4-b][1,4]-oxazinderivatbasen der allgemeinen Formel I oder in andere Säureadditionssalze überführt.

**2.** Verfahren nach Anspruch 1, dadurch gekennzeichnet, daß man Pyrimido[5,4-b][1,4]oxazinderivate herstellt, bei welchen
a) der Alkylrest, für den $R_1$ und/oder $R_3$ und/oder $R_4$ und/oder $R_5$ und/oder $R_6$ stehen kann beziehungsweise können, und/oder
b) die Alkylteile des Di-(alkyl)-aminorestes, durch welchen der Alkylrest, für welchen $R_6$ stehen kann, substituiert sein kann, oder
c) der Alkylrest oder Alkoxyrest, durch welchen der Phenyl- oder Phenoxyrest, durch den der Alkylrest, für welchen $R_6$ stehen kann, substituiert sein kann, substituiert sein kann, oder
d) der Alkylrest, durch welchen der Benzylrest, durch den der heterocyclische Rest, durch welchen der Alkylrest, für welchen $R_6$ stehen kann, jeweils substituiert sein kann oder
e) der Hydroxyalkylrest, durch den der heterocyclische Rest, den $R_5$ und $R_6$ zusammen mit dem Stickstoffatom, an welchen sie gebunden sind, darstellen können, substituiert sein kann,

[ein] solche[r] mit 1 bis 3, insbesondere 1 oder 2, Kohlenstoffatom(en) ist beziehungsweise sind und/oder
f) der Alkylrest, für den $R_9$ stehen kann,

[ein] solche[r] mit 1 bis 4, insbesondere 1 bis 3, Kohlenstoffatomen ist und/oder
g) der Alkoxycarbonylrest, durch welchen der heterocyclische Rest, den $R_5$ und $R_6$ zusammen mit dem Stickstoffatom, an welches sie gebunden sind, darstellen können, substituiert sein kann, und/oder
h) der Alkoxycarbonylrest, durch welchen der Alkylrest, für den $R_9$ stehen kann, substituiert sein kann,

[ein] solche[r] mit 2 bis 4 Kohlenstoffatomen ist.

**3.** Verfahren nach Anspruch 1 oder 2, dadurch gekennzeichnet, daß man Pyrimido[5,4-b][1,4]-oxazinderivate herstellt, bei welchen der Cycloalkylrest, für welchen $R_6$ stehen kann, ein solcher mit 3 bis 5, insbesondere 3 oder 4, Kohlenstoffatomen ist.

**4.** Verfahren nach Anspruch 1 bis 3, dadurch gekennzeichnet, daß man Pyrimido[5,4-b][1,4]oxazinderivate herstellt, bei welchen
a) das Halogenatom, für welches $R_2$ stehen kann, oder
b) das Halogenatom, durch welches der Phenyl- oder Phenoxyrest, durch den der Alkylrest, für welchen $R_6$ stehen kann, substituiert sein kann, substituiert sein kann,

ein Fluor- oder Chloratom ist.

**5.** Verfahren nach Anspruch 1 bis 3, dadurch gekennzeichnet, daß man Pyrimido[5,4-b][1,4]oxazinderivate herstellt, bei welchen
a) der heterocyclische Rest, durch welchen der Alkylrest, für den $R_6$ stehen kann, substituiert sein kann, oder
b) der heterocyclische Rest, den $R_5$ und $R_6$ zusammen mit dem Stickstoffatom, an welches sie gebunden sind, darstellen können,

ein Morpholinylrest, ein Piperidylrest oder Piperazinylrest ist.

**6.** Verfahren nach Anspruch 1 bis 5, dadurch gekennzeichnet, daß man

2-[Methyl]-4-[⟨2'-(morpholin-4''-yl)-ethyl⟩-amino]-6,7-di-[hydro]-8H-pyrimido[5,4-b][1,4]oxazin-7-on,

4-[N-⟨Benzyl⟩-N-⟨2'-(hydroxy)-ethyl⟩-amino]-2-[methyl]-6,7-di-[hydro]-8H-pyrimido[5,4-b][1,4]oxazin-7-on,

2-[Methyl]-4-[⟨3'-(morpholin-4''-yl)-propyl⟩-amino]-6,7-di-[hydro]-8H-pyrimido[5,4-b][1,4]oxazin-7-on,

4-[Chlor]-8-[2',3'-di-(hydroxy)-propyl]-2-[methyl]-6,7-di-[hydro]-8H-pyrimido[5,4-b][1,4]oxazin-7-on,

8-[2',3'-Di-(hydroxy)-propyl]-2-[methyl]-4-[morpholin-4''-yl]-6,7-di-[hydro]-8H-pyrimido[5,4-b][1,4]oxazin-7-on,

4-[⟨4'-(Benzyl)-morpholin-2'-yl)-methyl⟩-amino]-2-[methyl]-6,7-di-[hydro]-8H-pyrimido[5,4-b][1,4]oxazin-7-on,

8-[(Ethoxy-carbonylmethyl]-4-[⟨2''-(hydroxy)-ethyl⟩-amino]-2-[methyl]-6,7-di-[hydro]-8H-pyrimido[5,4-b]-[1,4]oxazin-7-on,

4-[⟨2'-(Hydroxy)-ethyl⟩-amino]-2-[methyl]-8-[2''-(oxo)-propyl]-6,7-di-[hydro]-8H-pyrimido[5,4-b][1,4]-oxazin-7-on,

4-[⟨2'-(Hydroxy)-ethyl⟩-amino]-2-[methyl]-8-[morpholin-4''-ylcarbonylmethyl]-6,7-di-[hydro]-8H-pyrimido-[5,4-b][1,4]oxazin-7-on,

8-[(n-Butoxy)-carbonylmethyl]-4-[⟨2'-(hydroxy)-ethyl⟩-amino]-2-[methyl]-6,7-di-[hydro]-8H-pyrimido[5,4-b][1,4]oxazin-7-on,

8-[Benzyl]-4-[⟨2'-(hydroxy)-ethyl⟩-amino]-2-[methyl]-6,7-di-[hydroxy]-8H-pyrimido[5,4-b][1,4]oxazin-7-on,

4-[Amino]-8-[(ethoxy)-carbonylmethyl]-2-[methyl]-6,7-di-[hydro]-8H-pyrimido[5,4-b][1,4]oxazin-7-on sowie ihre Säureadditionssalze herstellt.

7. Verfahren zur Herstellung der Pyrimido[5,4-b][1,4]oxazinderivate der allgemeinen Formel

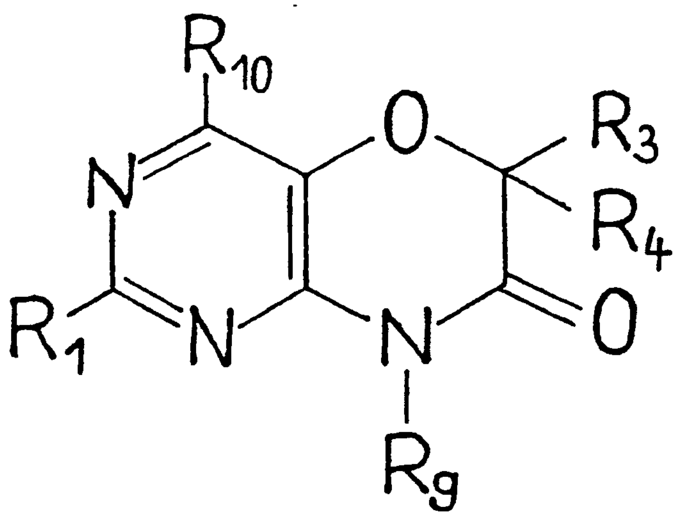

III ,

worin

| | |
|---|---|
| $R_{10}$ | für einen Alkysulfonyloxy- oder Arylsulfonyloxyrest oder Methylsulfonylrest steht und |
| $R_1$ , $R_3$ , $R_4$ und $R_9$ | die im Anspruch 1 oder 2 angegebenen Bedeutungen haben, |

dadurch gekennzeichnet, daß man

a) zur Herstellung derjenigen, bei welchen $R_{10}$ für einen Alkylsulfonyloxy- oder Arylsulfonyloxyrest steht,

Pyrimido[5,4-b][1,4]oxazinderivate der allgemeinen Formel I mit der Abwandlung, daß $R_2$ für eine Hydroxygruppe steht, mit den entsprechenden Arylsulfonylchloriden in einem basischen Medium umsetzt oder

b) zur Herstellung derjenigen, bei welchen $R_{10}$ für einen Methylsulfonylrest steht,

Pyrimido[5,4-b][1,4]oxazinderivate der allgemeinen Formel III, bei welchen $R_{10}$ für einen Methylthiorest steht, in an sich bekannter Weise oxydiert.

## Claims

## Claims for the following Contracting States: BE,CH,DE,FR,GB,IT,LI,LU,NL,SE

**1.** Pyrimido[5,4-b][1,4]oxazine derivatives of the general formula

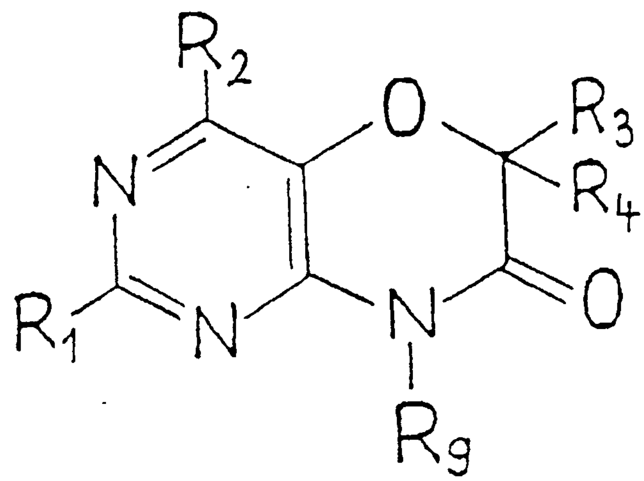

I ,

wherein

$R_1$ represents an alkyl group having 1 to 4 carbon atoms,

$R_2$ represents a hydrogen atom, a halogen atom, an azido group, a hydrazino group or a residue of the general formula

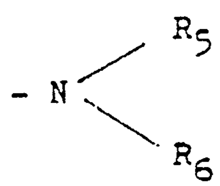

II ,

in the latter of which

$R_5$ represents a hydrogen atom, an alkyl residue having 1 to 4 carbon atoms or benzyl group, optionally substituted by a hydroxy group, and

$R_6$ represents a hydrogen atom, an amino group, a cycloalkyl group having 3 to 6 carbon atoms, a alkyl group having 1 to 4 carbon atoms, optionally substituted by a hydroxy group, a mercapto group, an aminocarbonyl group, a furyl group, a 2-benzo[1,4]-dioxanyl group, a di-(alkyl)amino group having 1 to 4 carbon atoms in each alkyl portion or a phenyl or phenoxy group, optionally substituted by a halogen atom or an alkyl group having 1 to 4 carbon atoms or 1 or more alkoxy groups having 1 to 4 carbon atoms, or an alkyl group having 1 to 4 carbon atoms, optionally substituted by a 6-membered saturated nitrogen containing heterocyclic residue optionally having in addition to the nitrogen atom 1 further nitrogen atom and/or 1 oxygen atom, which is optionally substituted by an alkyl group having 1 to 4 carbon atoms or benzyl group, or

$R_5$ and $R_6$ together with the nitrogen atom to which they are bound represent a 6-membered saturated heterocyclic residue optionally having in addition to the nitrogen atom 1 further nitrogen atom and/or 1 oxygen atom, which is optionally substituted by an alkoxy carbonyl group having 2 to 4 carbon atoms or a hydroxyalkyl group having 1

to 4 carbon atoms,

$R_3$ and $R_4$ which may be the same or different, independently represent a hydrogen atom or an alkyl group having 1 to 4 carbon atoms and

$R_9$ represents a hydrogen atom, an alkyl group, optionally monosubstituted by an oxo group, a cyano group, an aminocarbonyl group, an alkoxycarbonyl group having 2 to 5 carbon atoms, a pyridyl group, a morpholinylcarbonyl group or a phenyl group or mono- or polysubstituted by 1 or more hydroxy groups,

with the further provisos that

a)

$R_2$ is other than a hydrogen atom, chlorine atom, a 4-morpholinyl group or a piperidyl group when $R_9$ represents a hydrogen atom, and

b)

$R_2$ is other than a chlorine atom or a 4-morpholinyl group when $R_9$ represents a methyl or benzyl group,

including their tautomers and their mixtures as well as their acid addition salts.

**2.** Pyrimido[5,4-b][1,4]oxazine derivatives according to claim 1, characterized in that

a) the alkyl group for which $R_1$ and/or $R_3$ and/or $R_4$ and/or $R_5$ and/or $R_6$ may stand, and/or

b) the alkyl portions of the di-(alkyl)-amino group, by which the alkyl group, for which $R_6$ may stand, may be substituted, or

c) the alkyl group or alkoxy group, by which the phenyl or phenoxy group may be substituted, by which the alkyl group, for which $R_6$ may stand, may be substituted, or

d) the alkyl group by which the benzyl group may be substituted, by which the heterocyclic residue may be substituted, by which the alkyl group, for which $R_6$ may stand, may be substituted or

e) the hydroxyalkyl group by which the heterocyclic residue may be substituted, for which $R_5$ and $R_6$ may stand together with the nitrogen atom to which they are bound, has or have 1 to 3, particularly 1 or 2, carbon atoms and/or

f) the alkyl group, for which $R_9$ may stand, has 1 to 4, particularly 1 to 3, carbon atoms and/or

g) the alkoxycarbonyl group by which the heterocyclic residue may be substituted, for which $R_5$ and $R_6$ may stand together with the nitrogen atom to which they are bound and/or

h) the alkoxycarbonyl group by which the alkyl group, for which $R_9$ may stand, may be substituted

has 2 to 4 carbon atoms.

**3.** Pyrimido[5,4-b][1,4]oxazine derivatives according to claim 1 or 2, characterized in that the cycloalkyl group, for which $R_6$ may stand, has 3 to 5, particularly 3 or 4, carbon atoms.

**4.** Pyrimido[5,4-b][1,4]oxazine derivatives according to claims 1 to 3, characterized in that

a) the halogen atom, for which $R_2$ may stand, or

b) the halogen atom by which the phenyl or phenoxy group may be substituted, by which the alkyl group, for which $R_6$ may stand, may be substituted

is a fluorine or chlorine atom.

**5.** Pyrimido[5,4-b][1,4]oxazine derivatives according to claims 1 to 3, characterized in that

a) the heterocyclic residue by which the alkyl group, for which $R_6$ may stand, may be substituted or

b) the heterocyclic residue, for which $R_5$ and $R_6$ may stand together with the nitrogen atom to which they are bound,

is a morpholinyl group, a piperidyl group or a piperazinyl group.

**6.** 2-[methyl]-4-[(2'-(morpholine-4''-yl)-ethyl)-amino]-6,7-di-[hydro]-8H-pyrimido[5,4-b][1,4]oxazine-7-one,

4-[N-(benzyl)-N-(2'-(hydroxy)-ethyl)-amino]-2-[methyl]-6,7-di-[hydro]-8H-pyrimido[5,4-b][1,4]oxazine-7-one,

2-[methyl]-4-[(3'-(morpholine-4''-yl)-propyl)-amino]-6,7-di-[hydro]-8H-pyrimido[5,4-b][1,4]oxazine-7-one,

4-[chloro]-8-[2',3'-di-(hydroxy)-propyl]-2-[methyl]-6,7-di-[hydro]-8H-pyrimido[5,4-b][1,4]oxazine-7-one,

8-[2',3'-di-(hydroxy)-propyl]-2-[methyl]-4-[morpholine-4''-yl]-6,7-di-[hydro]-8H-pyrimido[5,4-b][1,4]-

oxazine-7-one,

4-[(4'-(benzyl)-morpholine-2'-yl)-methyl)-amino]-2-[methyl]-6,7-di-[hydro]-8H-pyrimido[5,4-b][1,4]-oxazine-7-one,

8-[(ethoxy-carbonylmethyl]-4-[(2"-(hydroxy)-ethyl)-amino]-2-[methyl]-6,7-di-[hydro]-8H-pyrimido[5,4-b]-[1,4]oxazine-7-one,

4-[(2'-(hydroxy)-ethyl)-amino]-2-[methyl]-8-[2"-(oxo)-propyl]-6,7-di-[hydro]-8H-pyrimido[5,4-b][1,4]-oxazine-7-one,

4-[(2'-(hydroxy)-ethyl)-amino]-2-[methyl]-8-[morpholine-4"-yl-carbonylmethyl]-6,7-di-[hydro]-8H-pyrimido[5,4-b][1,4]oxazine-7-one,

8-[(n-butoxy)-carbonylmethyl]-4-[(2'-(hydroxy)-ethyl)-amino]-2-[methyl]-6,7-di-[hydro]-8H-pyrimido[5,4-b][1,4]oxazine-7-one,

8-[benzyl]-4-[(2'-(hydroxy)-ethyl)-amino]-2-[methyl]-6,7-di-[hydro]-8H-pyrimido[5,4-b][1,4]oxazine-7-one,

4-[amino]-8-[(ethoxy)-carbonylmethyl]-2-[methyl]-6,7-di-[hydro]-8H-pyrimido[5,4-b][1,4]oxazine-7-one, as well as the acid addition salts thereof.

**7.** A process for the preparation of the compounds according to claims 1 to 6, characterized in that
a) for the preparation of the pyrimido[5,4-b][1,4]oxazine derivatives of the general formula I, wherein $R_2$ represents a residue of general formula II, in which $R_5$ and $R_6$ are as defined in claims 1 to 5, and $R_1$, $R_3$, $R_4$ and $R_9$ are as defined in claim 1 or 2, pyrimido[5,4-b][1,4]oxazine derivatives of the general formula

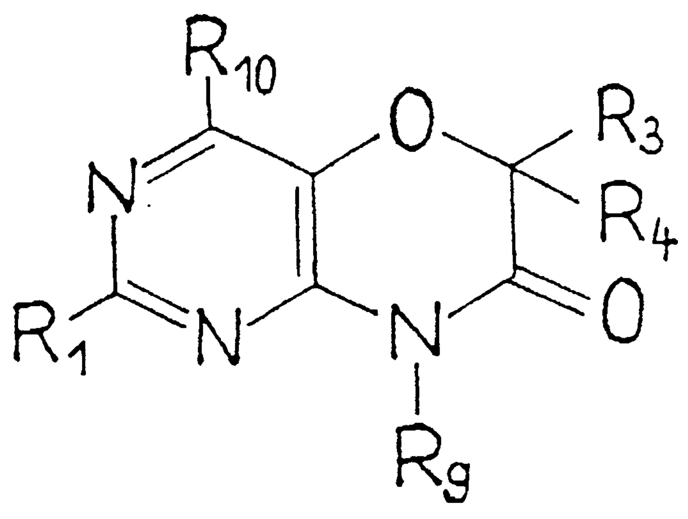 III ,

wherein $R_1$, $R_3$, $R_4$ and $R_9$ are as defined in claim 1 or 2 and $R_{10}$ represents a leaving group, are reacted with amines, optionally having a protective group, of the general formula

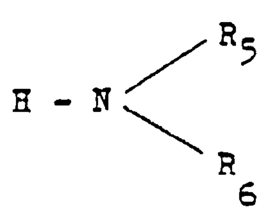 IV ,

wherein $R_5$ and $R_6$ are as defined in claims 1 to 5 or
b) for the preparation of the pyrimido[5,4-b][1,4]oxazine derivatives of general formula I, wherein $R_2$

represents an azido group and $R_1$, $R_3$, $R_4$ and $R_9$ are as defined in claim 1 or 2, pyrimido[5,4-b]-[1,4]oxazine derivatives of general formula I, wherein $R_1$, $R_3$, $R_4$ and $R_9$ are as defined in claim 1 or 2 and $R_2$ represents a hydrazino group, are diazotized and/or

c) for the preparation of the pyrimido[5,4-b][1,4]oxazine derivatives of general formula I, wherein $R_1$, $R_2$, $R_3$ and $R_4$ are as defined in claim 1 or 2 and $R_9$ represents a 2,3-di-(hydroxy)-propyl group, pyrimido[5,4-b][1,4]oxazine derivatives of general formula I, wherein $R_1$, $R_2$, $R_3$ and $R_4$ are as defined in claim 1, 2 or 4 and $R_9$ represents hydrogen, are reacted with 2,3-(epoxy)-propanol, and

$\alpha$) the pyrimido[5,4-b][1,4]oxazine derivatives, obtained according to a) or b), of general formula I, wherein $R_1$, $R_2$, $R_3$ and $R_4$ are as defined in claim 1, 2 or 4 and $R_9$ represents hydrogen, are optionally reacted with compounds, optionally having a protective group, of the general formula

$R_9$ - X V ,

wherein

$R_9$ is as defined in claim 1 or 2 with the exception of hydrogen and

X represents a leaving group and/or

$\beta$) the protective group(s) are optionally removed from the pyrimido[5,4-b][1,4]oxazine derivatives of general formula I, obtained according to a), b) or c) and optionally $\alpha$), and/or

$\gamma$) pyrimido[5,4-b][1,4]oxazine derivatives of general formula I, obtained according to a), b) or c) and optionally $\alpha$) and/or $\beta$), are optionally converted into other pyrimido[5,4-b][1,4]oxazine derivatives of general formula I and/or

$\delta$) pyrimido[5,4-b][1,4]oxazine derivative bases of general formula I, obtained according to a), b) or c) and optionally $\alpha$), $\beta$) and/or $\delta$) are optionally converted into acid addition salts in a manner known per se and/or resulting acid addition salts of the pyrimido[5,4-b][1,4]oxazine derivatives of general formula I are optionally converted into the free pyrimido[5,4-b][1,4]-oxazine derivative bases of general formula I or into other acid addition salts.

**8.** Medicament, characterized by a content of 1 or more compounds according to any one of claims 1 to 6 as active substance(s), suitably together with 1 or more conventional pharmaceutical carriers and/or excipients.

**9.** Pyrimido[5,4-b][1,4]oxazine derivatives of the general formula

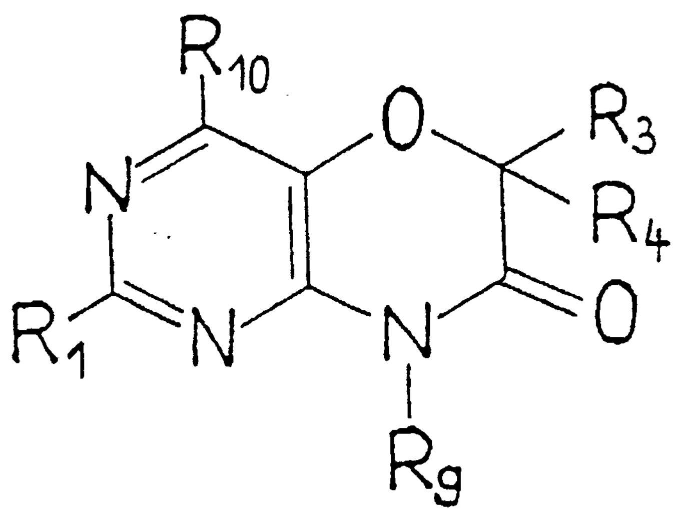

III ,

wherein

$R_{10}$ represents an alkylsulfonyloxy or arylsulfonyloxy residue or methylsulfonyl residue and

$R_1$, $R_3$, $R_4$ and $R_9$ are as defined in claim 1 or 2.

**Claims for the following Contracting State: AT**

1. A process for the preparation of pyrimido[5,4-b][1,4]oxazine derivatives of the general formula

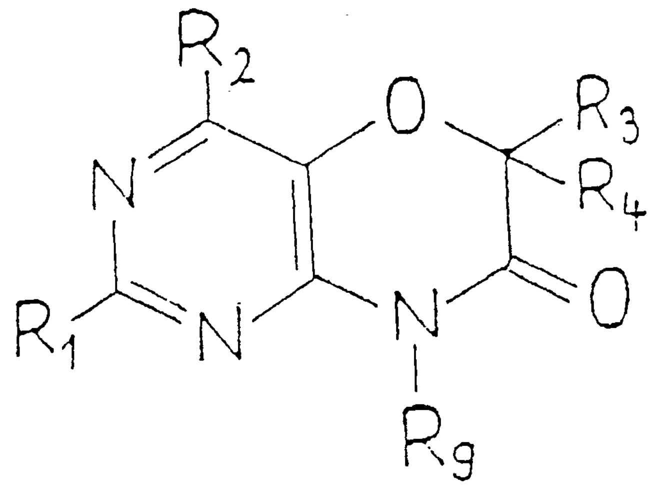

I ,

wherein

| | |
|---|---|
| $R_1$ | represents an alkyl group having 1 to 4 carbon atoms, |
| $R_2$ | represents a hydrogen atom, a halogen atom, an azido group, a hydrazino group or a residue of the general formula |

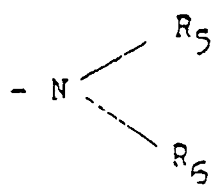

II ,

| | |
|---|---|
| | in the latter of which |
| $R_5$ | represents a hydrogen atom, an alkyl group having 1 to 4 carbon atoms or benzyl group, optionally substituted by a hydroxy group, and |
| $R_6$ | represents a hydrogen atom, an amino group, a cycloalkyl group having 3 to 6 carbon atoms, a alkyl group having 1 to 4 carbon atoms, optionally substituted by a hydroxy group, a mercapto group, an aminocarbonyl group, a furyl group, a 2-benzo[1.4]-dioxanyl group, a di-(alkyl)-amino group having 1 to 4 carbon atoms in each alkyl portion or a phenyl or phenoxy group, optionally substituted by a halogen atom or an alkyl group having 1 to 4 carbon atoms or 1 or more alkoxy groups having 1 to 4 carbon atoms, or an alkyl group having 1 to 4 carbon atoms, optionally substituted by a 6-membered saturated nitrogen containing heterocyclic residue optionally having in addition to the nitrogen atom 1 further nitrogen atom and/or 1 oxygen atom, which is optionally substituted by an alkyl group having 1 to 4 carbon atoms or benzyl group, or |
| $R_5$ and $R_6$ | together with the nitrogen atom to which they are bound represent a 6-membered saturated heterocyclic residue optionally having in addition to the nitrogen atom 1 further nitrogen atom and/or 1 oxygen atom, which is optionally substituted by an alkoxy carbonyl group having 2 to 4 carbon atoms or a hydroxyalkyl group having 1 to 4 carbon atoms, |
| $R_3$ and $R_4$ | which may be the same or different, independently represent a hydrogen atom or an alkyl group having 1 to 4 carbon atoms, and |

$R_9$ represents a hydrogen atom, an alkyl group, optionally monosubstituted by an oxo group, a cyano group, an aminocarbonyl group, an alkoxycarbonyl group having 2 to 5 carbon atoms, a pyridyl group, a morpholinylcarbonyl group or a phenyl group or mono- or polysubstituted by 1 or more hydroxy groups,

with the further provisos that

a)

$R_2$ is other than a hydrogen atom, chlorine atom, a 4-morpholinyl group or a piperidyl group when $R_9$ represents a hydrogen atom, and

b)

$R_2$ is other than a chlorine atom or a 4-morpholinyl group when $R_9$ represents a methyl or benzyl group,

including their tautomers and their mixtures as well as their acid addition salts, characterized in that

a) for the preparation of the pyrimido[5,4-b][1,4]oxazine derivatives of the general formula I, wherein $R_2$ represents a residue of general formula II, in which $R_5$ and $R_6$ are as defined above, and $R_1$, $R_3$, $R_4$ and $R_9$ are as defined above, pyrimido[5,4-b][1,4]oxazine derivatives of the general formula

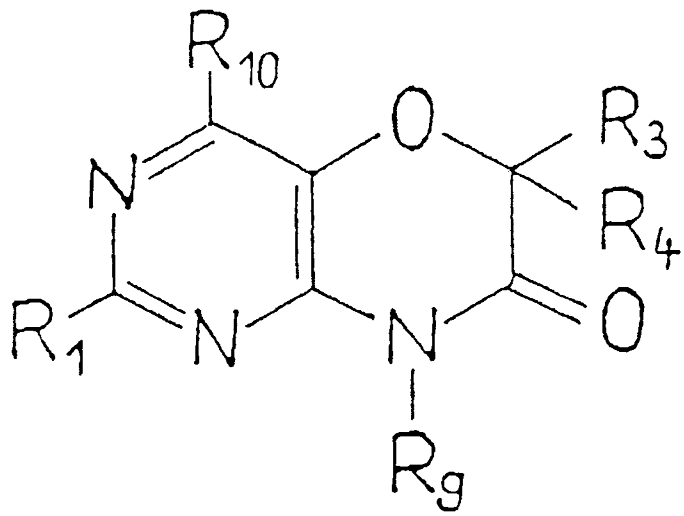

III ,

wherein $R_1$, $R_3$, $R_4$ and $R_9$ are as defined above and $R_{10}$ represents a leaving group, are reacted with amines, optionally having a protective group, of the general formula

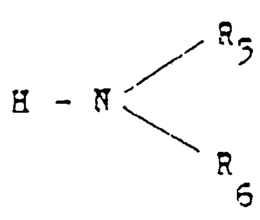

IV ,

wherein $R_5$ and $R_6$ are as defined above or

b) for the preparation of the pyrimido[5,4-b][1,4]oxazine derivatives of general formula I, wherein $R_2$ represents an azido group and $R_1$, $R_3$, $R_4$ and $R_9$ are as defined above, pyrimido[5,4-b][1,4]oxazine derivatives of general formula I, wherein $R_1$, $R_3$, $R_4$ and $R_9$ are as defined above and $R_2$ represents a hydrazino group, are diazotized and/or

c) for the preparation of the pyrimido[5,4-b][1,4]oxazine derivatives of general formula I, wherein $R_1$, $R_2$, $R_3$ and $R_4$ are as defined above and $R_9$ represents a 2,3,-di-(hydroxy)-propyl group, pyrimido-[5,4-b][1,4]oxazine derivatives of general formula I, wherein $R_1$, $R_2$, $R_3$ and $R_4$ are as defined above and $R_9$ represents hydrogen, are reacted with 2,3-(epoxy)-propanol, and

α) the pyrimido[5,4-b][1,4]oxazine derivatives, obtained according to a) or b), of general formula

I, wherein $R_1$, $R_2$, $R_3$ and $R_4$ are as defined above and $R_9$ represents hydrogen, are optionally reacted with compounds, optionally having a protective group, of the general formula

$R_9$ - X V,

wherein

$R_9$ is as defined above with the exception of hydrogen and

X represents a leaving group and/or

β) the protective group(s) are optionally removed from the pyrimido[5,4-b][1,4]oxazine derivatives of general formula I, obtained according to a), b) or c) and optionally α), and/or

γ) pyrimido[5,4-b][1,4]oxazine derivatives of general formula I, obtained according to a), b) or c) and optionally α) and/or β), are optionally converted into other pyrimido[5,4-b][1,4]oxazine derivatives of general formula I and/or

δ) pyrimido[5,4-b][1,4]oxazine derivative bases of general formula I, obtained according to a), b) or c) and optionally α), β) and/or γ), are optionally converted into acid addition salts in a manner known per se and/or resulting acid addition salts of the pyrimido[5,4-b][1,4]oxazine derivatives of general formula I are optionally converted into the free pyrimido[5,4-b][1,4]-oxazine derivative bases of general formula I or into other acid addition salts.

**2.** The process according to claim 1, characterized in that pyrimido[5,4-b][1,4]oxazine derivatives are prepared, wherein

a) the alkyl group for which $R_1$ and/or $R_3$ and/or $R_4$ and/or $R_5$ and/or $R_6$ may stand, and/or

b) the alkyl portions of the di-(alkyl)-amino group, by which the alkyl group, for which $R_6$ may stand, may be substituted, or

c) the alkyl group or alkoxy group, by which the phenyl or phenoxy group may be substituted, by which the alkyl group, for which $R_6$ may stand, may be substituted or

d) the alkyl group by which the benzyl group may be substituted, by which the heterocyclic residue may be substituted, by which the alkyl group, for which $R_6$ may stand, may be substituted or

e) the hydroxyalkyl group by which the heterocyclic residue may be substituted, for which $R_5$ and $R_6$ may stand together with the nitrogen atom to which they are bound,

has or have 1 to 3, particularly 1 or 2, carbon atoms and/or

f) the alkyl group, for which $R_9$ may stand, has 1 to 4, particularly 1 to 3, carbon atoms and/or

g) the alkoxycarbonyl group by which the heterocyclic residue may be substituted, for which $R_5$ and $R_6$ may stand together with the nitrogen atom to which they are bound and/or

h) the alkoxycarbonyl group by which the alkyl group, for which $R_9$ may stand, may be substituted

has 2 to 4 carbon atoms.

**3.** The process according to claim 1 or 2, characterized in that pyrimido[5,4-b][1,4]oxazine derivatives are prepared in which the cycloalkyl group, for which $R_6$ may stand, has 3 to 5, particularly 3 or 4, carbon atoms.

**4.** The process according to claims 1 to 3, characterized in that pyrimido[5,4-b][1,4]oxazine derivatives are prepared, in which

a) the halogen atom for which $R_2$ may stand, or

b) the halogen atom by which the phenyl or phenoxy group may be substituted, by which the alkyl group, for which $R_6$ may stand, may be substituted

is a fluorine or chlorine atom.

**5.** The process according to claims 1 to 3, characterized in that pyrimido[5,4-b][1,4]oxazine derivatives are prepared, in which

a) the heterocyclic residue by which the alkyl group, for which $R_6$ may stand, may be substituted or

b) the heterocyclic residue, for which $R_5$ and $R_6$ may stand together with the nitrogen atom to which they are bound,

is a morpholinyl group, a piperidyl group or a piperazinyl group.

**6.** The process according to claims 1 to 5, characterized by preparing

2-[methyl]-4-[⟨2'-(morpholine-4''-yl)-ethyl⟩-amino]-6,7-di-[hydro]-8H-pyrimido[5,4-b][1,4]oxazine-7-one,

4-[N-⟨benzyl⟩-N-⟨2'-(hydroxy)-ethyl⟩-amino]-2-[methyl]-6,7-di-[hydro]-8H-pyrimido[5,4-b][1,4]oxazine-7-one,

2-[methyl]-4-[⟨3'-(morpholine-4''-yl)-propyl⟩-amino]-6,7-di-[hydro]-8H-pyrimido[5,4-b][1,4]oxazine-7-one,

4-[chloro]-8-[2',3'-di-(hydroxy)-propyl]-2-[methyl]-6,7-di-[hydro]-8H-pyrimido[5,4-b][1,4]oxazine-7-one,

8-[2',3'-di-(hydroxy)-propyl]-2-[methyl]-4-[morpholine-4''-yl]-6,7-di-[hydro]-8H-pyrimido[5,4-b][1,4]-oxazine-7-one,

4-[⟨4'-(benzyl)-morpholine-2'-yl)-methyl⟩-amino]-2-[methyl]-6,7-di-[hydro]-8H-pyrimido[5,4-b][1,4]-oxazine-7-one,

8-[(ethoxy-carbonylmethyl]-4-[⟨2''-(hydroxy)-ethyl⟩-amino]-2-[methyl]-6,7-di-[hydro]-8H-pyrimido[5,4-b]-[1,4]oxazine-7-one,

4-[⟨2'-(hydroxy)-ethyl⟩-amino]-2-[methyl]-8-[2''-(oxo)-propyl]-6,7-di-[hydro]-8H-pyrimido[5,4-b][1,4]-oxazine-7-one,

4-[⟨2'-(hydroxy)-ethyl⟩-amino]-2-[methyl]-8-[morpholine-4''-yl-carbonylmethyl]-6,7-di-[hydro]-8H-pyrimido[5,4-b][1,4]oxazine-7-one,

8-[(n-butoxy)-carbonylmethyl]-4-[⟨2'-(hydroxy)-ethyl⟩-amino]-2-[methyl]-6,7-di-[hydro]-8H-pyrimido[5,4-b][1,4]oxazine-7-one,

8-[benzyl]-4-[⟨2'-(hydroxy)-ethyl⟩-amino]-2-[methyl]-6,7-di-[hydro]-8H-pyrimido[5,4-b][1,4]oxazine-7-one,

4-[amino]-8-[(ethoxy)-carbonylmethyl]-2-[methyl]-6,7-di-[hydro]-8H-pyrimido[5,4-b][1,4]oxazine-7-one, as well as the acid addition salts thereof.

7. A process for the production of pyrimido[5,4-b][1,4]oxazine derivatives of the general formula

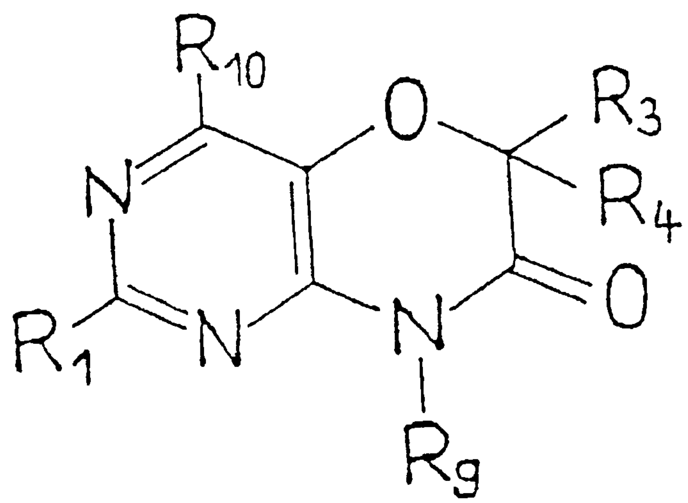

III ,

wherein

| | |
|---|---|
| $R_{10}$ | represents an alkylsulfonyloxy or arylsulfonyloxy residue or methylsulfonyl residue and |
| $R_1$, $R_3$ $R_4$ and $R_9$ | are as defined in claim 1 or 2, |

characterized in that

a) for the preparation of those in which $R_{10}$ represents an alkylsulfonyloxy or arylsulfonyloxy group, pyrimido[5,4-b][1,4]oxazine derivatives of general formula I, with the modification that $R_2$ represents a hydroxy group, are reacted with the corresponding arylsulfonylchlorides in a basic medium or

b) for the preparation of those in which $R_{10}$ represents a methylsulfonyl group, pyrimido[5,4-b][1,4]-oxazine derivatives of the general formula III, in which $R_{10}$ represents a methylthio group, are oxidized in a manner known per se.

## Revendications

**Revendications pour les Etats contractants suivants : BE,CH,DE,FR,GB, IT,LI,LU,NL,SE**

**1.** Dérivés de la pyrimido [5,4-b] [1,4] oxazine de la formule générale

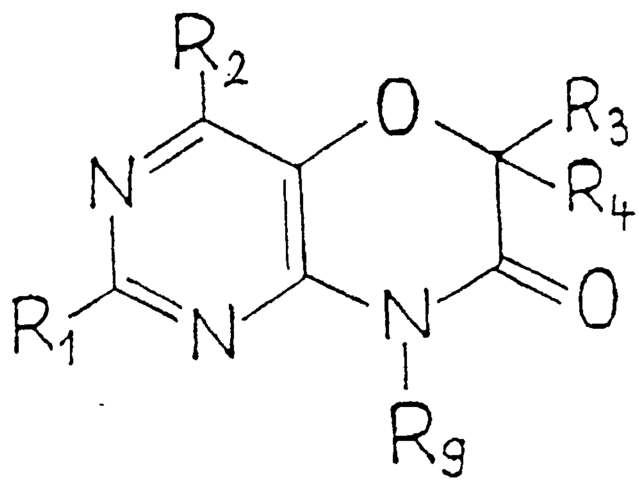

I ,

dans laquelle

| | |
|---|---|
| $R_1$ | représente un résidu d' alcoyle comprenant de 1 à 4 atome(s) de carbone |
| $R_2$ | un atome d'hydrogène, un atome d'halogène, un groupe azine, un groupe hydrazine ou un résidu de la formule générale |

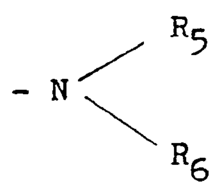

II ,

dans laquelle dernière

| | |
|---|---|
| $R_5$ | représente un atome d'hydrogène, un résidu de benzyle ou un résidu d'alcoyle comprenant de 1 à 4 atome(s) de carbone, le cas échéant substitué par un groupe hydroxy et |
| $R_6$ | constitue un atome d' hydrogène, un groupe amino, un résidu de cycloalcoyle comprenant de 3 à 6 atomes de carbone, un résidu d' alcoyle comprenant de 1 à 4 atome(s) de carbone, substitué par un résidu hétérocyclique substitué, le cas échéant, par un groupe hydroxy, un groupe mercapto, un résidu d'aminocarbonyle, un résidu de furyle, un résidu de 2-Benzo[1,4]dioxanyle, un résidu Di-(alkyl)-amino comprenant de 1 à 4 atome (s) de carbone dans chaque fraction alcoyle ou un résidu de phenyle ou phenoxy, le cas échéant, substitué par un atome d'halogène ou un résidu d'alcoyle comprenant de 1 à 4 atome(s) de carbone ou 1 ou plusieurs résidu(s) alcoxy présentant de 1 à 4 atome(s) de carbone ou présentant de l'azote saturé à 6 chainons, éventuellement substitué par un résidu de benzyle ou, le cas échéant, par un résidu d'alcoyle comprenant de 1 à 4 atome (s) de carbone et présentant ,le cas échéant, |

outre l'azote, encore un autre atome d'azote et/ou un atome d'oxygène ou

$R_5$ et $R_6$ conjointement avec l'atome d'azote, auquel ils sont liés, constituent un résidu hétérocyclique, le cas échéant, substitué par un résidu d'alcoxycarbonyle comprenant de 2 à 4 atomes de carbone ou un résidu d'hydroxyalcoyle comprenant de 1 à 4 atome(s) de carbone, lequel résidu hétérocyclique est saturé à 6 chainons et présente, le cas échéant,outre l'atome d'azote, encore 1 autre atome d'azote et/ou 1 atome d'oxygène

$R_3$ et $R_4$ qui peuvent être identiques ou différents, représentant indépendamment entre eux un atome d'hydrogène ou un résidu d'alcoyle comprenant de 1 à 4 atome(s) de carbone et

$R_9$ signifie un atome d' hydrogène, un résidu alcoyle, le cas échéant, monosubstitué par un groupe oxo, un groupe cyano, un résidu d'aminocarbonyle, un résidu d'alcoxycarbonyle comprenant de 2 à 5 atomes de carbone, un résidu de pyridyle, un résidu de morpholinylcarbonyle ou un résidu de phenyle ou mono - ou polysubstitué par un ou plusieurs groupe(s) hydroxy,

étant de plus entendu que

a)

$R_2$ est différent d'un atome d'hydrogène, d'un atome de chlore, d'un résidu de 4-morpholinyle ou d'un résidu de piperidyle, lorsque $R_9$ représente un atome d'hydrogène et

b)

$R_2$ est différent d'un atome de chlore ou d'un résidu de 4-morpholinyle, lorsque $R_9$ représente un résidu de methyle ou de benzyle,

y compris leurs tautomères et leurs mélanges ainsi que leurs sels acides d'addition.

2. Dérivés de la pyrimido[5,4-b][1,4] conformément à la revendication 1 caractérisés en ce que

a) le résidu d'alcoyle, que $R_1$ et/ou $R_3$ et/ou $R_4$ et/ou $R_5$ et/ou $R_6$ peut, voire peuvent, représenter, et/ou

b) les fractions d'alcoyle du résidu di-(alkyl)-amino, auquel on peut substituer le résidu d'alcoyle , que peut constituer $R_6$ , ou

c) le résidu d'alcoyle ou d'alcoxy , auquel on peut substituer le résidu de phenyle ou de phenoxy,par lequel on peut substituer le résidu d'alcoyle pouvant constituer $R_6$ ou

d) le résidu d'alcoyle, auquel on peut substituer le résidu de benzyle, par lequel on peut substituer le résidu hétérocyclique pouvant substituer le résidu d' alcoyle, que $R_6$ peut constituer ou

e) le résidu d'hydroxyalkyle, auquel on peut substituer le résidu hétérocyclique, que peuvent constituer $R_5$ et $R_6$ conjointement avec l'atome d'azote, auquel ils sont liés, en est (sont) un tel(s) comprenant de 1 à 3, en particulier 1 ou 2 atome(s) de carbone et/ou

f) le résidu d'alcoyle pouvant représenter $R_9$, en est (sont) un tel(s) comprenant de 1 à 4, en particulier 1 à 3 atomes de carbone et/ou

g) le résidu d'alcoxycarbonyle, auquel on peut substituer le résidu hétérocyclique que peuvent constituer $R_5$ et $R_6$ conjointement avec l'atome d'azote, auquel ils sont liés, et/ou

h) le résidu d'alcoxycarbonyle, auquel on peut substituer le résidu d'alcoyle que peut constituer $R_9$ ,

en

est (sont) un tel(s) comprenant de 2 à 4 atomes de carbone.

3. Dérivés de la pyrimido[5,4-b][1,4]oxazine conformément à la revendication 1 ou 2 caractérisés en ce que le résidu de cycloalkyle, que $R_6$ peut constituer, en est un tel comprenant de 3 à 5, en particulier 3 ou 4 atomes de carbone.

4. Dérivés de la pyrimido[5,4-b][1,4]oxazine conformément à la revendication 1 à 3 caractérisés en ce que

a) l'atome d'halogène, que peut constituer $R_2$ , ou

b) l'atome d'halogène, auquel on peut substituer le résidu de phenyle ou phenoxy, par lequel on peut substituer le résidu d' alcoyle, que peut constituer $R_6$,

est un atome de fluor ou de chlore.

5. Dérivés de la pyrimido[5,4-b][1,4]oxazine conformément à la revendication 1 à 3 caractérisés en ce que

a) le résidu hétérocyclique, auquel on peut substituer le résidu d'alcoyle pouvant constituer $R_6$ , ou

b) le résidu hétérocyclique, que peuvent constituer $R_5$ et $R_6$ conjointement avec l'atome d'azote, auquel ils sont liés,

est un résidu de morpholinyle, un résidu de piperidyle ou un résidu de piperazinyle.

6. 2-[Methyl]-4-[⟨2'-(morpholin-4''-yl)-ethyl⟩-amino]7-on,

4-[N-⟨Benzyl⟩-N⟨2'-(hydroxy)-ethyl⟩ -amino]-2-[methyl]-6,7-di-[hydro]-8H-pyrimido[5,4-b][1,4]oxazine-7-on

2-[Methyl]-4-[⟨3'-(morpholin-4''-yl)-propyl⟩-amino]-6,7-di-[hydro]-8H-pyrimido[5,4-b][1,4]oxazine-7-on,

4-[Chlore]-8-[2',3'-di-(hydroxy)-propyle]-2-[methyl]-6,7-di-[hydro]-8H-pyrimido[5,4-b][1,4]oxazine-7-on,

8-[2',3'-Di(hydroxy)-propyl]-2-[methyl]-4-[morpholin-4''-yl]-6,7-di-[hydro]-8H-pyrimido[5,4-b]]1,4]oxazine-7-on,

4-[⟨4'-(Benzyl)-morpholin-2'-yl)-methyl⟩-amino]-2-[methyl]-6,7-di-[hydro]-8H-pyrimido[5,4-b][1,4]oxazine-7-on,

8-[Ethoxy-carbonylmethyl]-4-[⟨2''-(hydroxy)-ethyl⟩-amino]-2-[methyl]-6,7-di-[hydro]-8H-pyrimido[5,4-b]-[1,4]oxazine-7-on,

4-[⟨2'-(Hydroxy)-ethyl⟩-amino]-2-[methyl]-8-[2''-(oxo)-propyl]-6,7-di-[hydro]-8H-pyrimido[5,4-b][1,4]-oxazine-7-on,

4-[⟨2'-(Hydroxy)-ethyl⟩-amino-2-[methyl]-8-[morpholin-4''-ylcarbonylmethy]-6,7-di-[hydro]-8H-pyrimido-[5,4-b][1,4]oxazine-7-on,

8-[(n-Butoxy)-carbonylmethyl]-4-[⟨2'(hydroxy)-ethyl⟩-amino]-2-[methyl]-6,7-di-[hydro]-8H-pyrimido[5,4-b]-[1,4]oxazine-7-on,

8-[Benzyl]-4-[⟨2'-(hydroxy)-ethyl⟩amino]-2-[methyl]-6,7-di-[hydro]-8H-pyrimido[5,4-b] [1,4]oxazine-7-on,

4-[Amino]-8-[(ethoxy)-carbonylmethyl]-2-[methyl]-6,7-di-[hydro]-8H-pyrimido[5,4-b][1,4]oxazine-7-on ainsi que leurs sels acides d'addition.

7. Procédé de production des composés conformément à la revendication 1 à 6 caractérisé en ce que
a) pour la production des dérivés de la pyrimido[5,4-b][1,4]oxazine de la formule générale I, dans laquelle $R_2$ représente un résidu de la formule générale II, dans laquelle $R_5$ et $R_6$ ont les significations indiquées dans les revendications 1 à 5 et $R_1$ , $R_3$ , $R_4$ et $R_9$ celles indiquées dans la revendication 1 ou 2, on fait réagir
des dérivés de la pyrimido[5,4-b][1,4]oxazine de la formule générale

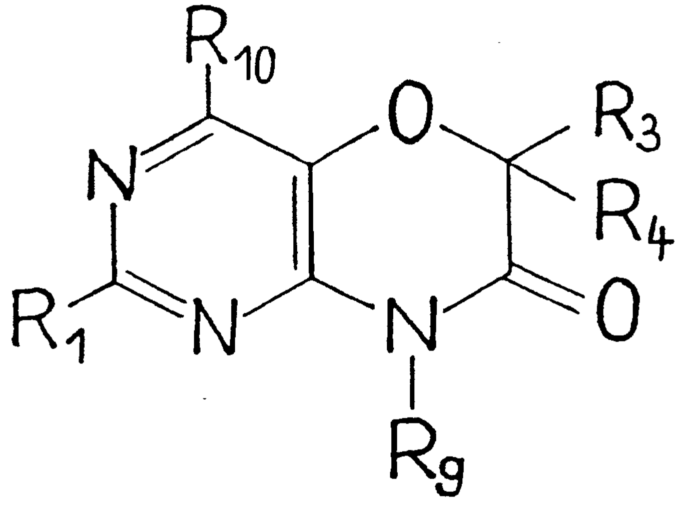

III ,

dans laquelle $R_1$ , $R_3$ , $R_4$ et $R_9$ ont les significations indiquées dans la revendication 1 ou 2 et $R_{10}$ représente un groupe sortant, avec des amines, présentant le cas échéant, un groupe de protection, de la formule générale

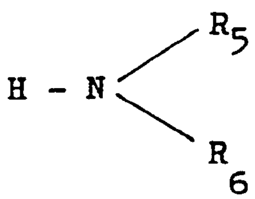

IV ,

dans laquelle $R_5$ et $R_6$ ont les significations indiquées dans les revendications 1 à 5 ou

b) pour la production des dérivés de la pyrimido[5,4-b][1,4]oxazine de la formule générale I, dans laquelle $R_2$représente un groupe azine et $R_1$ , $R_3$ , $R_4$ et $R_9$ ont les significations indiquées dans la revendication 1 ou 2 , on diazote

des dérivés de la pyrimido[5,4-b][1,4]oxazine de la formule générale I, dans laquelle $R_1$ , $R_3$ , $R_4$ et $R_9$ ont les significations indiquées dans la revendication 1 ou 2 et $R_2$ représente un groupe hydrazine, et/ou

c) pour la production des dérivés de la pyrimido[5,4-b][1,4]oxazine de la formule générale I, dans laquelle $R_1$ , $R_2$ , $R_3$ et $R_4$ ont les significations indiquées dans la revendication 1 ou 2 et $R_9$ représente un résidu de 2,3-di-(hydroxy)-propyle,

on fait réagir

des dérivés de la pyrimido[5-4-b][1,4]oxazine de la formule générale I, dans laquelle $R_1$ , $R_2$ , $R_3$ et $R_4$ ont les significations indiquées dans la revendication 1, 2 ou 4 et $R_9$ représente de l'hydrogène, avec du 2,3-(Epoxy)-propanol et

α) on fait réagir,le cas échéant, les dérivés de la pyrimido[5-4-b][1,4]oxazine de la formule générale I, obtenus d'après a) ou b), dans laquelle $R_1$ , $R_2$ , $R_3$ et $R_4$ ont les significations de la revendication 1, 2 ou 4 et $R_9$ représente de l'hydrogène avec des composés présentant, le cas échéant, un groupe de protection de la formule générale

R9 - X V ,

dans laquelle

$R_9$ a les significations indiquées dans la revendication 1 ou 2 à l'exception de l' hydrogène et

X représente un groupe sortant, et/ou

β) on élimine, le cas échéant,des dérivés obtenus d'après a), b) ou c) et, le cas échéant d' après α), de la pyrimido[5,4-b][1,4]oxazine de la formule générale I, le(s) groupe(s) de

protection et/ou

γ) on transfère, le cas échéant, les dérivés obtenus d'après a), b) ou c) et, le cas échéant, α) et/ou β) de la pyrimido 5,4-b 1,4 oxazine de la formule générale I pour les convertir en d'autres dérivés de la pyrimido[5,4-b][1,4] de la formule générale I et/ou

δ) on transfère les bases de dérivés obtenus d'après a), b) ou c) et, le cas échéant, d'après α), β) et/ou γ) de la pyrimido[5,4-b][1,4]oxazine de la formule générale I pour les convertir d'une manière connue en soi en sels acides d'addition et/ou on transfère, le cas échéant, les sels obtenus acides d'addition des dérivés de la pyrimido[5,4-b][1,4]oxazine de la formule générale I pour les convertir en bases libres de dérivés de la pyrimido[5,4-b][1,4] de la formule ou en d'autres sels.

8. Médicament, caractérisé par une teneur en 1 ou plusieurs composé(s) conformément à la revendication 1 à 6 agissant comme constituant(s) actif(s), utilement, conjointement avec 1 ou plusieurs supports et/ou produits auxiliaires pharmaceutiques classiques.

9. Dérivés de la pyrimido[5,4-b][1,4]oxazine de la formule générale

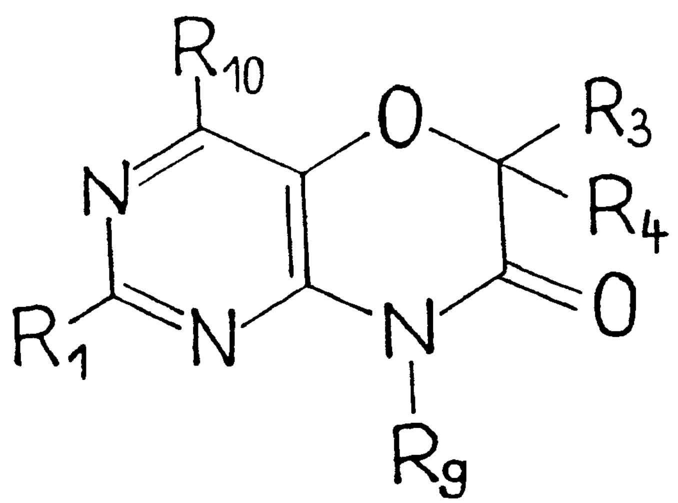

III ,

dans laquelle

| | |
|---|---|
| $R_{10}$ | représente un résidu d'alkylsulfonyloxy ou d'arylsulfonyloxy ou un résidu de methylsulfonyle et |
| $R_1$ , $R_3$ , $R_4$ et $R_9$ | ont les significations indiquées dans la revendication 1 ou 2. |

**Revendications pour l'Etat contractant suivant: AT**

1. Procédé de production de dérivés de la pyrimido[5,4-b][1,4]oxazine de la formule générale

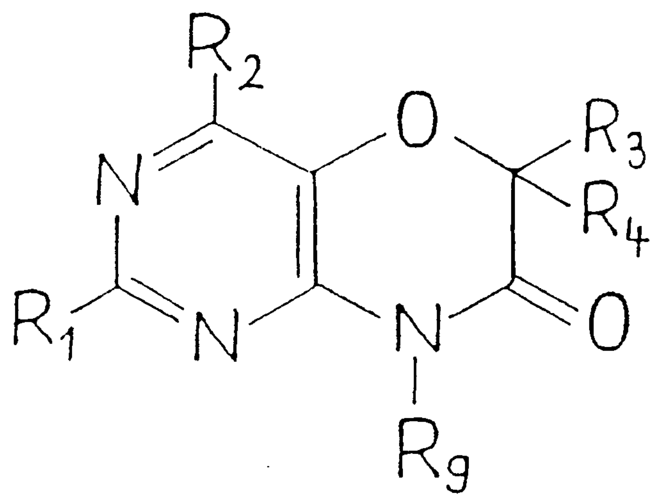

I ,

dans laquelle

$R_1$ représente un résidu d' alcoyle comprenant de 1 à 4 atome(s) de carbone

$R_2$ un atome d'hydrogène, un atome d'halogène, un groupe azine, un groupe hydrazine ou un résidu de la formule générale

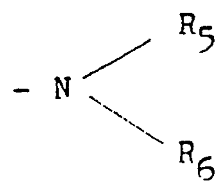

II ,

dans laquelle dernière

$R_5$ représente un atome d'hydrogène, un résidu de benzyle ou d'alcoyle comprenant de 1 à 4 atome(s) de carbone, le cas échéant, substitué par un groupe hydroxy et

$R_6$ constitue un atome d' hydrogène, un groupe amino, un résidu de cycloalcoyle comprenant de 3 à 6 atomes de carbone, un résidu d' alcoyle comprenant de 1 à 4 atome(s) de carbone, substitué par un résidu hétéro-cyclique substitué, le cas échéant, par un groupe hydroxy, un groupe mercapto, un résidu d' aminocarbonyle, un résidu de furyle, un résidu de 2-Benzo[1,4]dioxanyle, un résidu Di-(alkyl)-amino comprenant de 1 à 4 atome(s) de carbone dans chaque fraction alcoyle ou un résidu de phenyle ou phenoxy, le cas échéant, substitué par un atome d'halogène ou un résidu d'alcoyle présentant de 1 à 4 atome(s) de carbone ou 1 ou plusieurs résidu(s) alcoxy présentant de 1 à 4 atome(s) de carbone ou présentant de l'azote saturé à 6 chainons, éventuellement substitué par un résidu de benzyle ou,le cas échéant, par un résidu d'alcoyle présentant de 1 à 4 atome(s) de carbone et présentant, le cas échéant, outre l'azote, encore un autre atome d'azote et/ou un atome d'oxygène ou

$R_5$ et $R_6$ conjointement avec l'atome d'azote, auquel ils sont liés, constituent un résidu hétérocyclique, le cas échéant substitué par un résidu d'alcoxycarbonyle comprenant de 2 à 4 atome(s) de carbone ou un résidu d'hydroxyalcoyle comprenant de 1 à 4 atome(s) de carbone, lequel résidu hétérocyclique est saturé à 6 chainons et présente, le cas échéant, outre l'atome d'azote, encore 1 autre atome d'azote et/ou 1 atome d'oxygène,

$R_3$ et $R_4$ qui peuvent être identiques ou différents, représentant indépendamment entre eux un atome d'hydrogène ou un résidu d'alcoyle comprenant de 1 à 4 atome(s) de carbone et

$R_9$ signifie un atome d' hydrogène, un résidu d' alcoyle, le cas échéant, mono-substitué par un groupe oxo, un groupe cyano, un résidu d' aminocarbonyle, un résidu d'alcoxy-

carbobyle comprenant de 2 à 5 atomes de carbone, un résidu de pyridyle, un résidu de morpholinylcarbonyle ou un résidu de phenyle ou mono- ou polysubstitué par 1 ou plusieurs groupe(s) hydroxy,

étant de plus entendu que

a)

$R_2$ est différent d'un atome d'hydrogène, d'un atome de chlore, d'un résidu de 4-morpholinyle ou d'un résidu de piperidyle, lorsque $R_9$ représente un atome d'hydrogène et

b)

$R_2$ est différent d'un atome de chlore ou d'un résidu de 4-morpholinyle, lorsque $R_9$ représente un résidu de methyle ou de benzyle,

y compris leurs tautomères et leurs mélanges ainsi que leurs sels acides d'addition, caractérisé en ce que

a) pour la production des dérivés de la pyrimido[5,4-b][1,4]oxazine de la formule générale I, dans laquelle $R_2$ représente un résidu de la formule générale II, dans laquelle $R_5$ et $R_6$ ont les significations indiquées ci-dessus et $R_1$ , $R_3$ , $R_4$ et $R_9$ les significations indiquées ci-dessus, on fait réagir des dérivés de la pyrimido[5,4-b][1,4]oxazine de la formule générale

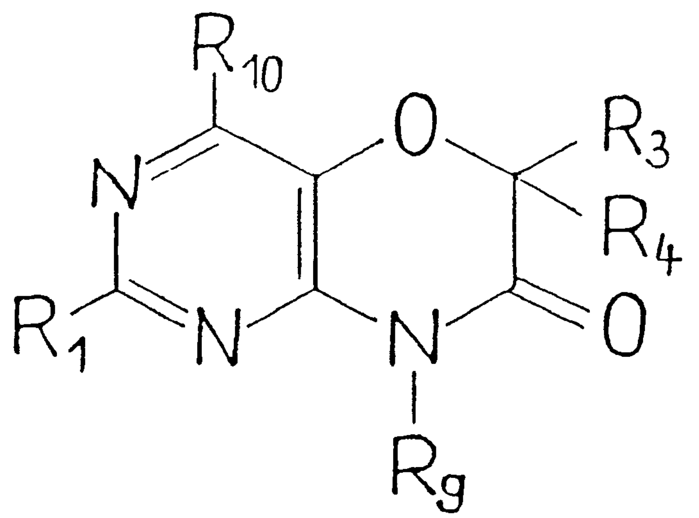

III ,

dans laquelle $R_1$ , $R_3$ , $R_4$ et $R_9$ ont les significations indiquées ci-dessus et $R_{10}$ représente un groupe sortant, avec des amines, présentant , le cas échéant, un groupe de protection , de la formule générale

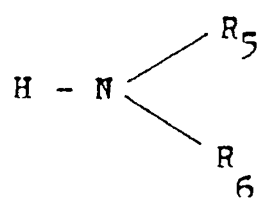

IV ,

dans laquelle $R_5$ et $R_6$ ont les significations indiquées ci-dessus, ou

b) pour la production des dérivés de la pyrimido[5,4-b][1,4]oxazine de la formule générale I, dans laquelle $R_2$ représente un groupe azide et $R_1$ , $R_3$ , $R_4$ et $R_9$ ont les significations indiquées ci-dessus, on diazote

des dérivés de la pyrimido[5,4-b,][1,4]oxazine de la formule générale I, dans laquelle $R_1$ , $R_3$ , $R_4$ et $R_9$ ont les significations indiquées ci-dessus et $R_2$ représente un groupe hydrazine et/ou

c) pour la production des dérivés de la pyrimido[5,4-b][1,4]oxazine de la formule générale I, dans laquelle $R_1$ , $R_2$ , $R_3$ et $R_4$ ont les significations indiquées ci-dessus et $R_9$ représente un résidu de 2,3-Di-(hydroxy)-propyle,

on fait réagir

des dérivés de la pyrimido[5,4-b][1,4]oxazine de la formule générale I, dans laquelle $R_1$ , $R_2$ , $R_3$ et

$R_4$ ont les significations indiquées ci-dessus et $R_9$ représente de l'hydrogène, avec du 2,3-(Epoxy)-propanol et

α) on fait réagir, le cas échéant, les dérivés obtenus d'après a) ou b) de la pyrimido[5,4-b][1,4]-oxazine de la formule générale I, dans laquelle $R_1$ , $R_2$ , $R_3$ et $R_4$ ont les significations indiquées ci-dessus et $R_9$ représente de l'hydrogène avec des composés présentant, le cas échéant, un groupe de protection de la formule générale

$R_9$ - X V ,

dans laquelle

$R_9$ a les significations indiquées ci-dessus à l'exception de l' hydrogène et

X représente un groupe sortant, et/ou

β) on élimine, le cas échéant, des dérivés obtenus d'après a), b) ou c) et,le cas échéant, α), de la pyrimido[5,4-b][1,4]oxazine de la formule générale I, le(s) groupe(s) de protection et/ou

γ) on transfère, le cas échéant, les dérivés obtenus d'après a), b) ou c) et, le cas échéant, α) et/ou β) de la pyrimido[5,4-b][1,4]oxazine de la formule générale pour les convertir en d'autres dérivés de la pyrimido[5,4-b][1,4]oxazine de la formule générale I et/ou

δ) on transfère les bases de dérivés obtenus d' après a), b) ou c) et, le cas échéant, α), β) et/ou γ) de la pyrimido[5,4-b][1,4]oxazine de la formule générale I pour les convertir d'une manière connue en soi en sels acides d'addition et/ou on transfère, le cas échéant, les sels obtenus acides d'addition des dérivés de la pyrimido[5,4-b][1,4]oxazine de la formule générale I pour les convertir en bases libres de dérivés de la pyrimido[5,4-b][1,4]oxazine de la formule générale I ou en d'autres sels acides d'addition.

**2.** Procédé conformément à la revendication 1, caractérisé en ce qu'on produit des dérivés de la pyrimido[5,4-b][1,4]oxazine , dans lesquels

a) le résidu d'alcoyle, que $R_1$ et/ou $R_3$ et/ou $R_4$ et/ou $R_5$ et/ou $R_6$ peut, voire peuvent, représenter, et/ou

b) les fractions d'alcoyle du résidu di-(alkyl)-amino, auquel on peut substituer le résidu d'alcoyle, que peut représenter $R_6$ ou

c) le résidu d'alcoyle ou d'alcoxy, auquel on peut substituer le résidu de phenyle ou de phenoxy, par lequel on peut substituer le résidu d'alcoyle pouvant consituer $R_6$ ou

d) le résidu d'alcoyle, auquel on peut substituer le résidu de benzyle, par lequel on peut substituer le résidu hétérocyclique, par lequel on peut substituer le résidu d' alcoyle, que peut constituer $R_6$ ou

e) le résidu d' hydroxyalkyle, auquel on peut substituer le résidu hétérocyclique, que peuvent constituer $R_5$ et $R_6$ conjointement avec l'atome d'azote, auquel ils sont liés,

en est (sont) un tel(s) comprenant de 1 à 3, en particulier 1 ou 2 atome(s) de carbone et/ou

f) le résidu d'alcoyle que peut représenter $R_9$

en est (sont) un(de) tel(s) comprenant de 1 à 4, en particulier 1 à 3 atomes de carbone et/ou

g) le résidu d'alcoxycarbonyle, auquel on peut substituer le résidu hétérocyclique que peuvent constituer $R_5$ et $R_6$ conjointement avec l'atome d'azote, auquel ils sont liés et/ou

h) le résidu d'alcoxycarbonyle, auquel on peut substituer le résidu d'alcoyle que peut constituer $R_9$ ,

en est (sont) un (de) tel(s) comprenant de 2 à 4 atomes de carbone.

**3.** Procédé conformément à la revendication 1 ou 2, caractérisé en ce qu'on produit des dérivés de la pyrimido[5,4-b][1,4]oxazine, dans lesquels le résidu de cycloalcoyle, que $R_6$ peut représenter en est un présentant de 3 à 5, en particulier 3 ou 4 atomes de carbone.

**4.** Procédé conformément à la revendication 1 à 3, caractérisé en ce qu'on produit des dérivés de la pyrimido[5,4-b][1,4]oxazine, dans lesquels

a) l'atome d'halogène, que $R_2$ peut représenter ou

b) l'atome d'halogène auquel le résidu de phenyle ou phenoxy peut être substitué, auquel le résidu d'alcoyle, que $R_6$ peut représenter, peut être substitué,

est un atome de fluor ou de chlore.

5. Procédé conformément à la revendication 1 à 3, caractérisé en ce qu'on produit des dérivés de la pyrimido[5,4-b][1,4]oxazine, dans lesquels
   a) le résidu hétérocyclique, auquel le résidu d'alcoyle, que $R_6$ peut représenter, peut être substitué, ou
   b) le résidu hétérocyclique, que peuvent constituer $R_5$ et $R_6$ conjointement avec l'atome d'azote, auquel ils sont liés,

   est un résidu de morpholinyle, un résidu de pipéridyle ou un résidu de piperazinyle.

6. Procédé conformément à la revendication 1 à 3, caractérisé en ce qu'on produit des

   2-[Methyl]-4-[⟨2'-(morpholin-4''-yl)-ethyl⟩- - amino]-6,7-di-[hydro]-8H-pyrimido[5,4-b][1,4]oxazine-7-on,

   4-[N-⟨Benzyl⟩-N-⟨2'-(hydroxy)-ethyl⟩-amino]-2-[methyl]-6,7-di-[hydro]-8H-pyrimido[5,4-b][1,4]oxazine-7-on,

   2-[Methyl]-4-[⟨3'-(morpholin-4''-yl)-propyl⟩-amino]-6,7-di-[hydro]-8H-pyrimido[5,4-b][1,4]oxazine-7-on,

   4-[Chlore]-8-[2',3'-di-(hydroxy)-propyl]-2-[methyl]-6,7-di-[hydro]-8H-pyrimido[5,4-b][1,4]oxazine-7-on,

   8-[2',3'-Di-(hydroxy)-propyl]-2-[methyl]-4-[morpholin-4''-yl]-6,7-di-[hydro]-8H-pyrimido[5,4-b][1,4]-oxazine-7-on,

   4-[⟨4'-(Benzyl)-morpholin-2'-yl)-methyl⟩-amino]-2-[methyl]-6,7-di-[hydro]-8H-pyrimido[5,4-b][1,4]oxazine-7-on,

   8-[Ethoxy-carbonylmethyl]-4-[⟨2''-(hydroxy)-ethyl⟩-amino]-2-[methyl]-6,7-di-[hydro]-8H-pyrimido[5,4-b]-[1,4]oxazine-7-on,

   4-[⟨2'-(Hydroxy)-ethyl⟩-amino]-2-[methyl]-8-[2''-(oxo)-propyl]-6,7-di-[hydro]-8H-pyrimido[5,4-b][1,4]-oxazine-7-on,

   4-[⟨2'-(Hydroxy)-ethyl⟩-amino]-2-[methyl]-8-[morpholin-4''-ylcarbonylmethyl]-6,7-di-[hydro]-8H-pyrimido-[5,4-b][1,4]oxazine-7-on,

   8-[(n-Butoxy)-carbonylmethyl]-4-[⟨2'-(hydroxy)-ethyl⟩-amino]-2-[methyl]-6,7-di-[hydro]-8H-pyrimido[5,4-b][1,4]oxazine-7-on,

   8-[Benzyl]-4-[⟨2'-(hydroxy)-ethyl⟩-amino]-2-[methyl]-6,7-di-[hydro]-8H-pyrimido[5,4-b][1,4]oxazine-7-on,

   4-[Amino]-8-[(ethoxy)-carbonylmethyl]-2-[methyl]-6,7-di-[hydro]-8H-pyrimido[5,4-b][1,4]oxazine-7-on,

   ainsi que leurs sels acides d'addition.

7. Procédé de production des dérivés de la pyrimido[5,4-b][1,4]oxazine de la formule générale

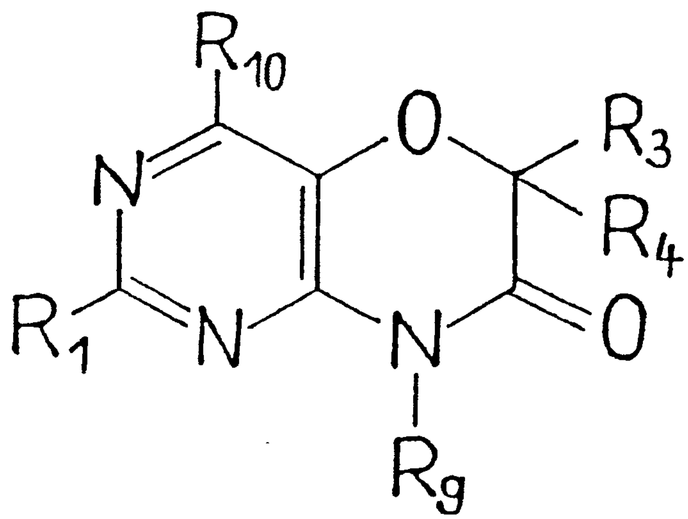

III ,

dans laquelle

$R_{10}$ représente un résidu d' alkylsulfonyloxy ou d'arylsulfonyloxy ou un résidu de methylsulfonyle et

$R_1$ , $R_3$ , $R_4$ et $R_9$ ont les significations indiquées dans la revendication 1 ou 2,

caractérisé en ce que

a) pour la production de ceux, dans lesquels $R_{10}$ représente un résidu d'alkylsulfonyloxy ou d'arylsulfonyloxy

on transforme des dérivés de la pyrimido[5,4-b][1,4]oxazine de la formule générale I à la différence que $R_2$ représente un groupe hydroxy, pour les faire réagir avec les chlorures correspondants d' arylsulfonyle pour obtenir un agent basique ou

b) pour la production de ceux, dans lesquels $R_{10}$ représente un résidu de methylsulfonyle

on oxyde d'une manière connue en soi des dérivés de la pyrimido[5,4-b][1,4]oxazine de la formule générale III, dans lesquels $R_{10}$ représente un résidu de methylthio.